# EUROPEAN PATENT APPLICATION

(11) **EP 2 805 664 A1**
(43) Date of publication of application: **26.11.2014**
(21) Application number: 12865740.0
(22) Date of filing: 26.11.2012
(51) Int. Cl.: A61B 1/00

(54) **ENDOSCOPE**

(30) Priority: 16.01.2012 JP 2012006302; 08.02.2012 JP 2012025357
(71) Applicant: Olympus Medical Systems Corp., Tokyo 151-0072 (JP)
(72) Inventor: OKAMOTO, Yasuhiro, Tokyo 151-0072 (JP)
(74) Representative: Schicker, Silvia
(86) International application number: PCT/JP2012/080477
(87) International publication number: WO 2013/108486

(57) **Abstract**

This invention has an object to provide an endoscope which efficiently and reliably obtains a stronger pulling force. To the end, the endoscope includes a drive section 12 which generates driving force for bending and driving a bending portion 2b, a C ring-shaped member 9 which is frictionally engageable with a pulley 11 and has a notch portion 9c at a portion of the ring-shaped member, an operation input member 5 which bends and operates the bending portion, an operation input-side pulling member 8a which is a first pulling member wound around the C ring-shaped member and extending from the C ring-shaped member toward the operation input member and is coupled to the operation input member such that a wrap distance, over which the operation input-side pulling member wraps around the C ring-shaped member across the notch portion from an extension position toward the operation input member of the C ring-shaped member, decreases with increase in the amount of operation of the operation input member, and a bending portion-side pulling member 8b which is a second pulling member wound around the C ring-shaped member and extending from the C ring-shaped member toward the bending portion and is coupled to the bending portion so as not to wrap around the C ring-shaped member across the notch portion from an extension position toward the bending portion on the C ring-shaped member.

## Description

### Technical Field

The present invention relates to an endoscope configured to be capable of moving a pulling member by performing a tilt operation of changing a tilt direction and a tilt angle of an operation unit which is provided at an operation portion and bending and operating a bending portion which is provided at the operation portion with assistance of driving force of drive means.

### Background Art

Endoscopes including an elongated insertion portion have recently been utilized in a medical field or an industrial field. In an endoscope in the medical field, an insertion portion is inserted into a body through an oral cavity, an anus, or the like and used for observation and the like. In an endoscope in the industrial field, an insertion portion is inserted into piping of plant equipment, such as a boiler, or into an interior of an engine and used for observation and the like.

An endoscope generally has an observation optical system provided at a distal end portion of an insertion portion. A bending portion which bends in, e.g., upward, downward, leftward, and rightward directions is also provided on a distal end side of the insertion portion to turn the observation optical system in a desired direction. An operation portion including a bending operation apparatus is further provided at a proximal end of the insertion portion. A bending operation knob serving as an operation member of the bending operation apparatus and a distal end bending piece constituting the bending portion are coupled by a pulling wire serving as a pulling member. The conventional endoscope with the above-described configuration is configured such that an operator can pull or slacken the pulling wire coupled between the bending operation knob and a predetermined position of the bending portion by operating to rotate the operation member, such as the bending operation knob, in a clockwise direction or in a counterclockwise direction with fingers of a hand grasping the operation portion to allow the bending portion to make a bending motion. An endoscope with the above-described configuration will be referred to as a conventional endoscope hereinafter.

Aside from a conventional endoscope with the above-described configuration, an endoscope has recently been proposed which is provided with, for example, a bending operation apparatus having drive means inside an operation portion of the endoscope, is configured to be capable of causing a bending portion to make a bending motion with assistance of driving force of the drive means in the bending operation apparatus when an operation unit which is provided standing at the operation portion is tilted and operated, for example, with a slight amount of operation force by fingers or the like, and has excellent operability.

An endoscope with the above-described configuration, i.e., including a pulling member operation apparatus is configured to change force of an operation wire which is fixed to an arm member and corresponding to the tilt operation against a pulley (rotated by a motor) by tilting and operating a bending lever and changing tension of the corresponding operation wire and bend a bending portion by moving the operation wire in a rotation direction of the pulley.

For example, Japanese Patent Application Laid-Open Publication No. 2003-325437, Japanese Patent Application Laid-Open Publication No. 2009-5836, or the like discloses an endoscope including a pulling member operation apparatus which is configured to be capable of bending and operating a bending portion by tilting and operating an operation instruction lever serving as an operation unit with a slight amount of operation force and moving a desired pulling member by a desired amount.

The endoscope is configured to be capable of changing tension of a pulling wire fixed to an arm member which links to a bending lever by tilting and operating the bending lever. The endoscope changes force of the pulling wire against a pulley being rotated by drive means (a motor) by changing the tension of the pulling wire while tilting and operating the bending lever. In the case, a C ring-shaped member having a rotation amount adjustment function is provided between the pulley and the pulling wire. With the configuration, the endoscope is configured to bend the bending portion by moving the pulling wire in a rotation direction of the pulley.

The endoscope with the above-described configuration is known to tend to be capable of obtaining a stronger pulling force by, for example, increasing an angle (amount) of winding of the pulling wire on the C ring-shaped member.

The conventional endoscope disclosed in Japanese Patent Application Laid-Open Publication No. 2003-325437, Japanese Patent Application Laid-Open Publication No. 2009-5836, or the like, however, is problematic in that a rotation amount adjustment mechanism of the C ring-shaped member may not act well or a necessary pulling force may not be obtained even if measures to, e.g., simply increase the angle of winding of the pulling wire on the C ring-shaped member are taken as means for obtaining a stronger pulling force.

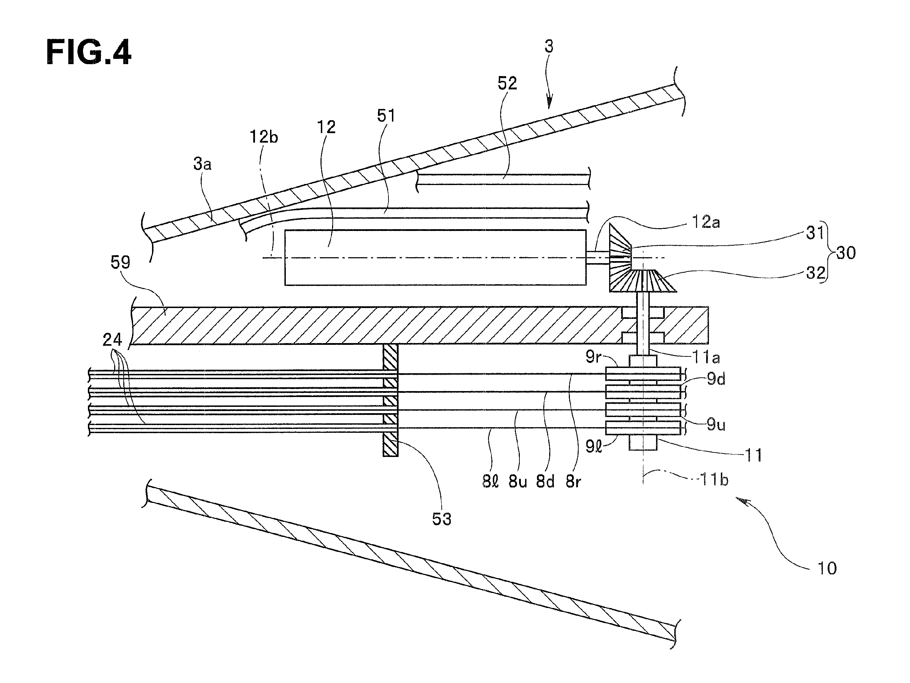

An endoscope with the configuration disclosed in Japanese Patent Application Laid-Open Publication No. 2009-5836 or the like prevents interference between operation wires by winding an intermediate portion of each operation wire such that the intermediate portion makes substantially one rotation around an outer circumferential face of a paired annular member (see Fig. 4 of the publication). That is, the wound operation wires are positionally shifted on the annular members in a thrust direction (a width direction X in Fig. 4 of the publication). As a result, when each annular member having a notch is reduced in diameter by tightening the operation wire intermediate portion through tilt operation of an operation lever, a force in a direction in which the wire intermediate portion reduces the annular member in diameter and a force in a direction different from the diameter reduction direction are generated. The forces distort the annular member, which may make a status of close contact between an inner surface of the annular member and an outer circumferential face of a pulley nonuniform and make sufficient force against the pulley difficult to obtain.

The present invention has been made in consideration of the above-described points and has an object to provide an endoscope capable of moving a pulling member with assistance of driving force of drive means by tilting and operating an operation unit and pulling the pulling member and bending and operating a bending portion, which can efficiently and reliably obtaining a stronger pulling force.

Another object of the present invention is to provide an endoscope capable of bending and operating a bending portion by reducing an annular member in diameter through operation of an operation lever without distorting the annular member, bringing an inner surface of the annular member into uniform and close contact with an outer circumferential face of a pulley to obtain sufficient force against the pulley, and pulling a pulling member.

### Disclosure of Invention

### Means for Solving the Problem

In order to attain the above-described objects, an endoscope according to one aspect of the present invention includes a drive section which generates driving force for bending and driving a bending portion, a C ring-shaped member which is a ring-shaped member frictionally engageable with a drive shaft of the drive section and has a notch portion at a portion of the ring-shaped member, an operation input member for bending and operating the bending portion, an operation input-side pulling member which is a first pulling member wound around the C ring-shaped member and extending from the C ring-shaped member toward the operation input member and is coupled to the operation input member such that a wrap distance, over which the operation input-side pulling member wraps around the C ring-shaped member across the notch portion from an extension position toward the operation input member on the C ring-shaped member, decreases with increase in the amount of operation of the operation input member, and a bending portion-side pulling member which is a second pulling member wound around the C ring-shaped member and extending from the C ring-shaped member toward the bending portion and is coupled to the bending portion so as not to wrap around the C ring-shaped member across the notch portion from an extension position toward the bending portion on the C ring-shaped member.

According to the present invention, there can be provided an endoscope capable of moving a pulling member with assistance of driving force of drive means by tilting and operating an operation unit and pulling the pulling member, and bending and operating a bending portion, which can efficiently and reliably obtain a stronger pulling force.

### Brief Description of the Drawings

Fig. 1 is a schematic configuration view showing a whole endoscope system including an endoscope according to a first embodiment of the present invention;
Fig. 2 is a schematic configuration view showing only a schematic configuration of a pulling member operation apparatus of an internal configuration of the endoscope in the endoscope system shown in Fig. 1;
Fig. 3 is an external perspective view showing a C ring-shaped member and a pulling wire of a pulling member operation apparatus in the endoscope of the endoscope system shown in Fig. 1;
Fig. 4 is an enlarged configuration view of a main portion showing a layout configuration of the pulling member operation apparatus in the endoscope of the endoscope system shown in Fig. 1;
Fig. 5 is an enlarged configuration view of a main portion showing a layout configuration of an operation member which works in conjunction with the pulling member operation apparatus and pulling members in the endoscope of the endoscope system shown in Fig. 1;
Fig. 6 is a conceptual view showing a detailed configuration when the pulling member operation apparatus in Fig. 2 is stationary;
Fig. 7 is a conceptual view showing an example of a working state of the pulling member operation apparatus in Fig. 2;
Fig. 8 is a conceptual view showing an example of a case where the C ring-shaped member in the pulling member operation apparatus in Fig. 2 rotates to fall outside an allowable range;
Fig. 9 is a conceptual view showing a detailed configuration when a pulling member operation apparatus in an endoscope according to a second embodiment of the present invention is stationary;
Fig. 10 is an external perspective view showing a C ring-shaped member and a pulling wire taken out from the pulling member operation apparatus in Fig. 9;
Fig. 11 is a conceptual view showing an example of a working state of the pulling member operation apparatus in Fig. 9;
Fig. 12 is a conceptual view showing a detailed configuration when a pulling member operation apparatus in an endoscope according to a third embodiment of the present invention is stationary;
Fig. 13 is an external perspective view showing a C ring-shaped member and a pulling wire taken out from the pulling member operation apparatus in Fig. 12;
Fig. 14 is a conceptual view showing an example of a working state of the pulling member operation apparatus in Fig. 12;
Fig. 15 is a conceptual view showing a detailed configuration when a pulling member operation apparatus in an endoscope according to a fourth embodiment of the present invention is stationary;
Fig. 16 is an external perspective view showing a C ring-shaped member, a pulling wire and a coil pipe taken out from the pulling member operation apparatus in Fig. 15;
Fig. 17 is a conceptual view showing an example of a working state of the pulling member operation apparatus in Fig. 15;
Fig. 18 is a top view of a neighborhood of the C ring-shaped member in Fig. 17;
Fig. 19 is a side view showing a C ring-shaped member according to a first modification of the fourth embodiment of the present invention;
Fig. 20 is an external perspective view showing a C ring-shaped member, a pulling wire and a coil pipe according to a second modification of the fourth embodiment of the present invention;
Fig. 21 is a side view of Fig. 20;
Fig. 22 is an external perspective view showing a C ring-shaped member, a pulling wire and a coil pipe according to a third modification of the fourth embodiment of the present invention;
Fig. 23 is a side view of Fig. 22;
Fig. 24 is a conceptual view showing a detailed configuration when a pulling member operation apparatus in an endoscope according to a fifth embodiment of the present invention is stationary;
Fig. 25 is a top view of a neighborhood of a C ring-shaped member in Fig. 24;
Fig. 26 is an external perspective view showing the C ring-shaped member and a pulling wire taken out from the pulling member operation apparatus in Fig. 24;
Fig. 27 is a conceptual view showing an example of a working state of the pulling member operation apparatus in Fig. 24;
Fig. 28 is a top view of a neighborhood of the C ring-shaped member in Fig. 27;
Fig. 29 is a conceptual view showing a detailed configuration when a pulling member operation apparatus in an endoscope according to a sixth embodiment of the present invention is stationary;
Fig. 30 is a top view of a neighborhood of a C ring-shaped member in Fig. 29;
Fig. 31 is an external perspective view showing the C ring-shaped member and a pulling wire taken out from the pulling member operation apparatus in Fig. 29;
Fig. 32 is a conceptual view showing an example of a working state of the pulling member operation apparatus in Fig. 29;
Fig. 33 is a top view of a neighborhood of the C ring-shaped member in Fig. 32;
Fig. 34 is a conceptual view showing a detailed configuration when a pulling member operation apparatus in an endoscope according to a seventh embodiment of the present invention is stationary;
Fig. 35 is a top view of a neighborhood of a C ring-shaped member in Fig. 34;
Fig. 36 is an external perspective view showing the C ring-shaped member and a pulling wire taken out from the pulling member operation apparatus in Fig. 34;
Fig. 37 is a conceptual view showing an example of a working state of the pulling member operation apparatus in Fig. 34;
Fig. 38 is a top view of a neighborhood of the C ring-shaped member in Fig. 37;
Fig. 39 is a conceptual view showing a detailed configuration when a pulling member operation apparatus in an endoscope according to an eighth embodiment of the present invention is stationary;
Fig. 40 is a conceptual view showing an example of a working state of the pulling member operation apparatus in Fig. 39;
Fig. 41 is a side view showing a C ring-shaped member according to a modification of the eighth embodiment of the present invention;
Fig. 42 is a conceptual view showing a detailed configuration when a pulling member operation apparatus in an endoscope according to a ninth embodiment of the present invention is stationary;
Fig. 43 is a conceptual view showing an example of a working state of the pulling member operation apparatus in Fig. 42;
Fig. 44 is a side view showing a C ring-shaped member according to a modification of the ninth embodiment of the present invention;
Fig. 45 is a conceptual view showing a detailed configuration when a pulling member operation apparatus in an endoscope according to a tenth embodiment of the present invention is stationary;
Fig. 46 is a conceptual view showing an example of a working state of the pulling member operation apparatus in Fig. 45;
Fig. 47 is a side view showing a C ring-shaped member according to a modification of the tenth embodiment of the present invention;
Fig. 48 is a conceptual view showing a detailed configuration when a pulling member operation apparatus in an endoscope according to an 11th embodiment of the present invention is stationary;
Fig. 49 is an external perspective view showing a C ring-shaped member and a pulling wire taken out from the pulling member operation apparatus in Fig. 48;
Fig. 50 is a conceptual view showing an example of a working state of the pulling member operation apparatus in Fig. 48;
Fig. 51 is a conceptual view showing a detailed configuration when a pulling member operation apparatus in an endoscope according to a 12th embodiment of the present invention is stationary;
Fig. 52 is an external perspective view showing a C ring-shaped member and a pulling wire taken out from the pulling member operation apparatus in Fig. 51;
Fig. 53 is a conceptual view showing an example of a working state of the pulling member operation apparatus in Fig. 51;
Fig. 54 is a conceptual view showing a detailed configuration when a pulling member operation apparatus in an endoscope according to a 13th embodiment of the present invention is stationary;
Fig. 55 is an external perspective view showing a C ring-shaped member and a pulling wire taken out from the pulling member operation apparatus in Fig. 54;
Fig. 56 is a conceptual view showing an example of a working state of the pulling member operation apparatus in Fig. 54;
Fig. 57 is a conceptual view showing a detailed configuration when a pulling member operation apparatus in an endoscope according to a 14th embodiment of the present invention is stationary (a cross section of a C ring-shaped member is shown);
Fig. 58 is an external perspective view showing the C ring-shaped member and a pulling wire taken out from the pulling member operation apparatus in Fig. 57;
Fig. 59 is a conceptual view showing an example of a working state of the pulling member operation apparatus in Fig. 57 (a cross section along line [59]-[59] in Fig. 60 of the C ring-shaped member is shown);
Fig. 60 is a top view of a neighborhood of the C ring-shaped member in the state in Fig. 59;
Fig. 61 is a view on arrow [60] in Fig. 59;
Fig. 62 is a conceptual view showing a detailed configuration when a pulling member operation apparatus in an endoscope according to a 15th embodiment of the present invention is stationary (a cross section of a C ring-shaped member is shown);
Fig. 63 is an external perspective view showing the C ring-shaped member and a pulling wire taken out from the pulling member operation apparatus in Fig. 62;
Fig. 64 is a conceptual view showing an example of a working state of the pulling member operation apparatus in Fig. 62 (a cross section along line [64]-[64] in Fig. 65 of the C ring-shaped member is shown);
Fig. 65 is a top view of a neighborhood of the C ring-shaped member in the state in Fig. 64;
Fig. 66 is a conceptual view showing a detailed configuration when a pulling member operation apparatus in an endoscope according to a 16th embodiment of the present invention is stationary (a cross section of a C ring-shaped member is shown);
Fig. 67 is an external perspective view showing the C ring-shaped member and a pulling wire taken out from the pulling member operation apparatus in Fig. 66;
Fig. 68 is a conceptual view showing an example of a working state of the pulling member operation apparatus in Fig. 66 (a cross section along line [68]-[68] in Fig. 69 of the C ring-shaped member is shown);
Fig. 69 is a top view of a neighborhood of the C ring-shaped member in the state in Fig. 68;
Fig. 70 is a view on arrow [70] in Fig. 68;
Fig. 71 is a view for explaining an endoscope according to a 17th embodiment of the present invention in which an operation unit constituting a bending operation apparatus is provided standing at an operation portion;
Fig. 72 is a side view for explaining a configuration of an interior of the operation portion that is composed of a grasping portion and an operation portion body;
Fig. 73 is a view for explaining a relationship among a motor, a pulley, rotors, a suspension frame, and bending wires inside the operation portion;
Fig. 74 is a perspective view for explaining a rotor and a bending wire including a first wire, a second wire, and a wire relief member which are wound around the rotor;
Fig. 75 is a view of the rotor, around which the bending wire is wound, as viewed from a direction of an arrow Y75 in Fig. 74;
Fig. 76 is a cross-sectional view taken along a line pointed by arrows Y76 in Fig. 75;
Fig. 77 is a view of the rotor, around which the bending wire is wound, as viewed from a direction of an arrow Y77 in Fig. 74;
Fig. 78 is a view for explaining a relationship between a bending wire according to a first modification of the endoscope according to the 17th embodiment of the present invention which includes a first wire and a second wire and the rotor;
Fig. 79 is a cross-sectional view taken along a line pointed by arrows Y79 in Fig. 78;
Fig. 80 is a view for explaining a configuration of a rotor according to a second modification of the endoscope according to the 17th embodiment of the present invention;
Fig. 81 is a view for explaining a configuration of a rotor according to a fourth modification of the endoscope according to the 17th embodiment of the present invention and the rotor having a notch groove;
Fig. 82 is a view of the rotor as viewed from a direction of an arrow 82 in Fig. 81; and
Fig. 83 is a view for explaining an endoscope configured such that a first wire is threaded through a long hole of a wire relief member according to a fifth modification of the endoscope according to the 17th embodiment of the present invention.

### Best Mode for Carrying Out the Invention

The present invention will be described below in a context of the illustrated embodiments.

Note that each constituent element may be differently scaled in the individual drawings used in the description below such that the constituent element has a recognizable size on the drawings. The present invention is thus not limited only to the quantity of constituent elements, shapes of constituent elements, size ratios among constituent elements, and relative positional relationships among individual constituent elements described in the drawings.

### [First Embodiment]

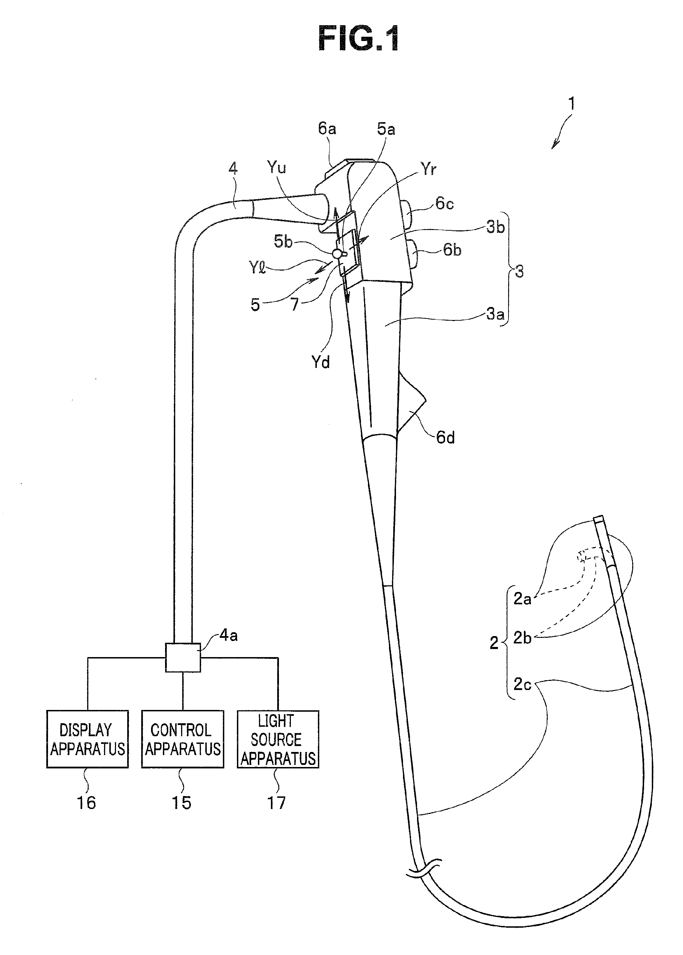

Figs. 1 to 8 are views showing a first embodiment of the present invention.

A configuration of a whole endoscope system including an endoscope according to the first embodiment of the present invention will be described below mainly with reference to Fig. 1.

The endoscope system including an endoscope 1 according to the present embodiment is mainly composed of the endoscope 1, a control apparatus 15, a display apparatus 16, a light source apparatus 17, and the like.

The endoscope 1 is composed of an insertion portion 2 in an elongated tube shape, an operation portion 3 which is provided on a proximal end side of the insertion portion 2 to be linked to the insertion portion 2, a universal cord 4 which extends from a side portion of the operation portion 3, and the like.

The insertion portion 2 is composed of a distal end portion 2a, a bending portion 2b that is configured to be bendable in, for example, upward, downward, leftward, and rightward directions, and a flexible tube portion 2c that has flexibility and is formed to be long, all of which are provided to be linked in order from a distal end side. An image pickup apparatus (not shown) having an image pickup device is built in the distal end portion 2a.

The operation portion 3 is configured to include a grasping portion 3a which is provided to be linked to the insertion portion 2 and an operation portion body 3b which is provided to be linked to the grasping portion 3a. A longitudinal axis of the grasping portion 3a and an insertion axis of the insertion portion 2 are coaxial or have a parallel positional relationship. An operation unit 5 serving as an operation input member for inputting an amount of force for pulling a pulling wire 8 serving as a pulling member (to be described later) (an operation input-side wire 8a, whose details will be described later) and performing an operation for a bending motion by the bending portion 2b is provided at a side portion on a distal end side of the operation portion body 3b. A longitudinal axis of the operation portion body 3b (i.e., a longitudinal axis of the operation portion 3) and the longitudinal axis of the grasping portion 3 a are coaxial or have a parallel positional relationship.

The operation unit 5 is made up of a shaft portion 5a and a spherical finger touch portion 5b which is securely provided at a distal end of the shaft portion 5a and is formed in a form of a so-called joystick. The operation unit 5 is provided so as to protrude outwardly from an opening portion (not shown) provided in one side face of the operation portion body 3b in a direction orthogonal to the longitudinal axis of the operation portion 3. A cover member 7 is provided over the opening portion (not shown), through which the operation unit 5 protrudes. The cover member 7 is in close contact with the shaft portion 5a of the operation unit 5 while watertightly covering the opening portion. The cover member 7 holds the operation unit 5 so as to allow a tilt operation of the operation unit 5.

The bending portion 2b is configured such that the bending portion 2b can be bent in an arbitrary one of upward, downward, leftward, and rightward directions by pulling or slackening (the operation input-side wire 8a of) the pulling wire 8 (to be described later) in response to a tilt operation with a tilt direction (pointed by an arrow Yu, Yd, Yl, or Yr in Fig. 1) and a tilt angle of the operation unit 5.

In the present embodiment, the bending portion 2b is configured to bend in the four directions, the upward, downward, leftward, and rightward directions. The bending portion 2b, however, may be configured to bend only in the upward and downward directions. The suffixes u, d, l, and r denote the upward, downward, leftward, and rightward directions, in which the bending portion 2b is bent. For example, in the description below, reference character 8u denotes the pulling wire 8 for the upward direction (u), and reference character 9d denotes a member for a C ring for the downward direction (d). Note that the lowercase letter "l" is distinguished from the numeral "1" by writing the lowercase letter "l" in cursive script in the drawings.

For example, a switch 6a which gives, e.g., an instruction for an image pickup motion by the image pickup apparatus (not shown) provided inside the distal end portion 2a, an air/water feeding button 6b, a suction button 6c, and the like are provided at predefined positions of a sheath of the operation portion body 3b, in addition to the operation unit 5. A channel insertion port 6d which communicates with a treatment instrument channel (not shown) which is threaded through and placed in the grasping portion 3a and the insertion portion 2 is provided at a sheath of the grasping portion 3a.

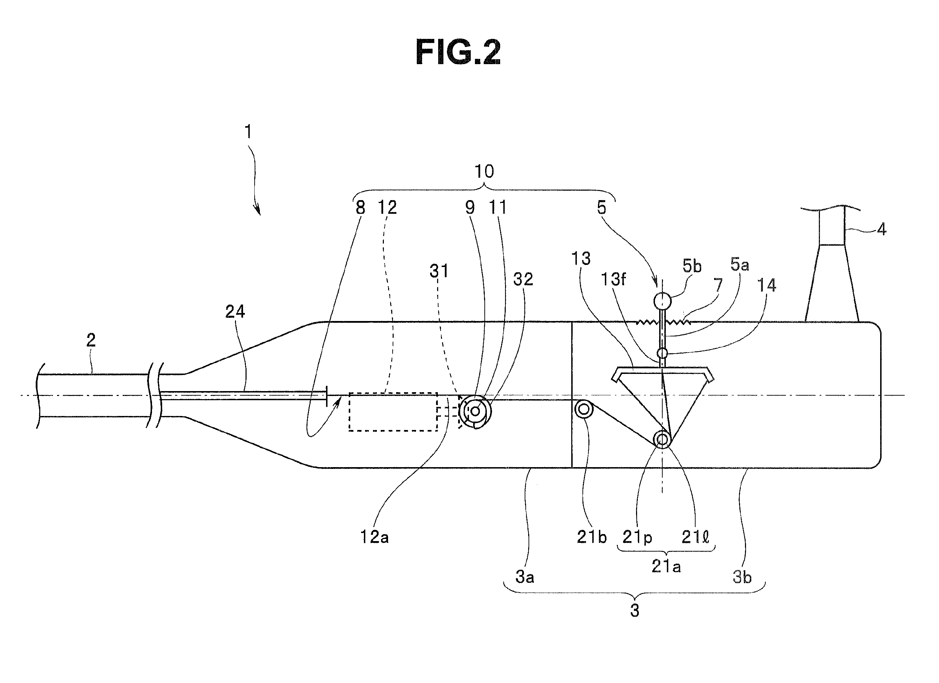

A signal cable which is connected to the above-described image pickup apparatus (not shown), a power line which supplies power to a motor 12 (see Fig. 2) serving as a drive section (to be described later), a light guide cable which conveys illuminating light from the light source apparatus 17, a tube for air feeding, a tube for water feeding, a tube for suction, and the like are threaded through the universal cord 4. A connector 4a is provided at a distal end of the universal cord 4. The control apparatus 15, the display apparatus 16, and the light source apparatus 17 are electrically connected to the connector 4a via respective connection cables. Note that although not shown, the tube for air feeding, the tube for water feeding, the tube for suction, and the like described above are connected to an air/water feeding apparatus, a suction apparatus, and the like via the connector 4a.

The operation unit 5 is provided at a position where an operator grasping the grasping portion 3 a of the operation portion 3 with a left hand in a same manner as a conventional endoscope operates the operation unit 5 with a thumb of the grasping hand of the operator. The air/water feeding button 6b and the suction button 6c are provided at positions where the operator operates with fingers other than the thumb of the grasping hand of the operator. The switch 6a is provided at a position where the operator can operate with the thumb or any other finger of the grasping hand of the operator.

The control apparatus 15 is control means which is configured to have, e.g., a control circuit which comprehensively controls the endoscope 1 according to the present embodiment and the whole endoscope system including the endoscope 1. Thus, the control apparatus 15 also functions as a drive control section which drives and controls the motor 12 serving as the drive section. Note that, alternatively, a motor control section may be disposed inside the endoscope 1 (e.g., inside the operation portion 3).

The display apparatus 16 is composed of, for example, a device for display which is made up of a liquid crystal display apparatus (LCD) monitor or the like, a processor for display which drives the device for display and receives an output signal from the image pickup apparatus (not shown) of the endoscope 1 to generate a video signal for video display by using the device for display, and the like.

The light guide cable (not shown) is connected to the light source apparatus 17 via the connector 4a. The light guide cable is threaded through the universal cord 4, as described above, is further threaded through the operation portion 3 and the insertion portion 2, and arrives at an illuminating light exit window (not shown) at a distal end of the insertion portion 2. With the configuration, illuminating light from the light source apparatus 17 exits forward from the illuminating light exit window at the distal end of the insertion portion 2 via the light guide cable, and a desired subject can be illuminated with the illuminating light.

A portion related to the present invention of an internal configuration of the operation portion 3, i.e., a configuration of a pulling member operation apparatus will be described below with reference to Figs. 2 to 5.

A pulling member operation apparatus 10 for bending the bending portion 2b by operating the operation unit 5 to pull the pulling wire 8 serving as a pulling member is provided inside the operation portion 3.

The pulling member operation apparatus 10 is mainly composed of four pulling wires 8 serving as pulling members, four C ring-shaped members 9, around which midway portions of the pulling wires 8 are respectively wound, a pulley 11 in a hollow cylindrical shape which pivotably holds the C ring-shaped members 9, the motor 12 serving as the drive section that rotates and drives the four C ring-shaped members 9 under a predetermined condition by rotating and driving the pulley 11 with a predetermined rotating torque and generates a driving force for pulling the pulling wires 8 and bending the bending portion 2b, a suspension frame 13 which has wire attaching portions, to which proximal end portions of the four pulling wires 8 are respectively coupled, and is formed in a substantially cross shape, the operation unit 5, the shaft portion 5a of which is integrally coupled to the suspension frame 13, guide roller suites 21a and 21b serving as wire travel path changing members which change travel paths of the four pulling wires 8 inside the operation portion 3 and including a plurality of guide rollers, and the like.

Reference numeral 51 shown in Fig. 4 denotes a signal cable; 52, a light guide cable; 53, a coil pipe stop; and 59, a partition plate. In the present embodiment, a barycenter of the operation portion 3 is located inside the grasping portion 3a.

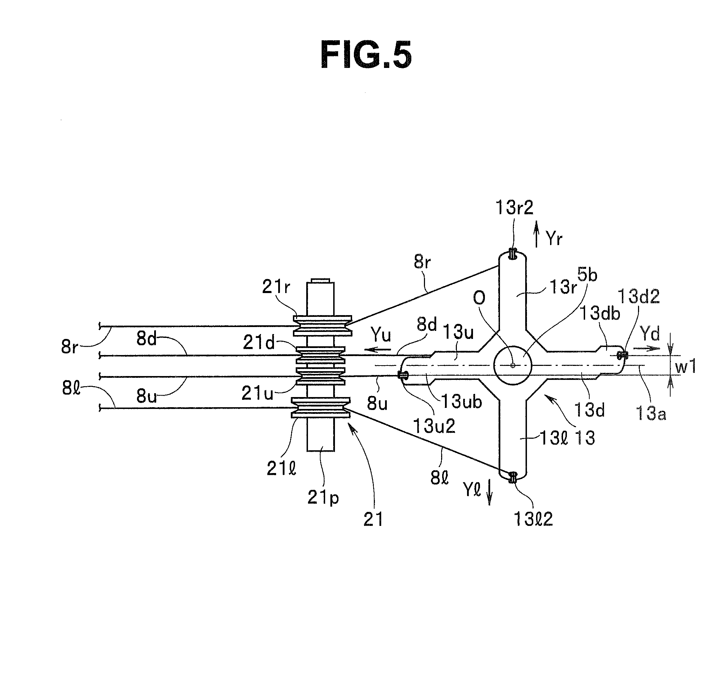

As shown in Figs. 4 and 5, the four pulling wires 8 are composed of one pair of pulling wires for bending operation in the upward and downward directions (the upward pulling wire 8u and a downward pulling wire 8d) and one pair of pulling wires for bending operation in the leftward and rightward directions (a leftward pulling wire 81 and a rightward pulling wire 8r).

In the present embodiment, a longitudinal axis of the pulley 11 and a longitudinal axis of the motor 12 intersect, as shown in Fig. 4. More specifically, a drive shaft 12a of the motor 12 is placed inside the grasping portion 3 a so as to have a parallel positional relationship with the longitudinal axis of the grasping portion 3a. A positional relationship between the motor 12 and the pulley 11 is set such that an imaginary line 12b which is an extension of the drive shaft 12a of the motor 12 and an imaginary line 11b which is an extension of a pulley shaft 11a serving as a rotating shaft of the pulley 11 are orthogonal. The pulley 11 and the motor 12 are placed in two spaces, respectively, into which the operation portion 3 is partitioned by the partition plate 59 serving as an internal fixed member, with the partition plate 59 between the two spaces.

The configuration is such that driving force of the motor 12 is transmitted to the pulley 11 via a driving force transmission mechanism portion 30 which is made up of a first bevel gear 31 and a second bevel gear 32. The first bevel gear 31 is securely provided integrally with a distal end portion of the drive shaft 12a of the motor 12, and the second bevel gear 32 is securely provided integrally with a distal end portion of the pulley shaft 11a of the pulley 11. That is, the drive shaft 12a of the motor 12 and the pulley shaft 11a of the pulley 11 work in conjunction with each other via the driving force transmission mechanism portion 30. Thus, the pulley 11 and the pulley shaft 11a are also included in a drive shaft of the drive section (the motor 12).

With the configuration, driving force of the motor 12 is transmitted to the pulley shaft 11a via the first bevel gear 31 and the second bevel gear 32. The pulley 11 is configured so as to rotate about the pulley shaft 11a with the driving force.

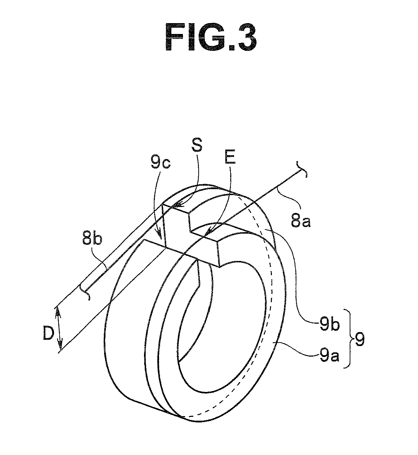

The C ring-shaped members 9 are disposed on an outer circumferential face of the pulley 11 so as to be frictionally engageable with the pulley 11. Each C ring-shaped member 9 is formed of two C ring-shaped members which are elastically deformable, have a notch portion 9c at a portion, and are different in outer diameter. The two C ring-shaped members here are an operation wire extension portion 9a where the operation input-side wire 8a extends and a bending wire extension portion 9b where a bending portion-side wire 8b extends (see Fig. 3).

The bending wire extension portion 9b is formed such that an outer diameter increases gradually from a predetermined part on an outer circumferential face of the operation wire extension portion 9a and is formed to have a part protruding in an outer diameter direction. With the configuration, the outer circumferential face of the smaller-diameter operation wire extension portion 9a and an outer circumferential face of the larger-diameter bending wire extension portion 9b are formed of a continuous outer circumferential face, and a level difference is made between the outer circumferential face of the operation wire extension portion 9a and the outer circumferential face of the bending wire extension portion 9Db. Note that although not shown, a wire guide groove in a circumferential groove shape is provided at the outer circumferential faces of the operation wire extension portion 9a and the bending wire extension portion 9b.

The provision of the wire guide groove allows the pulling wire 8 to be smoothly placed to be wound around the outer circumferential face of the C ring-shaped member 9 from a wrap start position (reference character E in Fig. 3) to a wrap end position (reference character S in Fig. 3) when the pulling wire 8 is to be wound around an outer circumferential face of the C ring-shaped member 9. In the case, the pulling wire 8 is placed in the wire guide groove and does not come off.

The pulling wire 8 is placed so as to extend from a part denoted by reference character S of the bending wire extension portion 9b toward the bending portion 2b. The pulling wire 8 is also placed so as to extend from a part denoted by reference character E of the operation wire extension portion 9a toward the operation input portion.

Note here that an operation portion-side part and a bending portion-side part in the pulling wire 8 are referred to as the operation input-side wire 8a serving as a first pulling member and an operation input-side pulling member and the bending portion-side wire 8b serving as a second pulling member and a bending portion-side pulling member, respectively.

That is, the pulling wire 8 is placed to be wound around a C ring-shaped member. In the case, the operation input-side wire 8a of the pulling wire 8 is a first pulling member which extends from the C ring-shaped member 9 toward the operation unit 5 (the operation input member). The operation input-side wire 8a is a part on the outer circumferential face of the C ring-shaped member 9 and is wrapped around and placed on the outer circumferential face of the C ring-shaped member 9 across the notch portion 9c from the extension position E toward the operation unit 5 (the operation input member) to a predetermined part. A distance of wrap around the C ring-shaped member 9 is set so as to decrease with increase in an amount by which the operation unit 5 is operated because the operation input-side wire 8a is pulled toward the operation unit 5. An end portion of the operation input-side wire 8a is coupled to the suspension frame 13 (the operation input member).

In contrast, the bending portion-side wire 8b of the pulling wire 8 is a second pulling member which extends from the C ring-shaped member 9 toward the bending portion 2b and is a bending portion-side pulling member. The bending portion-side wire 8b is disposed so as not to wrap around the outer circumferential face of the C ring-shaped member 9 across the notch portion 9c from the extension position S toward the bending portion 2b on the C ring-shaped member 9. An end portion of the bending portion-side wire 8b is coupled to the bending portion 2b.

In the case, the C ring-shaped member 9 is formed such that a level difference D is produced in a radial direction of the C ring-shaped member 9 between a part denoted by reference character S of the bending wire extension portion 9b and a part denoted by reference character E of the operation wire extension portion 9a. The C ring-shaped member 9 is formed to have the notch portion 9c that stretches from the operation wire extension portion 9a to the bending wire extension portion 9b and extends in a width direction in a neighborhood of a part where the level difference D is at a maximum.

With the above-described configuration, the C ring-shaped member 9 is in the state shown in Fig. 3, i.e., a state in which the pulling wire 8 is wound around the outer circumferential face. When the pulling wire 8 is pulled toward the operation portion, the C ring-shaped member 9 is narrowed at the notch portion 9c against elastic force of the C ring-shaped member 9 and is reduced in diameter.

Four C ring-shaped members 9 are prepared corresponding to the four pulling wires 8 (8u, 8d, 81, and 8r) for bending the bending portion 2b in the upward, downward, leftward, and rightward directions, respectively, as shown in Fig. 4. That is, four C ring-shaped members 9u, 9d, 91, and 9r are placed side by side on the outer circumferential face of the pulley 11 in a predefined loosely-fitting state and are rotatable independently. Note that the four C ring-shaped members 9 (9u, 9d, 91, and 9r) are placed in the order of reference characters 9r, 9d, 9u, and 91 from a side where driving force is inputted to the pulley 11, i.e., from a side with the second bevel gear 32.

The suspension frame 13 is placed in an internal space of the operation portion body 3b, as shown in Fig. 2. As shown in Fig. 5, the suspension frame 13 is configured in a substantially cross shape to include four frames 13u, 13d, 131, and 13r having equal lengths from a center O to respective end portions. The upward frame 13u and the downward frame 13d corresponding to the one pair of pulling wires 8u and 8d are placed in line with each other with the shaft portion 5a of the operation unit 5 between the frames. An upward wire attaching portion 13u2 is provided at an end portion of the upward frame 13u, and a downward wire attaching portion 13d2 is provided at an end portion of the downward frame 13d.

An upward frame distal end crooked portion 13ub which is curved in one direction with respect to an upward and downward frame center line 13a is provided at the end portion of the upward frame 13u, and a downward frame distal end crooked portion 13db which is curved in the other direction with respect to the upward and downward frame center line 13a is provided at the end portion of the downward frame 13d. The upward wire attaching portion 13u2 is provided at the upward frame distal end crooked portion 13ub, and the downward wire attaching portion 13d2 is provided at the downward frame distal end crooked portion 13db. A spacing w1 in a direction orthogonal to the longitudinal axis of the operation portion 3 between the upward wire attaching portion 13u2 and the downward wire attaching portion 13d2 is set to a predefined dimension.

The leftward frame 131 and the rightward frame 13r corresponding to the one pair of pulling wires 8l and 8r are orthogonal to the upward and downward frame center line 13a and are placed in line with each other with the shaft portion 5a between the frames. A leftward wire attaching portion 1312 is provided at an end portion of the leftward frame 131, and a rightward wire attaching portion 13r2 is provided at an end portion of the rightward frame 13r.

The upward frame 13u, the upward wire attaching portion 13u2, and the like are set in consideration of the tilt direction of the operation unit 5 and a bending direction of the bending portion 2b. That is, the present embodiment is configured such that, when the operation unit 5 is tilted in a direction of the arrow Yu in Fig. 1, the upward wire attaching portion 13u2 swings to be inclined in a direction of an arrow Yu in Fig. 5, and the bending portion 2b bends in the upward direction. When the operation unit 5 is similarly tilted in a direction of an arrow Yd in Fig. 1, the downward wire attaching portion 13d2 swings to be inclined in a direction of an arrow Yd in Fig. 5, and the bending portion 2b bends in the downward direction. When the operation unit 5 is tilted in a direction of an arrow Yl in Fig. 1, the leftward wire attaching portion 1312 swings to be inclined in a direction of an arrow Yl in Fig. 5, and the bending portion 2b bends in the leftward direction. When the operation unit 5 is tilted in a direction of an arrow Yr in Fig. 1, the rightward wire attaching portion 13r2 swings to be inclined in a direction of an arrow Yr in Fig. 5, and the bending portion 2b bends in the rightward direction. In the present embodiment, the suspension frame 13 is placed inside the operation portion 3 such that the upward and downward frame center line 13a and the longitudinal axis of the grasping portion 3a are parallel.

The guide roller suites 21a and 21b (see Figs. 2 and 5; note that only the one guide roller 21a is shown in Fig. 5) serve as support bodies. The guide roller suites 21a and 21 b are each configured to include, for example, a roller shaft 21p in a solid cylindrical shape and four guide rollers 21 u, 21 d, 21l, and 21r which are pivotably placed on the roller shaft 21p. The four guide rollers 21u, 21d, 211, and 21r correspond to the four pulling wires 8u, 8d, 81, and 8r and are spaced apart from the pulley 11 and the suspension frame 13 by predefined distances.

The four guide rollers 21 u, 21 d, 211, and 21r serve as attachment path setting members which guide the four pulling wires 8u, 8d, 81, and 8r to the wire attaching portions 13u2, 13d2, 1312, and 13r2 of the suspension frame 13.

The roller shaft 21p of the guide roller suite 21a has such a positional relationship that the roller shaft 21p is orthogonal to the longitudinal axis of the grasping portion 3a and is placed immediate below the shaft portion 5a. A center of the roller shaft 21p is located on a central axis of the shaft portion 5a in an upright state.

In the present embodiment, the travel paths of the pulling wires 8 extending from the suspension frame 13 are first changed by the guide roller suite 21 a and are then changed by the guide roller suite 21b. With the configuration, the pulling wires 8 (8u, 8d, 81, and 8r) run from the C ring-shaped members 9 (9u, 9d, 91, and 9r) to the respective wire attaching portions (13u2, 13d2, 1312, and 13r2) of the suspension frame 13.

Note that a position of the suspension frame 13 and a position of the roller shaft 21p of the guide roller suite 21 a are shifted from each other in Fig. 5 for explaining a positional relationship between the pulling wires 8u, 8d, 81, and 8r and the wire attaching portions 13u2, 13d2, 1312, and 13r2.

As shown in Fig. 5, the guide rollers 21 u, 21 d, 211, and 21r are placed on the roller shaft 21 p in the order of reference characters 21r, 21 d, 21 u, and 211 from one end.

The guide rollers 21r and 211 placed at two ends of the roller shaft 21p are configured to be different in radial dimension or width dimension from the guide rollers 21 u and 21 d that are placed inside the guide rollers 21r and 211 with the center of the roller shaft 21 p between the guide rollers 21 u and 21d. In the present embodiment, the radial dimensions and the width dimensions of the guide rollers 211 and 21r are set to predefined dimensions so as to be larger than the radial dimensions and the width dimensions of the guide rollers 21 u and 21d.

Travel paths of the pulling wires 8u, 8d, 81, and 8r inside the operation portion 3 will be described with reference to Figs. 2, 4, and 5. As shown in Fig. 5, proximal end portions of the four pulling wires 8u, 8d, 81, and 8r are fixed to predefined positions of the suspension frame 13, i.e., the wire attaching portions 13u2, 13d2, 1312, and 13r2.

Distal end portions of the pulling wires 8u, 8d, 81, and 8r are threaded through four guide members 24 (see Fig. 4) which are made of, for example, metal, are each formed of a coil pipe having a through-hole, through which a wire is capable of being threaded to freely advance and retract, and are provided corresponding to the respective wires and are fixed to corresponding upper, lower, left, and right positions of a distal end bending piece (not shown) constituting the bending portion 2b. The distal end bending piece is a bending piece constituting a most distal end of a bending portion suite that is configured to bend in the upward, downward, leftward, and rightward directions by linking a plurality of bending pieces (not shown) constituting the bending portion 2b.

The pulling wires 8u, 8d, 81, and 8r, the distal ends of which are fixed to the distal end bending piece, are extended into the operation portion 3 via the guide members 24. The pulling wires 8u, 8d, 81, and 8r are wound around the C ring-shaped members 9u, 9d, 91, and 9r that are placed on the pulley 11 in a slackened state.

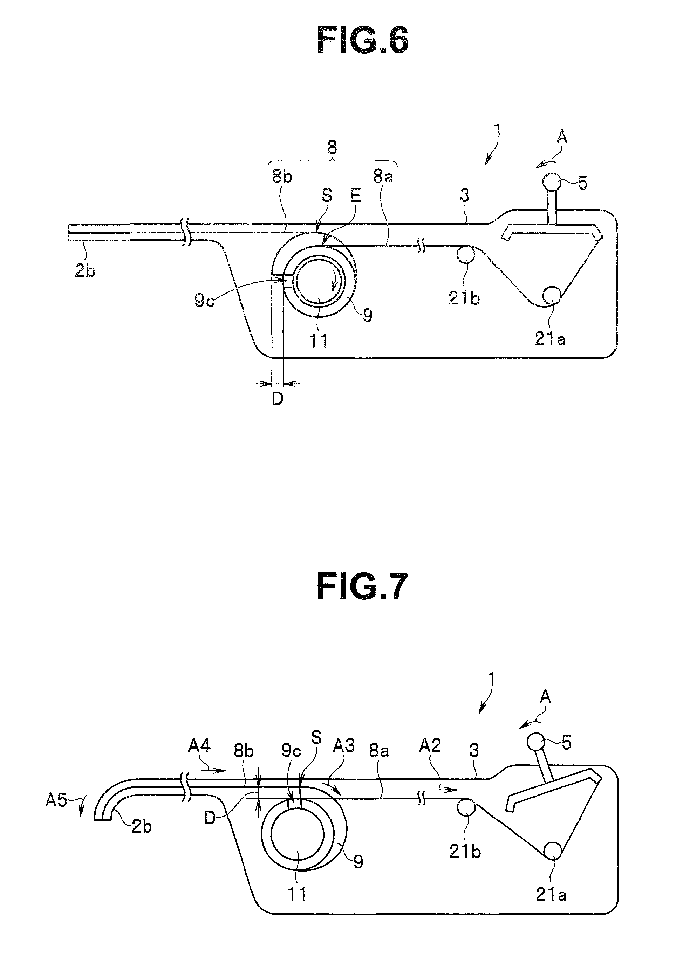

That is, the pulling wires 8u, 8d, 81, and 8r are wound around outer circumferential faces, each stretching from the bending wire extension portion 9b to the operation wire extension portion 9a, of the C ring-shaped members 9u, 9d, 91, and 9r from the respective predetermined parts S (see Fig. 3) such that the pulling wires 8u, 8d, 81, and 8r are in a predefined slackened state. The pulling wires 8u, 8d, 81, and 8r are led out from the predetermined parts E (see, e.g., Figs. 3 and 6) toward the respective guide rollers of the guide roller suite 21 b. After the travel paths are changed by the individual guide rollers of the guide roller suites 21b and 21a, the pulling wires 8u, 8d, 81, and 8r are guided to the respective wire attaching portions of the suspension frame 13.

The pulling wires 8u, 8d, 81, and 8r that are led out from the C ring-shaped members 9u, 9d, 91, and 9r are guided by the guide roller suites 21b and 21 a, which changes the wire travel paths. The respective proximal end portions of the pulling wires 8u, 8d, 81, and 8r are fixed to the respective corresponding wire attaching portions 13u2, 13d2, 1312, and 13r2 of the suspension frame 13.

Note that the shaft portion 5a of the operation unit 5 and a frame convex portion 13f (see Fig. 2) serving as a central shaft of the suspension frame 13 are coaxially attached and fixed to each other via a universal joint 14 (see Fig. 2) which is pivotably disposed at a frame (not shown). When the shaft portion 5a of the operation unit 5 is in an upright state (the state in Fig. 2), i.e., the pulling members are in an unloaded state, the pulling wires 8u, 8d, 8l, and 8r extending from the guide rollers 21u, 21 d, 21l, and 21r of the guide roller suite 21 a toward the suspension frame 13 are all in the predetermined slackened state.

Note that, in the present embodiment, each pulling wire 8 is composed of one wire continuous from a predetermined fixed position at a corresponding one of the individual wire attaching portions 13u2, 13d2, 1312, and 13r2 of the suspension frame 13 to a predetermined fixed position of the distal end bending piece of the bending portion 2b.

In the pulling wire 8, a part, for example from the C ring-shaped member 9 to an operation input side, i.e., the suspension frame 13 is the operation input-side wire 8a. A part from the C ring-shaped member 9 to a bending portion side, i.e., the distal end bending piece is the bending portion-side wire 8b.

As described above, the pulling wire 8 is wound around the C ring-shaped member 9 from the predetermined wrap start position E to the wrap end position S (see Fig. 3). In the case, reference character S in Fig. 3 denotes an extension position for the bending portion-side wire 8b in the pulling wire 8, and reference character E in Fig. 3 denotes an extension position for the operation input-side wire 8a in the pulling wire 8.

A configuration of the endoscope 1 according to the present embodiment has been described above. Note that various constituent members for performing basic functions of an endoscope are provided inside the operation portion 3, in addition to the pulling member operation apparatus 10, the motor 12 serving as the drive section, and the like. The various constituent members, however, are not directly related to the present invention. Thus, the endoscope 1 according to the present embodiment is assumed to have same various constituent members as a conventional endoscope, and a detailed description and a graphical expression of the constituent members are omitted.

Action of the endoscope 1 according to the present embodiment with the above-described configuration will be described below mainly with reference to Figs. 6 to 8 and the like. Note that Figs. 6 to 8 are conceptual views conceptually showing a configuration of the present invention shown for avoiding increase in complexity of the drawings. For example, the C ring-shaped members 9, the pulleys 11, and the like are actually disposed for four sets so as to correspond to bending motions in the upward, downward, leftward, and rightward operations. However, only one set of the C ring-shaped member 9, the pulley 11, and the like are illustrated, and a motion in one corresponding direction will only be described, for simplicity of illustration and description. Same applies to motions in other directions.

For example, action when the bending portion 2b is caused to make a bending motion in the downward direction will be described below.

The above-described endoscope system is energized and activated. Upon the activation, the control apparatus 15 or the motor control section (not shown) provided inside the operation portion 3 drives the motor 12. Driving force of the motor 12 is transmitted to the pulley 11 via the first bevel gear 31 and the second bevel gear 32 of the driving force transmission mechanism portion 30. The transmission puts the pulley 11 into a state rotating at all times. In the state, the operation unit 5 is at a neutral position in an upright state, and the pulling wires 8 are in an unloaded state, as shown in Figs. 2 and 6. An operator tilts and operates the shaft portion 5a in the direction of the arrow Yu in Fig. 1 (a direction of an arrow A in Figs. 6 and 7) while grasping the grasping portion 3a with a left hand and causing a pad of a thumb to abut on the finger touch portion 5b of the operation unit 5. With the tilt operation of the operation unit 5, the suspension frame 13 inclines, and the upward pulling wire 8d fixed to the downward wire attaching portion 13d2 in a slack state is gradually drawn in a direction of an arrow A2 in Fig. 7. The other pulling wires 8u, 81, and 8r change to a slacker state.

Of the pulling wires 8u, 8d, 8l, and 8r wound around the C ring-shaped members 9u, 9d, 9l, and 9r of the pulley 11 in a slackened state, only the downward pulling wire 8d is pulled. The downward pulling wire 8d narrows the notch portion 9c of the downward C ring-shaped member 9d against elastic force to reduce the downward C ring-shaped member 9d in diameter, and the downward C ring-shaped member 9u and the pulley 11 are brought into close contact. The close contact generates frictional resistance between the downward C ring-shaped member 9d and the pulley 11, which causes the downward C ring-shaped member 9d to be rotated in a direction equal to a direction of the pulley 11, i.e., in a direction of an arrow A3 in Fig. 7 while gliding with respect to the pulley 11. The rotation causes a part (i.e., the bending portion-side wire 8b) of the downward pulling wire 8d which is placed closer to the insertion portion 2 (the bending portion 2b) than the downward C ring-shaped member 9d to be pulled and moved in a direction of an arrow A4 in Fig. 7. With the movement, the bending portion 2b starts a motion of bending in a direction of an arrow A5 in Fig. 7 (referred to as the downward direction).

The operator continues to tilt and operate the shaft portion 5a in the above-described direction so as to bring the downward C ring-shaped member 9d into close contact with the pulley 11. The tilt operation further increases frictional force of the downward C ring-shaped member 9d in close contact with the pulley 11, further pulls and moves a portion of the downward pulling wire 8d (the bending portion-side wire 8b) which is placed closer to the insertion portion 2 (the bending portion 2b) than the downward C ring-shaped member 9d, and further bends the bending portion 2b in the above-described direction (the direction of the arrow A5 in Fig. 7). When the operator continues holding a tilt position of the operation unit 5, adhesion between the downward C ring-shaped member 9d and the pulley 11 is maintained. In a state in which tensile force is generated in the portion of the downward pulling wire 8d placed closer to the distal end side than the downward C ring-shaped member 9d, the pulling and movement of the downward pulling wire 8d are stopped. At the time, the other pulling wires 8u, 8l, and 8r are in a slackened state. By holding the operation unit 5 in the tilted and operated state, the downward pulling wire 8d is held in a drawn state, the pulling wires 8u, 81, and 8r are held in a slackened state, and the bending portion 2b is held in a state bent in the above-described direction (the direction of the arrow A5 in Fig. 7).

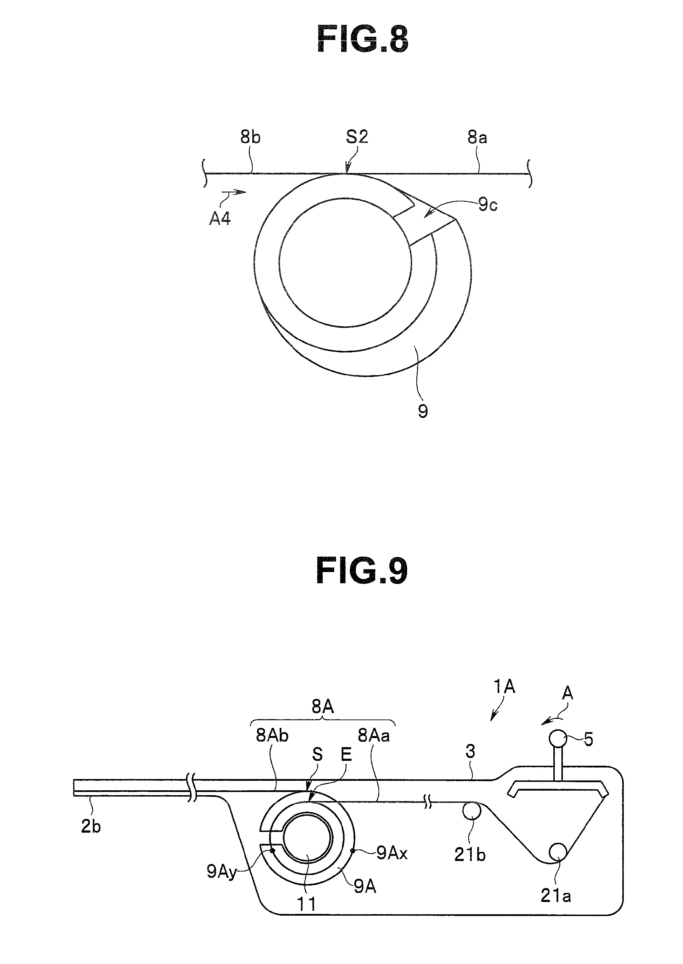

The level difference D is made in the C ring-shaped member 9. Even if the C ring-shaped member 9 rotates and changes from the state in Fig. 6 to the state in Fig. 7, the bending portion-side wire 8b that is extended from the extension position denoted by reference character S of the bending wire extension portion 9b does not interfere with the outer circumferential face of the operation wire extension portion 9a. Note that when the C ring-shaped member 9 in the state in Fig. 7 rotates further and changes to, for example, the state shown in Fig. 8, the bending portion-side wire 8b comes into contact with the outer circumferential face of the operation wire extension portion 9a at a part S2. If the C ring-shaped member 9 enters the state in Fig. 8, the bending portion-side wire 8b may block a diameter reduction action of the C ring-shaped member 9, and necessary frictional force may not be obtained. When the amount of force applied to the operation unit 5 is reduced in the state, the bending portion-side wire 8b may also block a diameter reduction release action of the C ring-shaped member 9, and necessary frictional force may not be similarly obtained.

Hence, a pivot range of the C ring-shaped member 9 according to the present embodiment is set such that the C ring-shaped member 9 pivots between the state in Fig. 6 and the state in Fig. 7. That is, the pivot range of the C ring-shaped member 9 is set so as to prevent the bending portion-side wire 8b from wrapping around an outer surface of the C ring-shaped member 9 across the notch portion 9c from the extension position (reference character S) toward the bending portion 2b on the C ring-shaped member 9 (the state in Fig. 8) when the operation unit 5 in the state in Fig. 6 (the operation unit 5 is at the neutral position) is tilted and operated, the operation input-side wire 8a is pulled, and the C ring-shaped member 9 rotates.

If the operator releases the tilt operation of the operation unit 5, the operation unit 5 returns to the neutral position where the shaft portion 5a is in an upright state by restoring force of the operation unit 5. Upon the return, the downward pulling wire 8d being drawn enters a slackened state like the other pulling wires 8u, 8l, and 8r, and the bending portion 2b is released from a bent state.

To briefly describe the above-described action, the endoscope 1 according to the present embodiment is configured such that the bending portion 2b can be bent in a desired direction by an operator's tilt operation of the operation unit 5, as described above. In the case, tilt operation of the operation unit 5 pulls the pulling wire 8. When the pulling wire 8 is pulled, the pulling wire 8 acts to tighten the C ring-shaped member 9. The C ring-shaped member 9 is placed on the outer circumferential face of the pulley 11 in a slackened state. When the pulling wire 8 acts to tighten the C ring-shaped member 9, the C ring-shaped member 9 transits from a slackened state on the pulley 11 to a tightened state according to the amount of pulling of the pulling wire 8, i.e., the tilt angle of the operation unit 5. As described above, the pulley 11 is constantly rotating. When the C ring-shaped member 9 shifts to a state tightening the pulley 11, frictional force generated between the C ring-shaped member 9 and the pulley 11 rotates the C ring-shaped member 9 by a predetermined rotation amount. The rotation bends the bending portion 2b.

The C ring-shaped member 9 is provided between the motor 12 serving as the drive section and the pulling wire 8 serving as a pulling member. The C ring-shaped member 9 is a constituent member capable of switching from a state in which driving force is not transmitted to the pulling wire 8 (a state in which the C ring-shaped member 9 is slackened on the pulley 11) to a state in which driving force is transmitted to the pulling wire 8, in conjunction with a pulling motion by the pulling wire 8.

As has been described above, according to the above-described first embodiment, the C ring-shaped member 9 in the pulling member operation apparatus 10 is shaped to be formed of the operation wire extension portion 9a and the bending wire extension portion 9b different in outer diameter, the operation input-side wire 8a is placed on the smaller-diameter operation wire extension portion 9a, and the bending portion-side wire 8b is placed on the larger-diameter bending wire extension portion 9b. With the configuration, a pulling distance of the bending portion-side wire 8b placed on the larger-diameter bending wire extension portion 9b can be made longer.

In the C ring-shaped member 9, the level difference portion D derived from a difference in outer diameter is made between the position (reference character E) where the operation input-side wire 8a (the operation input-side pulling member) starts to wrap around the C ring-shaped member 9 and the position (reference character S) where the bending portion-side wire 8b (the bending portion-side pulling member) extends from the C ring-shaped member 9. The operation input-side wire 8a (the operation input-side pulling member) coupled to the operation unit 5 (the operation input member) is set such that a wrap distance, over which the operation input-side wire 8a wraps around the operation wire extension portion 9a of the C ring-shaped member 9 across the notch portion 9c from the extension part (reference character E) toward the operation input member on the C ring-shaped member 9, decreases with increase in the amount of operation of the operation unit 5 (the operation input member). The bending portion-side wire 8b (the bending portion-side pulling member) is coupled to the bending portion 2b so as not to wrap around the C ring-shaped member 9 across the notch portion 9c from the extension part (reference character E) toward the bending portion 2b on the C ring-shaped member 9. In other words, the bending portion-side wire 8b (the bending portion-side pulling member) is placed so as not to cross the notch portion 9c of the C ring-shaped member 9.

With the above-described configuration, in the endoscope 1 according to the present embodiment, the bending portion 2b can make a reliable bending motion without blocking of the diameter reduction action and the diameter reduction release action after a diameter reduction of the C ring-shaped member 9 by the bending portion-side wire 8b (the bending portion-side pulling member).

### [Second Embodiment]

Figs. 9 to 11 are views showing a second embodiment of the present invention.

The present embodiment has a substantially same basic configuration as that of the above-described first embodiment and is different from the first embodiment only in configurations of a C ring-shaped member and a pulling member in a pulling member operation apparatus. Thus, in the description below, same components as those of the first embodiment are denoted by equal reference numerals, a description of the components will be omitted, and only different components will be described in detail.

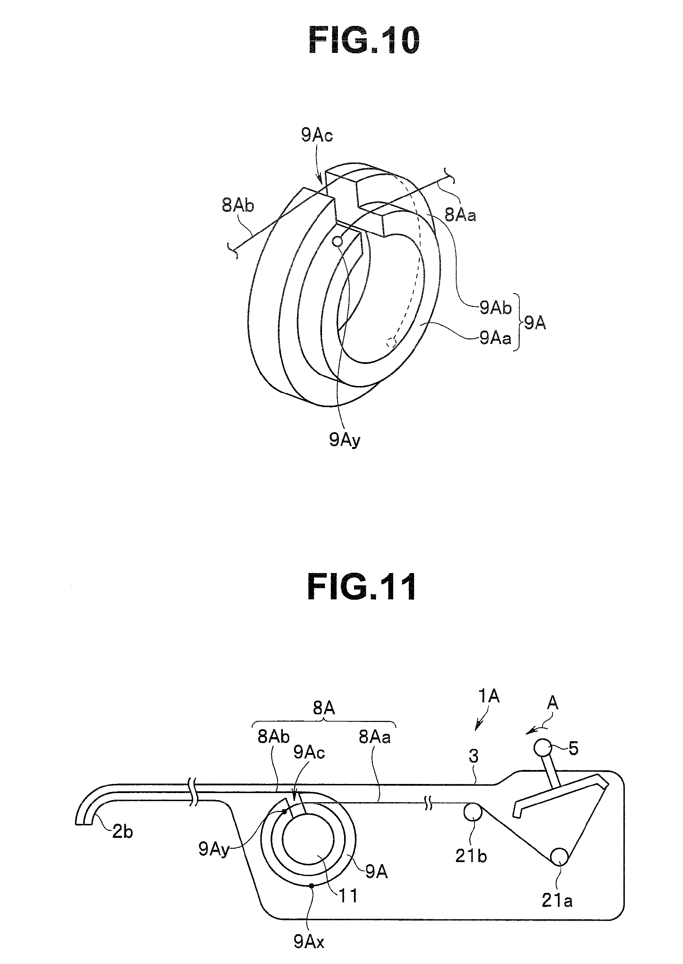

As shown in Figs. 9 and 10, a C ring-shaped member 9A in a pulling member operation apparatus of an endoscope 1 A according to the present embodiment is formed such that two C ring-shaped members which have different outer diameters and have a notch portion 9Ac at a portion, i.e., an operation wire extension portion 9Aa and a bending wire extension portion 9Ab are coaxially stacked on top of another. Of the operation wire extension portion 9Aa and the bending wire extension portion 9Ab, the operation wire extension portion 9Aa is formed to have a smaller diameter, and the bending wire extension portion 9Ab is formed to have a larger diameter. Thus, the C ring-shaped member 9A is formed to have a level difference corresponding to a difference in outer diameter between an outer circumferential face of the operation wire extension portion 9Aa and an outer circumferential face of the bending wire extension portion 9Ab over a whole circumference.

That is, in the C ring-shaped member 9 according to the first embodiment, the outer circumferential face of the operation wire extension portion 9Aa and the outer circumferential face of the bending wire extension portion 9Ab are formed of a continuous outer circumferential face and are formed to have a level difference. The C ring-shaped member 9A according to the present embodiment is different from the C ring-shaped member 9 in that the outer circumferential face of the operation wire extension portion 9Aa and the outer circumferential face of the bending wire extension portion 9Ab are formed independently of each other and that a level difference is made between the outer circumferential faces over the whole circumference. Additionally, a pulling member according to the present embodiment is composed of two wire members, an operation input-side wire 8Aa serving as an operation input-side pulling member and a bending portion-side wire 8Ab serving as a bending portion-side pulling member.

One end of the operation input-side wire 8Aa is coupled to (a pulling wire attaching portion of a suspension frame of) an operation unit 5, and the other end is fixed and placed at a predetermined part (reference character 9Ay) on the outer circumferential face of the operation wire extension portion 9Aa of the C ring-shaped member 9A. The part (reference character 9Ay) where the other end of the operation input-side wire 8Aa is fixed and placed is a part on the outer circumferential face of the operation wire extension portion 9Aa which is across the notch portion 9Ac from a part (reference character E) where the operation input-side wire 8Aa extends toward an operation input side, as shown in Figs. 9 to 11. In the present embodiment, the part (reference character 9Ay) where the other end of the operation input-side wire 8Aa is fixed and placed is set to a part to which the operation input-side wire 8Aa is wrapped in a circular circumferential direction across the notch portion 9Ac and a notch portion 9Eca, a neighborhood of the notch portion 9Ac.

One end of the bending portion-side wire 8Ab is coupled to a bending portion 2b, and the other end is fixed and placed at a predetermined part (reference character 9Ax) on the outer circumferential face of the bending wire extension portion 9Ab of the C ring-shaped member 9A. The part (reference character 9Ax) where the other end of the bending portion-side wire 8Ab is fixed and placed is a part to which the bending portion-side wire 8Ab does not wrap around the C ring-shaped member 9 across the notch portion 9Ac from a part (reference character S) where the bending portion-side wire 8Ab extends toward the bending portion, as shown in Figs. 9 to 11. Other components are substantially same as in the first embodiment.

In the endoscope 1A according to the present embodiment with the above-described configuration as well, when the operation unit 5 in the neutral state in Fig. 9 is tilted, for example, in a direction of an arrow A shown in Fig. 11, the bending portion 2b bends in a predetermined direction in a substantially same manner as in the first embodiment.

As has been described above, according to the above-described second embodiment, substantially same effects as those of the first embodiment can be obtained.

### [Third Embodiment]

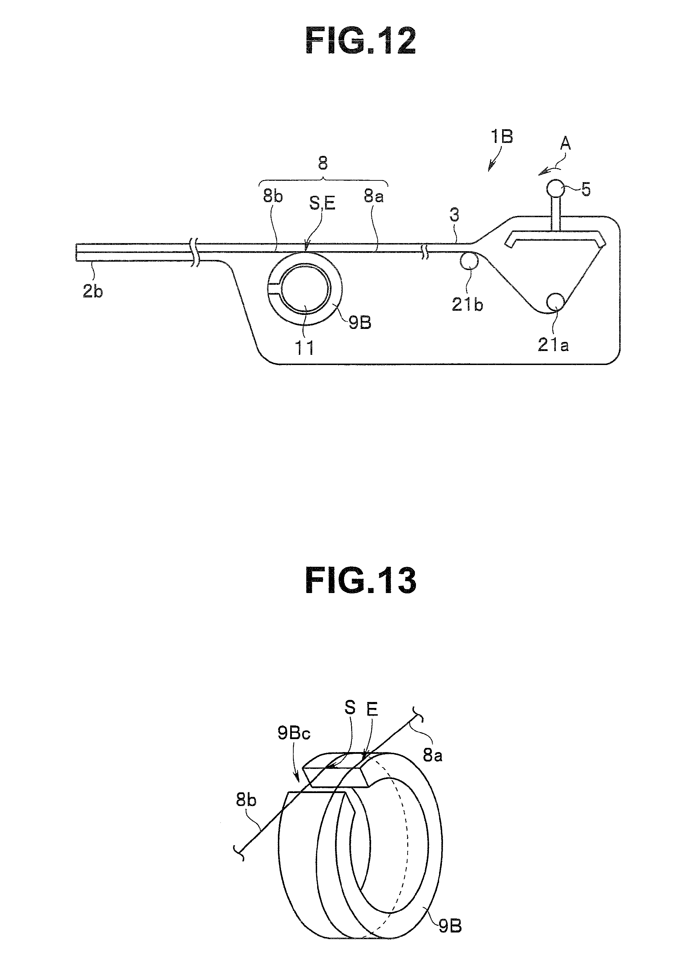

Figs. 12 to 14 are views showing a third embodiment of the present invention.

The present embodiment has a substantially same basic configuration as the above-described first embodiment and is different from the first embodiment only in a configuration of a C ring-shaped member in a pulling member operation apparatus. Thus, in the description below, same components as those of the first embodiment are denoted by equal reference numerals, a description of the components will be omitted, and only different components will be described in detail.

As shown in Figs. 12 and 13, a C ring-shaped member 9B in a pulling member operation apparatus of an endoscope 1B according to the present embodiment is made up of a single C ring-shaped member having a notch portion 9Bc at a portion. Thus, the C ring-shaped member 9B according to the present embodiment is different from the C ring-shaped members 9 and 9A according to the above-described first and second embodiments and is formed as an integrated form of an operation wire extension portion and a bending wire extension portion.

That is, the C ring-shaped member 9B is formed as an integrated form of an operation wire extension portion from which an operation input-side wire 8a extends and a bending wire extension portion from which a bending portion-side wire 8b extends, and a single continuous outer circumferential face is formed. There is no level difference between the operation wire extension portion and the bending wire extension portion.

A pulling member according to the present embodiment is composed of one pulling wire 8, as in the first embodiment. Of the pulling wire 8, a part (reference character E) which extends from the C ring-shaped member 9B toward an operation input side will be referred to as the operation input-side wire 8a. Similarly, a part (reference character S) which extends from the C ring-shaped member 9B toward a bending portion will be referred to as the bending portion-side wire 8b.

In the present embodiment, one end of the operation input-side wire 8a is coupled to (a pulling wire attaching portion of a suspension frame of) an operation unit 5. A portion to a position (reference character E) where the pulling wire 8 starts to wrap around the C ring-shaped member 9B corresponds to the operation input-side wire 8a. One end of the bending portion-side wire 8b is coupled to a bending portion 2b. A portion to a position (reference character S; a wrap end position) where the pulling wire 8 starts to wrap around the C ring-shaped member 9B corresponds to the bending portion-side wire 8b.

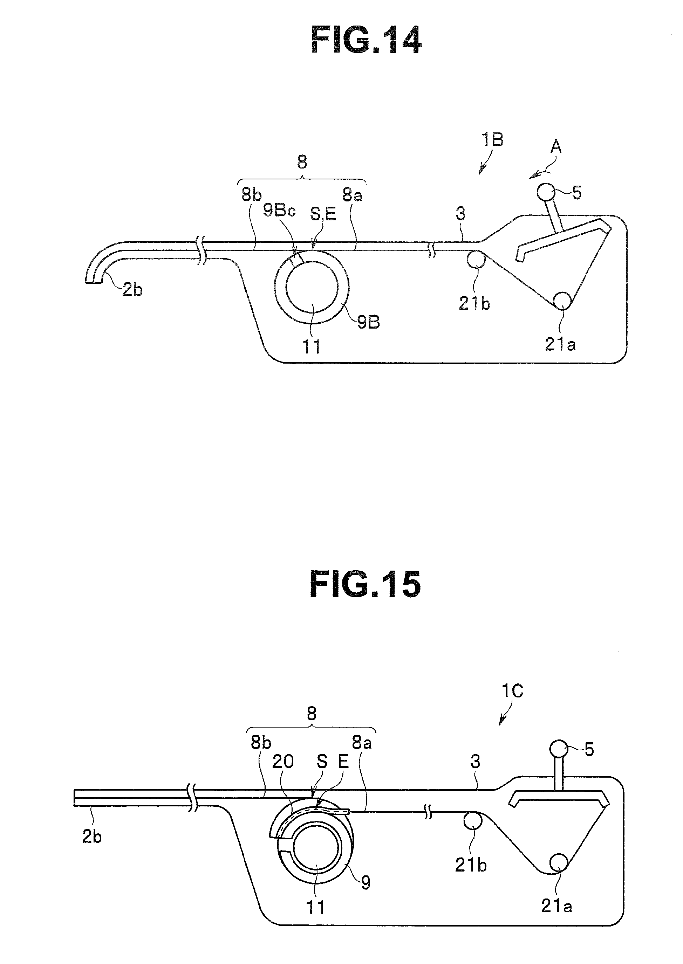

In the case, a range between the state shown in Fig. 12 (the operation unit 5 is at a neutral position) and the state shown in Fig. 14 (the operation unit 5 is at a maximum tilt position) is set as a pivot range of the C ring-shaped member 9B. That is, when the C ring-shaped member 9B is put into the state shown in Fig. 14, the bending portion 2b is at a maximum bending angle.

For example, the pivot range of the C ring-shaped member 9B is set so as to prevent the bending portion-side wire 8b from wrapping around the C ring-shaped member 9B across the notch portion 9Bc from the extension position (reference character S) toward the bending portion 2b on the C ring-shaped member 9B (the state in Fig. 14) when the operation unit 5 in the state in Fig. 12 (the operation unit 5 is at the neutral position) is tilted and operated, the operation input-side wire 8a is pulled, and the C ring-shaped member 9B rotates. Other components are substantially same as in the above-described first embodiment.

In the endoscope 1B according to the present embodiment with the above-described configuration as well, when the operation unit 5 in the neutral state in Fig. 12 is tilted, for example, in a direction of an arrow A shown in Fig. 14, the bending portion 2b bends in a predetermined direction in a substantially same manner as in the first embodiment.

As has been described above, according to the above-described third embodiment, substantially same effects as those of the first embodiment can be obtained. Note that the C ring-shaped member 9B does not have a level difference in the present embodiment, unlike the first embodiment. In regard to the point, the third embodiment is different from the first embodiment in an effect of making a pulling distance longer.

### [Fourth Embodiment]

Figs. 15 to 18 are views showing a fourth embodiment of the present invention.

The present embodiment has a substantially same basic configuration as the above-described first embodiment and is different from the first embodiment only in that a coil spring is further disposed at a predetermined part of a pulling member in a pulling member operation apparatus. Thus, in the description below, same components as those of the first embodiment are denoted by equal reference numerals, a description of the components will be omitted, and only different components will be described in detail.

Configurations of a C ring-shaped member 9 and a pulling wire 8 in a pulling member operation apparatus of an endoscope 1C according to the present embodiment are exactly same as those of the first embodiment.

In the present embodiment, a coil pipe member 20 which is a coil-shaped tubular member is placed on an outer circumferential face of an operation wire extension portion 9a of the C ring-shaped member 9 and in a neighborhood of a notch portion 9c with one end securely provided. The pulling wire 8 is threaded through the coil pipe member 20. The coil pipe member 20 has a length corresponding to a region of wrapping around the C ring-shaped member 9 beginning at a position (S) where the operation input-side wire 8Aa starts to wrap around the C ring-shaped member 9. The operation input-side wire 8a of the pulling wire 8 is thus configured so as not to come into direct contact with an outer circumferential face of the C ring-shaped member 9 in a neighborhood of a position where the operation input-side wire 8a extends from the C ring-shaped member 9.

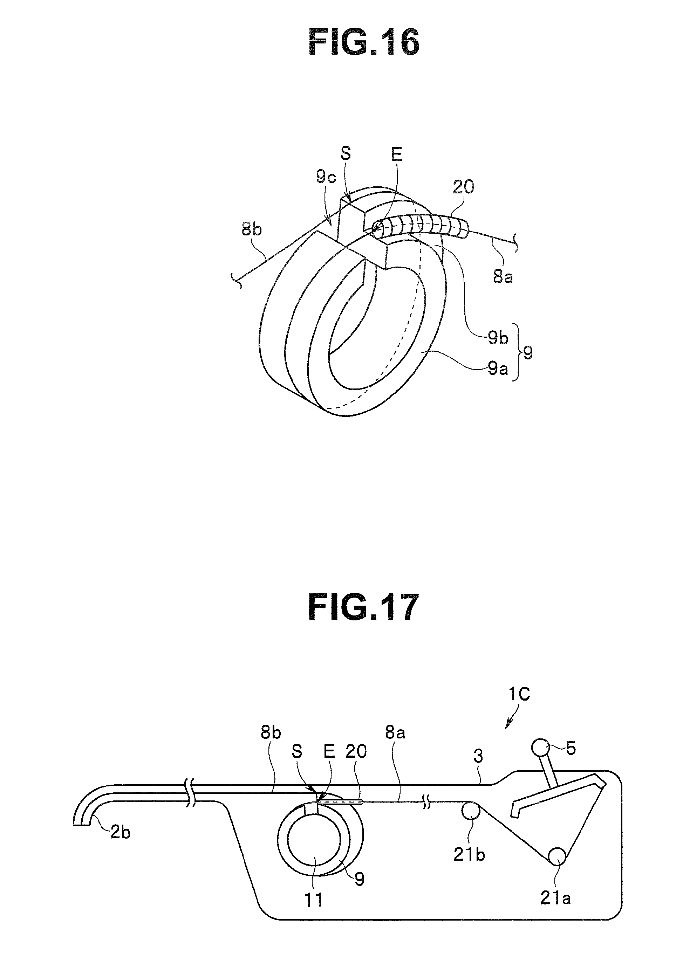

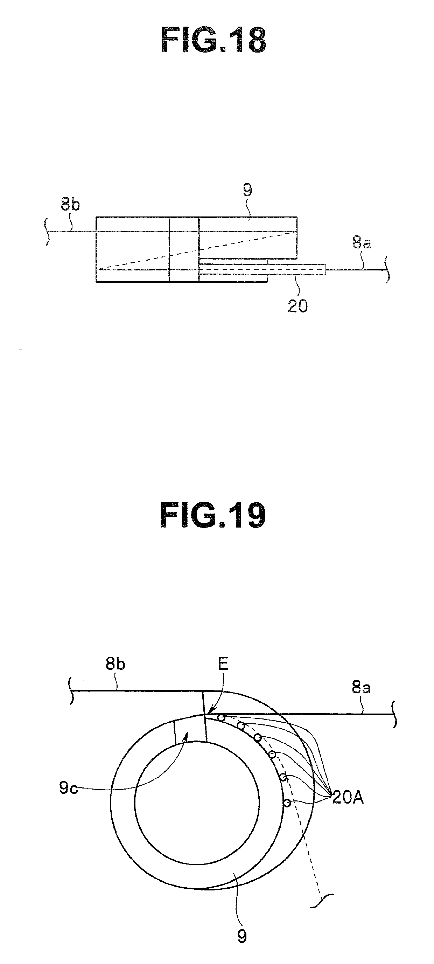

Note that the coil pipe member 20 is disposed so as not to wrap around the C ring-shaped member 9 across the notch portion 9c within a range between when an operation unit 5 is in the neutral state shown in Fig. 15 and when a bending portion 2b is in the maximum bent state shown in Figs. 17 and 18. Other components and actions are substantially same as in the above-described first embodiment.

As has been described above, according to the above-described fourth embodiment, since the operation input-side wire 8a of the pulling wire 8 is inhibited from coming into direct contact with the outer circumferential face of the C ring-shaped member 9 in the neighborhood of the extension position for the wire 8a, contact resistance between the operation input-side wire 8a and the outer circumferential face can be lowered. Thus, the amount of operation force can be made smaller.

Note that although the coil pipe member 20 is disposed to inhibit direct contact of the operation input-side wire 8a with the C ring-shaped member 9 in the present embodiment, the present invention is not limited to the form. For example, the coil pipe member 20 may be provided to inhibit direct contact of the bending portion-side wire 8b with the C ring-shaped member 9.

### (First Modification of Fourth Embodiment)

Fig. 19 is a view showing a first modification of the above-described fourth embodiment.

In the first modification, roll members 20A are disposed on an outer circumferential face of the C ring-shaped member 9 instead of the above-described coil pipe member 20, as shown in Fig. 19.

A plurality of roll members 20A are disposed on the outer circumferential face of the C ring-shaped member 9 and at a part where the coil pipe member 20 is to be disposed in the fourth embodiment e.g., a part where the operation input-side wire 8a wraps from a neighborhood of the notch portion 9c.

The above-described configuration can also inhibit direct contact of the bending portion-side wire 8b with the C ring-shaped member 9, and a smaller amount of operation force can be achieved.

### (Second Modification of Fourth Embodiment)

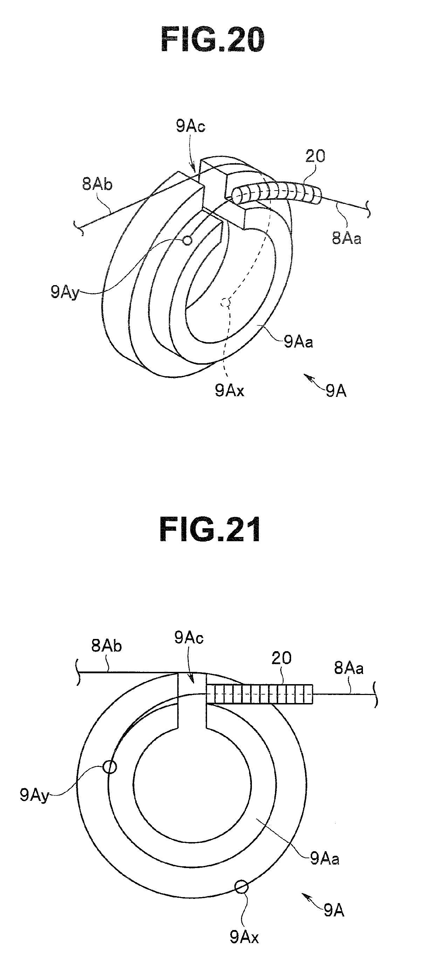

Figs. 20 and 21 are views showing a second modification of the above-described fourth embodiment.

The second modification is an illustration in which the coil pipe member 20 according to the fourth embodiment is applied to the endoscope according to the above-described second embodiment.

That is, the coil pipe member 20 is disposed in a neighborhood of a position, where an operation input-side wire 8Aa extends, on an operation wire extension portion 9Aa of a C ring-shaped member 9A. The coil pipe member 20 is disposed so as not to wrap around the C ring-shaped member 9A across a notch portion 9Ac within a range between when the operation unit 5 is in a neutral state and when the bending portion 2b is in a maximum bent state. Other components and actions are substantially same as in the above-described second embodiment. With the configuration, same effects as those of the above-described fourth embodiment can be obtained.

### (Third Modification of Fourth Embodiment)

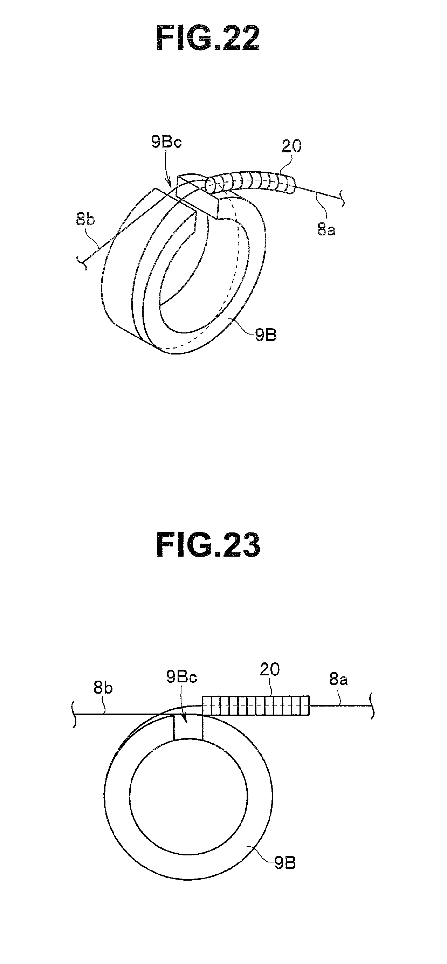

Figs. 22 and 23 are views showing a third modification of the above-described fourth embodiment.

The third modification is an illustration in which the coil pipe member 20 according to the fourth embodiment is applied to the endoscope according to the above-described third embodiment.

That is, the coil pipe member 20 is disposed in a neighborhood of a position, where the operation input-side wire 8a extends, on a C ring-shaped member 9B. The coil pipe member 20 is disposed so as not to wrap around the C ring-shaped member 9B across a notch portion 9Bc within a range between when the operation unit 5 is in a neutral state and when the bending portion 2b is in a maximum bent state. Other components and actions are substantially same as in the above-described third embodiment. With the configuration, same effects as those of the above-described fourth embodiment can be obtained.

### [Fifth Embodiment]

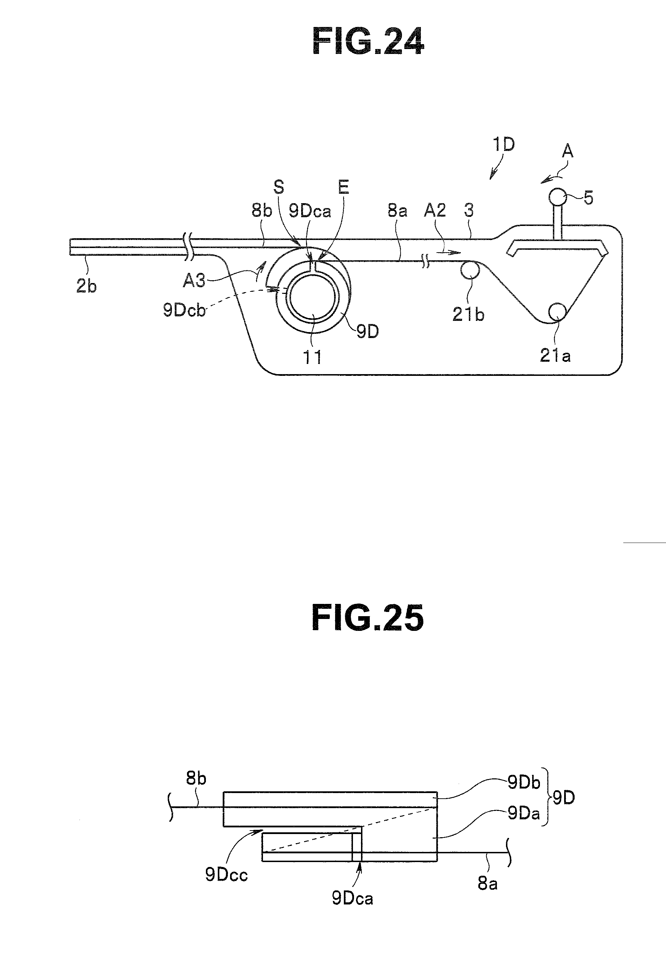

Figs. 24 to 28 are views showing a fifth embodiment of the present invention.

The present embodiment has a substantially same basic configuration as the above-described first embodiment and is different from the first embodiment only in a form of a notch portion of a C ring-shaped member in a pulling member operation apparatus. Thus, in the description below, same components as those of the first embodiment are denoted by equal reference numerals, a description of the components will be omitted, and only different components will be described in detail.

As shown in Figs. 24 to 28, a C ring-shaped member 9D in a pulling operation apparatus of an endoscope 1D according to the present embodiment has a substantially same configuration as that of the first embodiment.

That is, the C ring-shaped member 9D that is disposed on an outer circumferential face of a pulley 11 so as to be frictionally engageable with the outer circumferential face is formed of two C ring-shaped members which are elastically deformable, have notch portions (9Dca, 9Dcb, and 9Dcc) at portions, and are different in outer diameter. The two C ring-shaped members here are an operation wire extension portion 9Da which is made up of a smaller-diameter C ring-shaped member and on an outer circumferential face of which an operation input-side wire 8a extends and a bending wire extension portion 9Db which is a larger-diameter ring-shaped member and at which a bending portion-side wire 8b extends.

The bending wire extension portion 9Db is formed such that an outer diameter increases gradually from a predetermined part on the outer circumferential face of the operation wire extension portion 9Da and is formed to have a part protruding in an outer diameter direction. With the configuration, the outer circumferential face of the smaller-diameter operation wire extension portion 9Da and an outer circumferential face of the larger-diameter bending wire extension portion 9Db are formed of a continuous outer circumferential face, and a level difference is made between the outer circumferential face of the operation wire extension portion 9Da and the outer circumferential face of the bending wire extension portion 9Db. In regard to the point, the present embodiment is substantially same as in the above-described first embodiment.

The notch portion 9Dca serving as a first notch portion is formed in the operation wire extension portion 9Da serving as a first C ring portion, and the notch portion 9Dcb serving as a second notch portion is formed in the bending wire extension portion 9Db serving as a second C ring portion. The notch portions 9Dca and 9Dcb are formed so as to be located at positions which are shifted and separated by a predetermined angle in a circular circumferential direction of a C ring-shaped member 9D. In the case, the notch portion 9Dcb is formed in a neighborhood of a level difference portion of the bending wire extension portion 9Db. In contrast, the notch portion 9Dca is formed at a position shifted and separated by the predetermined angle from a position where the notch portion 9Dcb is disposed in the circular circumferential direction and a rotation direction (a clockwise direction in Fig. 24; a direction denoted by reference character A3 in Fig. 24) of the C ring-shaped member 9D.

The notch portion 9Dca and the notch portion 9Dcb are provided to be linked by the notch portion 9Dcc that is formed to extend in the circumferential direction between the operation wire extension portion 9Da and the bending wire extension portion 9Db. With the configuration, the C ring-shaped member 9D is formed so as to be reduced in diameter by a pulling wire 8 which is wound around an outer circumferential face.

As described above, the notch portion 9Dca and the notch portion 9Dcb are formed at positions shifted in the circular circumferential direction of the C ring-shaped member 9D. The notch portion 9Dca is formed at the position shifted and separated from the notch portion 9Dcb by the predetermined angle in the rotation direction (reference character A3 in Fig. 24) when a bending operation is performed, and the C ring-shaped member 9D pivots and moves.

That is, in the state shown in Fig. 24 (when an operation unit 5 is at a neutral position), the notch portion 9Dca of the operation wire extension portion 9Da of the C ring-shaped member 9D is placed in a neighborhood of a position (reference character E in Fig. 24) where the operation input-side wire 8a extends toward the operation unit 5. In the state, the operation input-side wire 8a wraps on a side across the notch portion 9Dca opposite from a side toward a pulling direction (i.e., toward the operation unit 5).

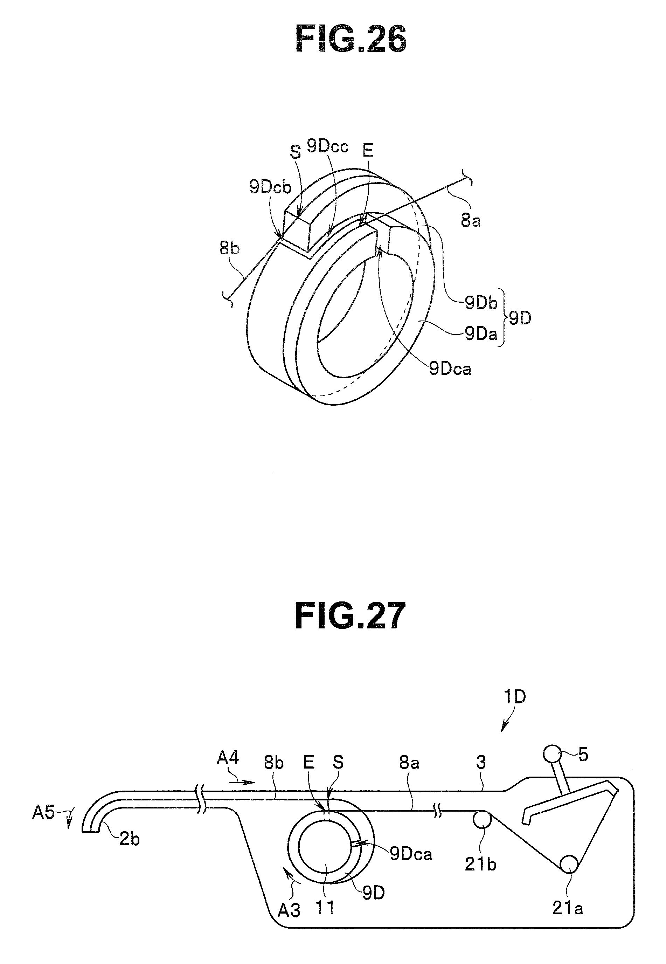

Upon transition to the state shown in Fig. 27 (at the time of maximum bending), the notch portion 9Dcb of the bending wire extension portion 9Db of the C ring-shaped member 9D is placed in a neighborhood of a position (reference character S in Fig. 27) where the bending portion-side wire 8b extends toward a bending portion 2b, i.e., in the neighborhood of the level difference portion of the C ring-shaped member 9D. At the time, the bending portion-side wire 8b does not wrap around the C ring-shaped member 9D across the notch portion 9Dcb due to presence of the level difference portion. Other components are substantially same as in the above-described first embodiment.

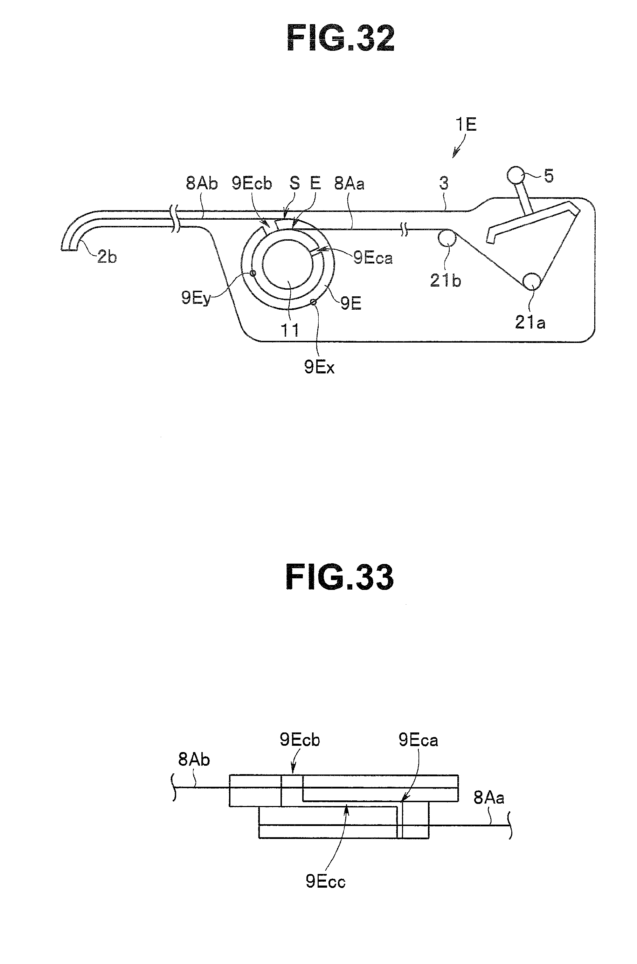

When the operation unit 5 is tilted and operated in a direction of an arrow A in Fig. 24 by an operator while the endoscope 1D according to the present embodiment with the above-described configuration is in the state in Fig. 24, i.e., the operation unit 5 is at the neutral position, the operation input-side wire 8a is pulled in a direction of an arrow A2 in Fig. 24. The C ring-shaped member 9D is narrowed at the notch portion 9Dca against elastic force of the C ring-shaped member 9D and is reduced in diameter. The reduction in diameter brings the C ring-shaped member 9D and the pulley 11 into close contact. The close contact generates frictional resistance between the C ring-shaped member 9D and the pulley 11, which causes the C ring-shaped member 9D to rotate in a direction (the direction of an arrow A3 in Figs. 24 and 27) equal to a direction of the pulley 11 while gliding with respect to the pulley 11. The bending portion-side wire 8b is pulled and moved in a direction of an arrow A4 in Fig. 27, and the bending portion 2b bends in a direction of an arrow A5 in Fig. 32.

If the tilt operation of the operation unit 5 is continued, the bending portion 2b enters the maximum bent state shown in Fig. 27. Upon transition to the state, the notch portion 9Dcb is placed in a neighborhood of a part denoted by reference character S in Fig. 27. Thus, the bending portion-side wire 8b is in a state not wrapped around the C ring-shaped member 9D across the notch portion 9Dcb due to presence of the level difference portion. That is, a bending operation is reliably performed so as not to block reduction in the diameter of the C ring-shaped member 9D by the bending portion-side wire 8b.

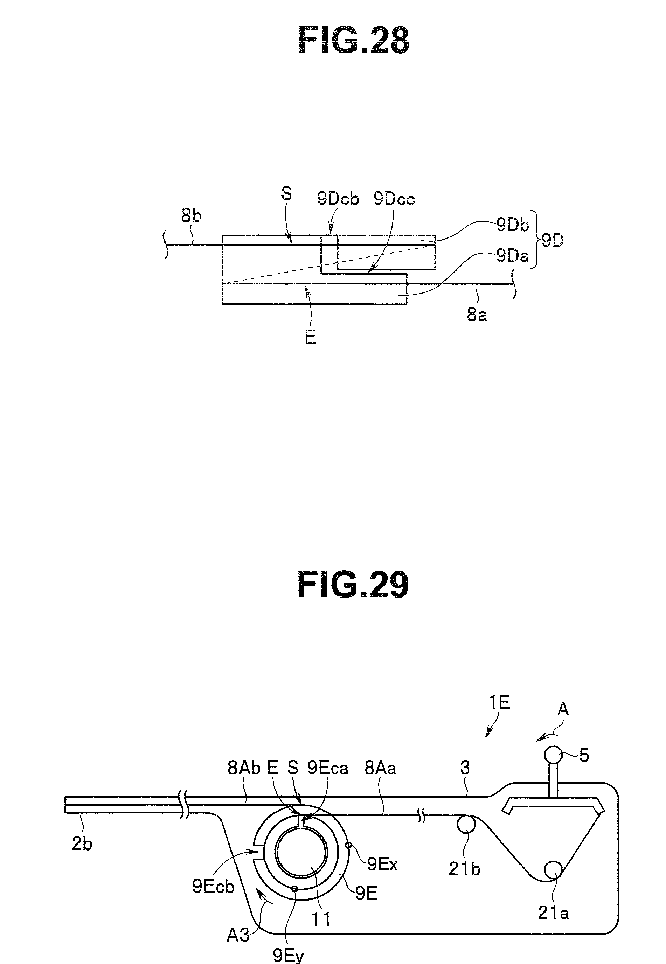

The operation input-side wire 8a is in a state wrapped around the operation wire extension portion 9Da, as shown in Fig. 28. At the time, the operation input-side wire 8a is not in a state wrapped across the notch portion 9Dca and is in a state wrapped around the bending wire extension portion 9Db at a part corresponding to the notch portion 9Dca. However, the notch portion 9Dcb is placed in a neighborhood of a part (reference character E) where the operation input-side wire 8a extends toward the operation unit 5, and pulling force of the operation input-side wire 8a acts to narrow the notch portion 9Dcb and continues to contribute to the diameter reduction action of the C ring-shaped member 9D. Thus, the bending portion 2b continues in the bent state without being released from the bent state.

As has been described above, according to the above-described fifth embodiment, substantially same effects as those of the above-described first embodiment can be obtained. Additionally, in the present embodiment, as for notch portions in the C ring-shaped member 9D, the notch portion 9Dca of the operation wire extension portion 9Da and the notch portion 9Dcb of the bending wire extension portion 9Db are formed at positions shifted and separated in the circular circumferential direction, and the notch portion 9Dca and the notch portion 9Dcb are formed so as to be provided to be linked by the notch portion 9Dcc.

With the above-described configuration, the pulling wire 8 (the operation input-side wire 8a and the bending portion-side wire 8b) can be configured so as not to come into contact with sides, across the notch portions 9Dca and 9Dcb, opposite from sides toward pulling directions, of the C ring-shaped member 9D. Thus, pulling force of the pulling wire 8 does not block the diameter reduction action or a diameter reduction release action of the C ring-shaped member 9D and can be caused to constantly contribute effectively to a bending action of the bending portion 2b.

### [Sixth Embodiment]

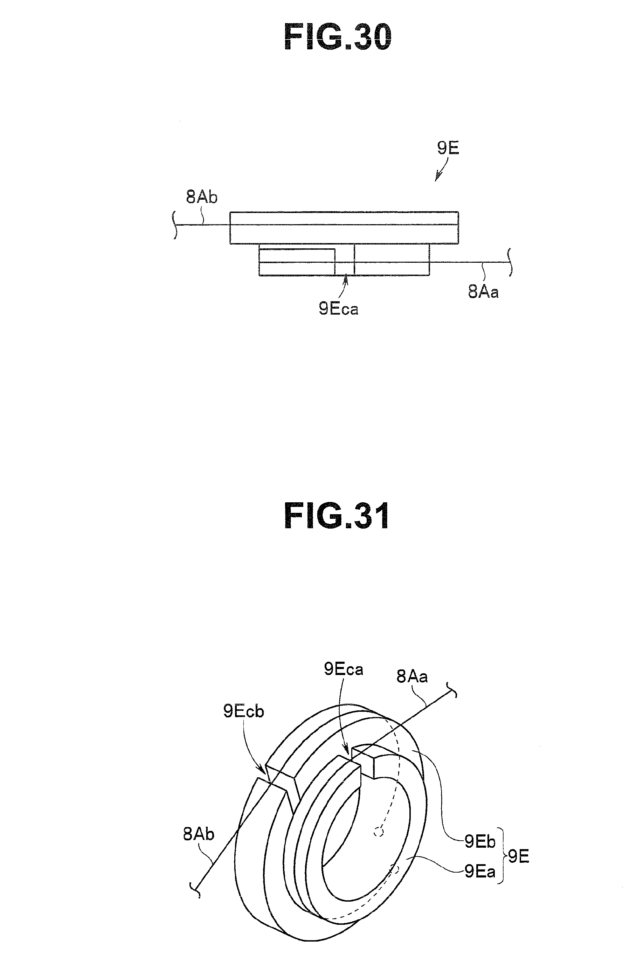

Figs. 29 to 33 are views showing a sixth embodiment of the present invention.

The present embodiment has a substantially same basic configuration as the above-described second embodiment and is different from the second embodiment only in a form of a notch portion of a C ring-shaped member in a pulling member operation apparatus. Thus, in the description below, same components as those of the above-described first and second embodiments are denoted by equal reference numerals, a description of the components will be omitted, and only different components will be described in detail.

As shown in Figs. 29 to 33, a C ring-shaped member 9E in a pulling member operation apparatus of an endoscope 1E according to the present embodiment has a substantially same configuration as that of the second embodiment.

As shown in Figs. 29 to 33, the C ring-shaped member 9E in the pulling member operation apparatus of the endoscope 1E according to the present embodiment is formed such that two C ring-shaped members which have different outer diameters and have notch portions (9Eca, 9Ecb, and 9Ecc) at portions, i.e., an operation wire extension portion 9Ea and a bending wire extension portion 9Eb are coaxially stacked on top of another. Of the operation wire extension portion 9Ea and the bending wire extension portion 9Eb, the operation wire extension portion 9Ea is formed to have a smaller diameter, and the bending wire extension portion 9Eb is formed to have a larger diameter. Thus, the C ring-shaped member 9E is formed to have a level difference corresponding to a difference in outer diameter between an outer circumferential face of the operation wire extension portion 9Ea and an outer circumferential face of the bending wire extension portion 9Eb over a whole circumference. In regard to the point, the present embodiment is substantially same as the above-described second embodiment.

The notch portion 9Eca is formed in the operation wire extension portion 9Ea, and the notch portion 9Ecb is formed in the bending wire extension portion 9Eb. The notch portions 9Eca and 9Ecb are formed so as to be located at positions which are shifted and separated by a predetermined angle in a circular circumferential direction of the C ring-shaped member 9E. Note that a positional relationship between the notch portions 9Eca and 9Ecb is substantially same as in the above-described fifth embodiment.

The notch portion 9Eca and the notch portion 9Ecb are provided to be linked by the notch portion 9Ecc that is formed to extend in the circumferential direction between the operation wire extension portion 9Ea and the bending wire extension portion 9Eb. With the configuration, the C ring-shaped member 9E is formed so as to be reduced in diameter by a pulling wire 8Ab which is wound around an outer circumferential face. The configuration is substantially same as in the fifth embodiment.

Note that a pulling member according to the present embodiment is composed of two wire members, an operation input-side wire 8Aa serving as an operation input-side pulling member and the bending portion-side wire 8Ab serving as a bending portion-side pulling member, as in the above-described second embodiment.

One end of the operation input-side wire 8Aa is coupled to (a pulling wire attaching portion of a suspension frame of) an operation unit 5, and the other end is fixed and placed at a predetermined part (reference character 9Ey) on the outer circumferential face of the operation wire extension portion 9Ea of the C ring-shaped member 9E. The part (reference character 9Ey) where the other end of the operation input-side wire 8Aa is fixed and placed is a part on the outer circumferential face of the operation wire extension portion 9Ea which is across the notch portion 9Eca from a part (reference character E) where the operation input-side wire 8Aa extends toward an operation input side, as shown in Figs. 29 to 33. In the present embodiment, the part (reference character 9Ay) where the other end of the operation input-side wire 8Aa is fixed and placed is set to a part to which the operation input-side wire 8Aa is wrapped in the circular circumferential direction across the notch portion 9Eca, a neighborhood of a part which is at an angle of substantially less than 180° in the circular circumferential direction with respect to the notch portion 9Eca and substantially faces the notch portion 9Eca. That is, in the present embodiment, a wrap distance, over which the operation input-side wire 8Aa wraps around the C ring-shaped member 9E across the notch portion 9Eca, is set to be longer than in the case of the above-described second embodiment.

One end of the bending portion-side wire 8Ab is coupled to a bending portion 2b, and the other end is fixed and placed at a predetermined part (reference character 9Ex) on the outer circumferential face of the bending wire extension portion 9Eb of the C ring-shaped member 9E. As shown in Figs. 29 to 33, the part (reference character 9Ex) where the other end of the bending portion-side wire 8Ab is fixed and placed is set to a neighborhood of a part which is at an angle of substantially less than 180° in the circular circumferential direction with respect to the notch portion 9Ecb and substantially faces the notch portion 9Ecb. In the case, the bending portion-side wire 8Ab is configured so as not to be placed at a position which is across the notch portion 9Ecb.

Thus, in the state shown in Fig. 29 (when an operation unit 5 is at a neutral position), the notch portion 9Eca of the operation wire extension portion 9Ea of the C ring-shaped member 9E is placed in a neighborhood of a position (reference character E in Fig. 29) where the operation input-side wire 8Aa extends toward the operation unit 5. In the case, the operation input-side wire 8Aa wraps on a side across the notch portion 9Eca opposite from a side toward a pulling direction (i.e., toward the operation unit 5).

Upon transition to the state shown in Fig. 32 (at the time of maximum bending), the notch portion 9Ecb of the bending wire extension portion 9Eb of the C ring-shaped member 9E is placed in a neighborhood of a position (reference character S in Fig. 32) where the bending portion-side wire 8Ab extends toward the bending portion 2b. At the time, the bending portion-side wire 8Ab does not wrap around the C ring-shaped member 9E across the notch portion 9Ecb. Other components are substantially same as in the above-described second embodiment.

When the operation unit 5 is tilted and operated in a direction of an arrow A in Fig. 29 by an operator while the endoscope 1E according to the present embodiment with the above-described configuration is in the state in Fig. 29 (a state in which the operation unit 5 is at the neutral position), the operation input-side wire 8Aa is pulled. The C ring-shaped member 9E is narrowed at the notch portion 9Eca against elastic force of the C ring-shaped member 9E and is reduced in diameter. The reduction in diameter generates frictional resistance between the C ring-shaped member 9E and a pulley 11, which causes the C ring-shaped member 9E to rotate in a direction (a direction of an arrow A3 in Fig. 29) equal to a direction of the pulley 11 while gliding with respect to the pulley 11. The bending portion-side wire 8Ab is pulled and moved, and the bending portion 2b bends in a predetermined direction.

If the tilt operation of the operation unit 5 is continued, the bending portion 2b enters the maximum bent state shown in Fig. 32. Upon transition to the state, the notch portion 9Ecb is placed in a neighborhood of a part denoted by reference character S in Fig. 32. At the time, the bending portion-side wire 8Ab is in a state not wrapped around the C ring-shaped member 9E across the notch portion 9Ecb. With the configuration, a bending operation is reliably performed so as not to block reduction in the diameter of the C ring-shaped member 9E by the bending portion-side wire 8Ab.

The operation input-side wire 8Aa is in a state wrapped around the operation wire extension portion 9Ea of the C ring-shaped member 9E at a position space apart from the notch portion 9Eca. Thus, pulling force of the operation input-side wire 8Aa acts poorly on the notch portion 9Eca. However, since the notch portion 9Ecb is placed in a neighborhood of the part (reference character E) where the operation input-side wire 8Aa extends toward the operation unit 5, as shown in Fig. 32, the pulling force of the operation input-side wire 8Aa acts to narrow the notch portion 9Ecb and continues to contribute to the diameter reduction action of the C ring-shaped member 9E. Thus, the bending portion 2b continues in the bent state without being released from the bent state.

As has been described above, according to the above-described sixth embodiment, substantially same effects as those of the above-described second embodiment can be obtained. In addition, since the notch portions (9Eca, 9Ecb, and 9Ecc) substantially same as in the above-described fifth embodiment are formed, substantially same effects as those of the fifth embodiment can be obtained in the present embodiment as well.

### [Seventh Embodiment]

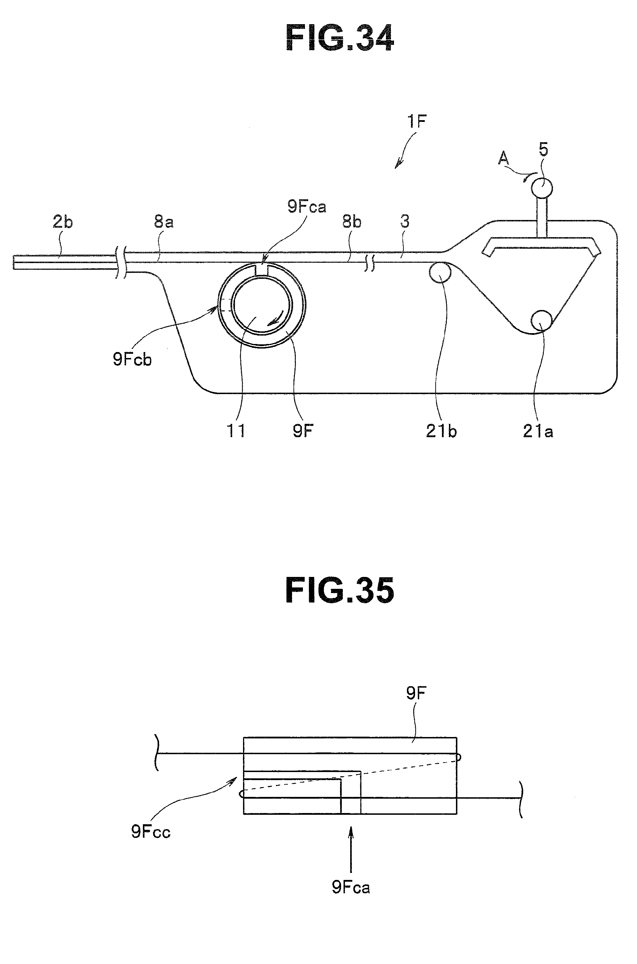

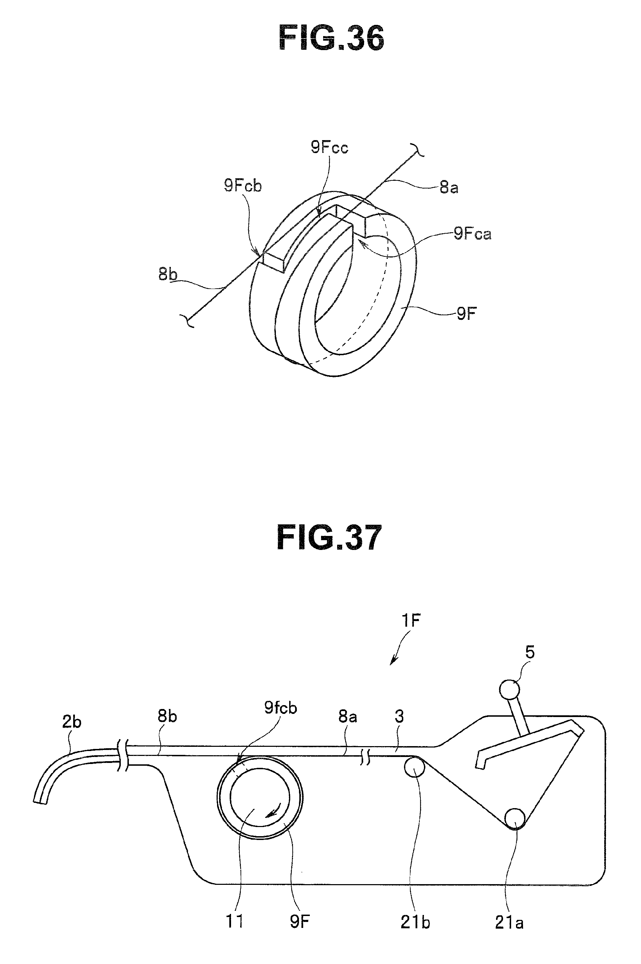

Figs. 34 to 38 are views showing a seventh embodiment of the present invention.

The present embodiment has a substantially same basic configuration as the above-described third embodiment and is different from the third embodiment only in a form of a notch portion of a C ring-shaped member in a pulling member operation apparatus. Thus, in the description below, same components as those of the above-described first and third embodiments are denoted by equal reference numerals, a description of the components will be omitted, and only different components will be described in detail.

As shown in Figs. 34 to 38, a C ring-shaped member 9F in a pulling member operation apparatus of an endoscope 1F according to the present embodiment has a substantially same configuration as that of the third embodiment but is different in forms of notch portions (9Fca, 9Fcb, and 9Fcc).

In the C ring-shaped member 9F according to the present embodiment, the two notch portions 9Fca and 9Fcb that are formed at positions shifted and separated by a predetermined angle in a circular circumferential direction and are made in a width direction and the notch portion 9Fcc that links the two notch portions in the circumferential direction are formed. Note that a positional relationship between the notch portions 9Fca and 9Fcb is substantially same as in the above-described fifth embodiment. In a pulling member according to the present embodiment, an operation input-side wire 8a serving as an operation input-side pulling member and a bending portion-side wire 8b serving as a bending portion-side pulling member are composed of one continuous wire member, as in the above-described first and third embodiments.

In the endoscope 1F according to the present embodiment with the above-described configuration as well, when an operation unit 5 in the neutral state in Fig. 35 is tilted, for example, in a direction of an arrow A in Fig. 35, a bending portion 2b bends in a predetermined direction, substantially as in the above-described first and third embodiments.

As has been described above, according to the above-described seventh embodiment, substantially same effects as those of the first and third embodiments can be obtained, and substantially same effects as those of the above-described fifth and sixth embodiments can be obtained.

### [Eighth Embodiment]

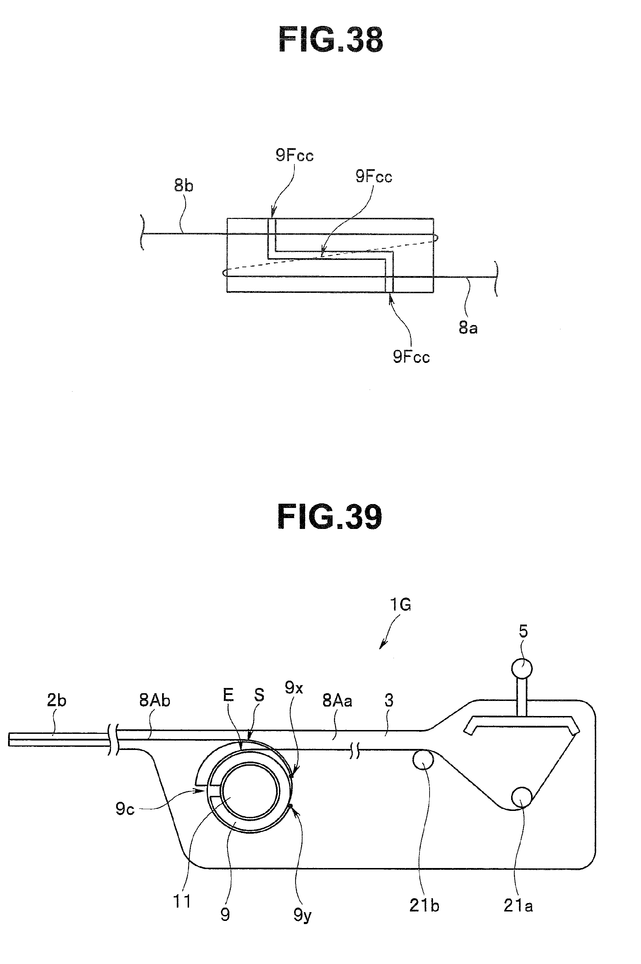

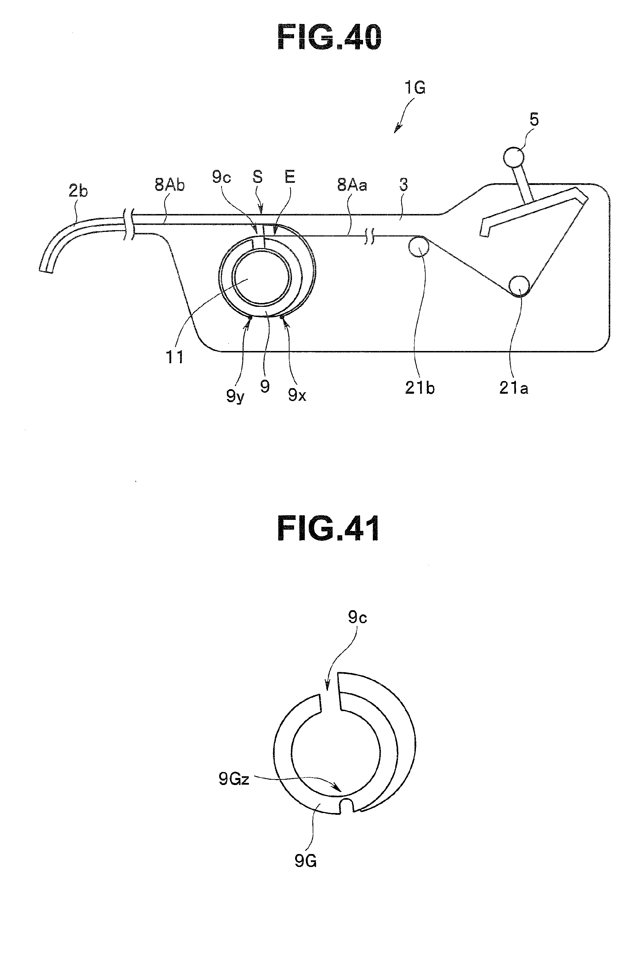

Figs. 39 and 40 are views showing an eighth embodiment of the present invention.

The present embodiment has a substantially same basic configuration as the above-described first embodiment and is different from the first embodiment only in a form of a pulling member (a pulling wire 8) in a pulling member operation apparatus. Thus, in the description below, same components as those of the first embodiment are denoted by equal reference numerals, a description of the components will be omitted, and only different components will be described in detail.

In contrast to the configuration of the first embodiment, in an endoscope 1G according to the present embodiment, the pulling wire 8 that is wound around a C ring-shaped member 9 is composed of two pulling wires, as in the above-described second embodiment. The two pulling wires here are an operation input-side wire 8Aa serving as a first pulling member and an operation input-side pulling member and a bending portion-side wire 8Ab serving as a second pulling member and a bending portion-side pulling member.

One end of the operation input-side wire 8Aa is coupled to a pulling wire attaching portion of a suspension frame of an operation unit 5, and the other end is fixed and placed at a predetermined part (9y) on an outer circumferential face of the C ring-shaped member 9. The part (9y) where the other end of the operation input-side wire 8Aa is fixed is set to a neighborhood of a part which faces a notch portion 9c on the outer circumferential face of the C ring-shaped member 9 and is a part which is at an angle of substantially less than 180° in a circular circumferential direction with respect to the notch portion 9c and substantially faces the notch portion 9c. With the configuration, the operation input-side wire 8Aa is placed to be wrapped around the outer circumferential face of the C ring-shaped member 9 across the notch portion 9c from an extension position E toward an operation input member of the C ring-shaped member 9 to the fixed part 9y.

One end of the bending portion-side wire 8Ab is coupled to a bending portion 2b, and the other end is fixed and placed at a predetermined part (9x) on the outer circumferential face of the C ring-shaped member 9. The part (9x) where the other end of the bending portion-side wire 8Ab is fixed is set to a neighborhood of a part which faces the notch portion 9c on the outer circumferential face of the C ring-shaped member 9 and a part which is at an angle of substantially less than 180° in the circular circumferential direction with respect to the notch portion 9c and substantially faces the notch portion 9c, like the other end of the operation input-side wire 8Aa. With the configuration, the bending portion-side wire 8Ab is placed to be wrapped around the outer circumferential face of the C ring-shaped member 9 from an extension position S toward the bending portion of the C ring-shaped member 9 to the fixed part 9x so as not to cross the notch portion 9c.

With the above-described configuration, when the operation unit 5 in the state in Fig. 39 (the operation unit 5 is in a neutral state) is tilted and operated, and the operation input-side wire 8Aa is pulled, the C ring-shaped member 9 is reduced in diameter. Since the configuration is such that the other end of the operation input-side wire 8Aa is fixed at the part (9y) that is at the angle of substantially less than 180° in the circular circumferential direction with respect to the notch portion 9c and substantially faces the notch portion 9c at the time, pulling force of the operation input-side wire 8Aa does not block the diameter reduction action or a diameter reduction release action of the C ring-shaped member 9.

For example, if the part 9y where the other end of the operation input-side wire 8Aa is fixed is at an angle of substantially not less than 180° in the circular circumferential direction with respect to the notch portion 9c, the pulling force of the operation input-side wire 8Aa acts not in a direction narrowing a gap in the notch portion 9c but in a direction expanding the gap. That is, the pulling force of the operation input-side wire 8Aa may block the diameter reduction action or the diameter reduction release action. The present embodiment, however, solves the problem by configuring the operation input-side wire 8Aa such that the other end is fixed at the part (9y) that is at the angle of substantially less than 180° in the circular circumferential direction with respect to the notch portion 9c and substantially faces the notch portion 9c.

Even in the state in Fig. 40 (a maximum bent state), the pulling force of the operation input-side wire 8Aa works in a direction reducing the C ring-shaped member 9 in diameter. The bending portion-side wire 8Ab is placed so as never to cross the notch portion 9c, due to presence of a level difference portion. Thus, the bending portion-side wire 8Ab does not block the diameter reduction action or the diameter reduction release action of the C ring-shaped member 9. Other components and actions are substantially same as in the above-described first embodiment.

As has been described above, according to the above-described eighth embodiment, same effects as those of the first embodiment can be obtained. Additionally, in the present embodiment, the bending portion-side wire 8Ab can be wrapped around the outer circumferential face of the C ring-shaped member 9 without crossing the notch portion 9c, and a wrap distance, over which the bending portion-side wire 8Ab wraps around the C ring-shaped member 9, can be set to be longer.

### (Modification of Eighth Embodiment)

Fig. 41 is a view showing a modification of the C ring-shaped member according to the above-described eighth embodiment.

In the present modification, a C ring-shaped member 9G is provided with a thin portion 9Gz having a small thickness in a radial direction in a neighborhood of a part which faces the notch portion 9c, i.e., at a part which is at an angle of substantially 180° in a circular circumferential direction with respect to the notch portion 9c and faces the notch portion 9c. With the configuration, the C ring-shaped member 9G is formed to be easily elastically deformable at the thin portion 9Gz. Other components and actions are substantially same as in the above-described eighth embodiment. Since the configuration facilitates elastic deformation of the C ring-shaped member 9G, a diameter reduction action of the C ring-shaped member 9G can be more easily performed.

### [Ninth Embodiment]

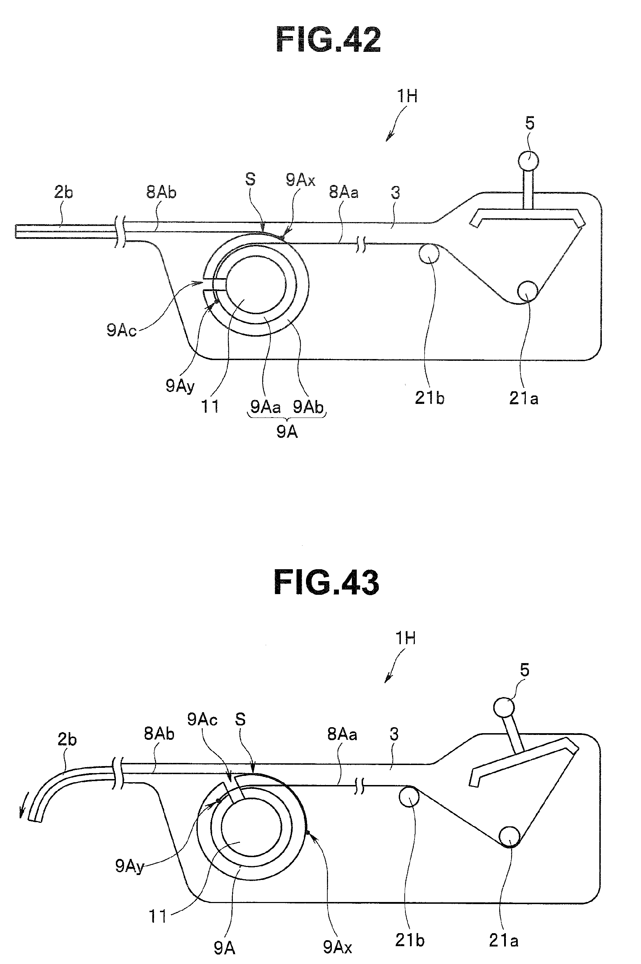

Figs. 42 and 43 are views showing a ninth embodiment of the present invention.

The present embodiment has a substantially same basic configuration as the above-described second embodiment and has been changed from the second embodiment only in a position where a pulling member (a pulling wire 8) in a pulling member operation apparatus is fixed. Thus, in the description below, same components as those of the above-described first and second embodiments are denoted by equal reference numerals, a description of the components will be omitted, and only different components will be described in detail.

In an endoscope 1 H according to the present embodiment, one end of a bending portion-side wire 8Ab is coupled to a bending portion 2b, and the other end is fixed and placed at a predetermined part (reference character 9Ax) on an outer circumferential face of a bending wire extension portion 9Ab of a C ring-shaped member 9A. The part where the other end of the bending portion-side wire 8Ab is fixed is a part on an outer circumferential face of the C ring-shaped member 9A which is separated from a notch portion 9Ac by an angle of substantially less than 180° in a circular circumferential direction. With the configuration, the bending portion-side wire 8Ab is placed to be wrapped around the outer circumferential face of the C ring-shaped member 9A from an extension position S toward the bending portion of the C ring-shaped member 9A to the fixed part 9Ax so as never to cross the notch portion 9Ac, within a movable range from the neutral state in Fig. 42 to the maximum bent state in Fig. 43. Other components and actions are substantially same as in the above-described first embodiment.

As has been described above, according to the above-described ninth embodiment, same effects as those of the above-described second embodiment can be obtained. Additionally, in the present embodiment, the bending portion-side wire 8Ab can be wrapped around the outer circumferential face of the C ring-shaped member 9A so as never to cross the notch portion 9Ac.

### (Modification of Ninth Embodiment)

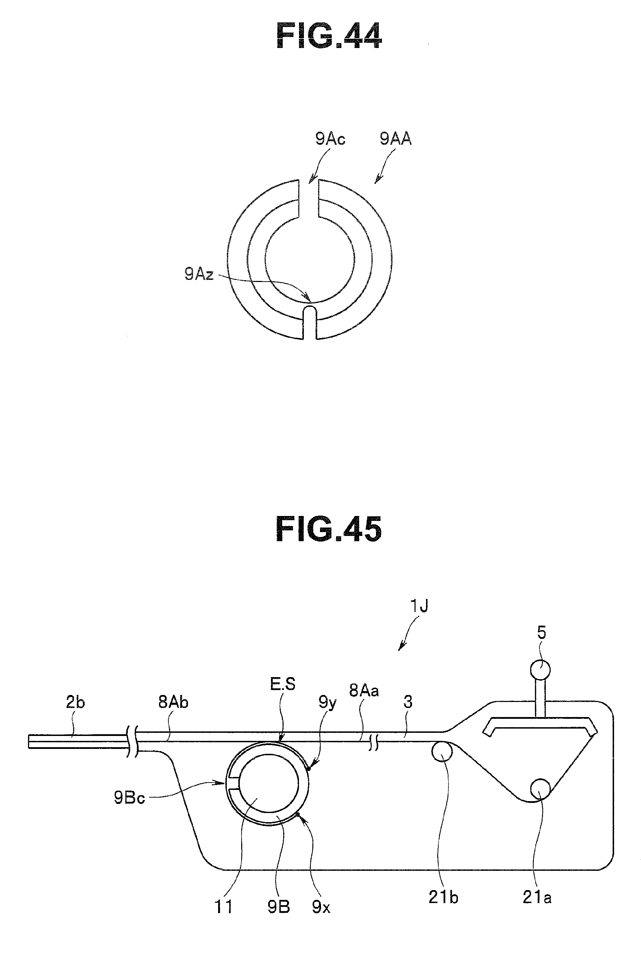

Fig. 44 is a view showing a modification of the C ring-shaped member according to the above-described ninth embodiment.

In the present modification, a C ring-shaped member 9AA is provided with a thin portion 9Az having a small thickness in a radial direction in a neighborhood of a part which faces the notch portion 9Ac, i.e., at a part which is at an angle of substantially 180° in a circular circumferential direction with respect to the notch portion 9Ac and faces the notch portion 9Ac. With the configuration, the C ring-shaped member 9AA is formed to be easily elastically deformable at the thin portion 9Az. Other components and actions are substantially same as in the above-described ninth embodiment. Since the configuration facilitates elastic deformation of the C ring-shaped member 9AA, a diameter reduction action of the C ring-shaped member 9AA can be more easily performed.

### [Tenth Embodiment]

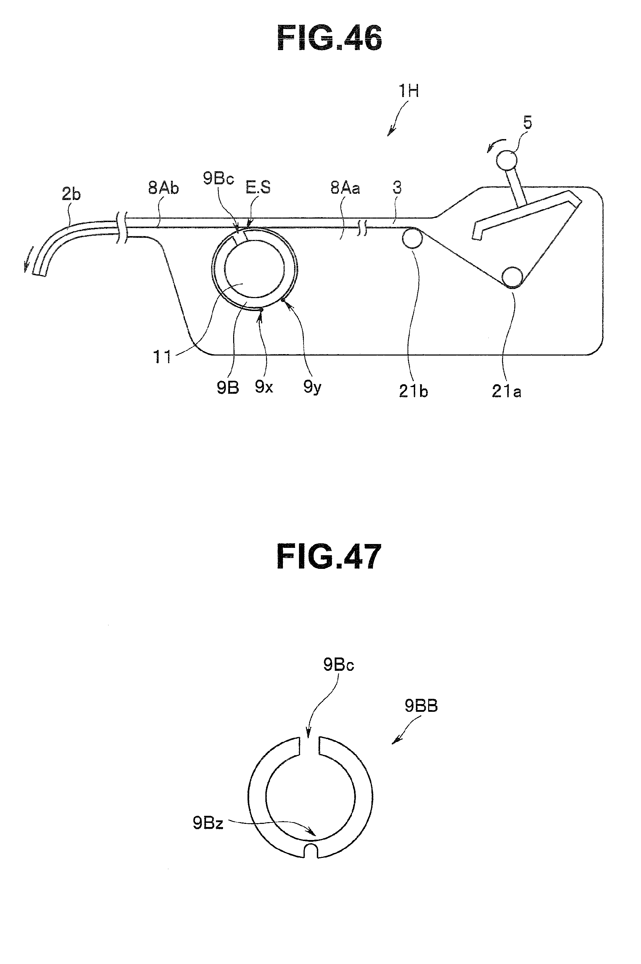

Figs. 45 and 46 are views showing a tenth embodiment of the present invention.

The present embodiment has a substantially same basic configuration as the above-described third embodiment and is different from the third embodiment only in a form of a pulling member (a pulling wire 8) in a pulling member operation apparatus. Thus, in the description below, same components as those of the above-described first and third embodiments are denoted by equal reference numerals, a description of the components will be omitted, and only different components will be described in detail.

The present embodiment is obtained by applying a same configuration as that of the above-described eighth embodiment to the configuration of the above-described third embodiment.

That is, in an endoscope 1J according to the present embodiment, the pulling wire 8 that is wound around a C ring-shaped member 9B is composed of two pulling wires, i.e., an operation input-side wire 8Aa and a bending portion-side wire 8Ab. The operation input-side wire 8Aa and the bending portion-side wire 8Ab are placed in a same manner as in the above-described eighth embodiment.

That is, the other end of the bending portion-side wire 8Ab is fixed and placed at a predetermined part 9x on an outer circumferential face of the C ring-shaped member 9B. The fixed part 9x is in a neighborhood of a part which faces a notch portion 9Bc on the outer circumferential face of the C ring-shaped member 9B and is a part which is at an angle of substantially less than 180° in a circular circumferential direction with respect to the notch portion 9Bc and substantially faces the notch portion 9Bc. With the configuration, the bending portion-side wire 8Ab is placed to be wrapped around the outer circumferential face of the C ring-shaped member 9B from an extension position S toward a bending portion of the C ring-shaped member 9B to the fixed part 9x so as not to cross the notch portion 9Bc. Other components and actions are substantially same as in the above-described third embodiment.

As has been described above, according to the above-described tenth embodiment, same effects as those of the third embodiment can be obtained. Additionally, same effects as those of the above-described eighth embodiment can be obtained in the present embodiment as well.

### (Modification of Tenth Embodiment)

Fig. 47 is a view showing a modification of the C ring-shaped member according to the above-described tenth embodiment.

In the present modification, a C ring-shaped member 9BB is provided with a thin portion 9Bz having a small thickness in a radial direction in a neighborhood of a part which faces the notch portion 9Bc, i.e., at a part which is at an angle of substantially 180° in a circular circumferential direction with respect to the notch portion 9Bc and faces the notch portion 9Bc. With the configuration, the C ring-shaped member 9BB is formed to be easily elastically deformable at the thin portion 9Bz. Other components and actions are substantially same as in the above-described tenth embodiment. Since the configuration facilitates elastic deformation of the C ring-shaped member 9BB, a diameter reduction action of the C ring-shaped member 9BB can be easily performed.

### [11th Embodiment]

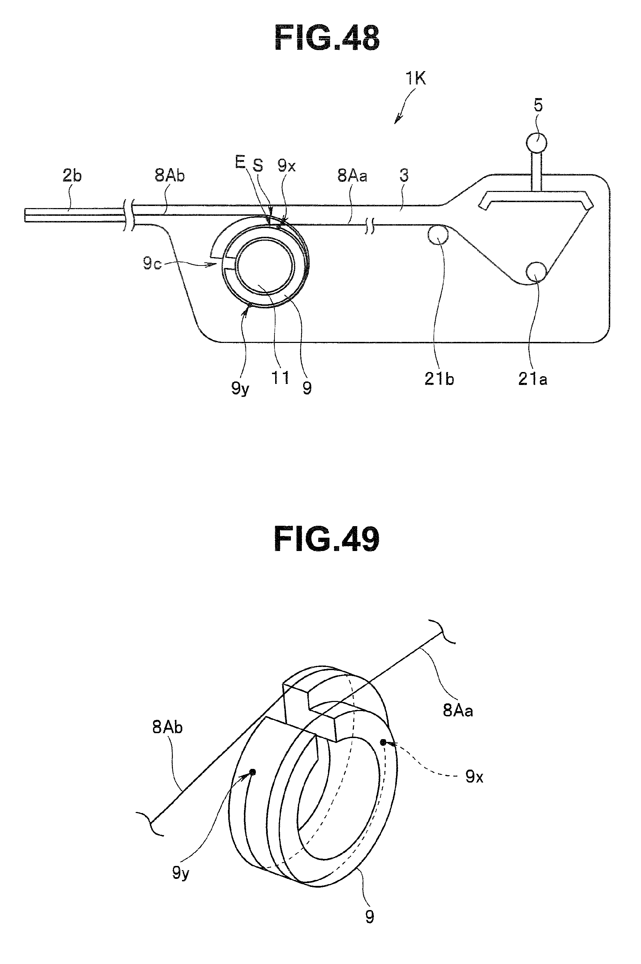

Figs. 48 to 50 are views showing an 11th embodiment of the present invention.

The present embodiment has a substantially same basic configuration as the above-described eighth embodiment and has been changed from the eighth embodiment in a position where a pulling member (a pulling wire 8) in a pulling member operation apparatus is fixed. Thus, in the description below, same components as those of the above-described first and eighth embodiments are denoted by equal reference numerals, a description of the components will be omitted, and only different components will be described in detail.

One end of an operation input-side wire 8Aa in an endoscope 1K according to the present embodiment is coupled to a pulling wire attaching portion of a suspension frame of an operation unit 5, and the other end is fixed and placed at a predetermined part (9y) on an outer circumferential face of a C ring-shaped member 9. The part where the other end of the operation input-side wire 8Aa is fixed is a part to which the operation input-side wire 8Aa is wrapped across a notch portion 9c from an extension part E toward the operation unit 5 by an angle of substantially not less than 360° in a circular circumferential direction, on the outer circumferential face of the C ring-shaped member 9.

One end of the bending portion-side wire 8Ab is coupled to a bending portion 2b, and the other end is fixed and placed at a predetermined part (reference character 9x) on an outer circumferential face of a bending wire extension portion 9b of the C ring-shaped member 9. The part 9x where the other end of the bending portion-side wire 8Ab is fixed is set to a part to which the bending portion-side wire 8Ab is wrapped from an extension part S toward the bending portion 2b by an angle of substantially not less than 180° in the circular circumferential direction, on the outer circumferential face of the C ring-shaped member 9, and is set so as not to cross the notch portion 9c.

As has been described in the above-described eighth embodiment, for example, if the part 9y where the other end of the operation input-side wire 8Aa is fixed is set to a part which is at an angle of substantially not less than 180° in the circular circumferential direction with respect to the notch portion 9c, pulling force of the operation input-side wire 8Aa may block a diameter reduction action or a diameter reduction release action of the C ring-shaped member 9.

In the present embodiment, however, a sufficiently long wrap distance is ensured by setting the part 9y where the other end of the operation input-side wire 8Aa is fixed to a part to which the operation input-side wire 8Aa is wrapped across the notch portion 9c from the extension part E toward the operation unit 5 by an angle of substantially 360° in the circular circumferential direction. With the configuration, a sufficient amount of increase in normal force of the C ring-shaped member 9 against a pulley 11 is obtained.

That is, the above-described configuration does not matter as long as the amount of increase in the normal force of the C ring-shaped member 9 against the pulley 11 caused by increase in the distance, over which the operation input-side wire 8Aa wraps around the C ring-shaped member 9, is larger than the amount of force which blocks the diameter reduction action or the diameter reduction release action of the C ring-shaped member 9 by the pulling force of the operation input-side wire 8Aa. Other components and actions are substantially same as the above-described eighth embodiment.

According to the 11th embodiment described above, same effects as those of the above-described first embodiment can be obtained. Additionally, in the present embodiment, longer wrap distances of the operation input-side wire 8Aa and the bending portion-side wire 8Ab can be obtained, and the pulling force of the operation input-side wire 8Aa does not block the diameter reduction action or the diameter reduction release action of the C ring-shaped member 9.

### [12th Embodiment]

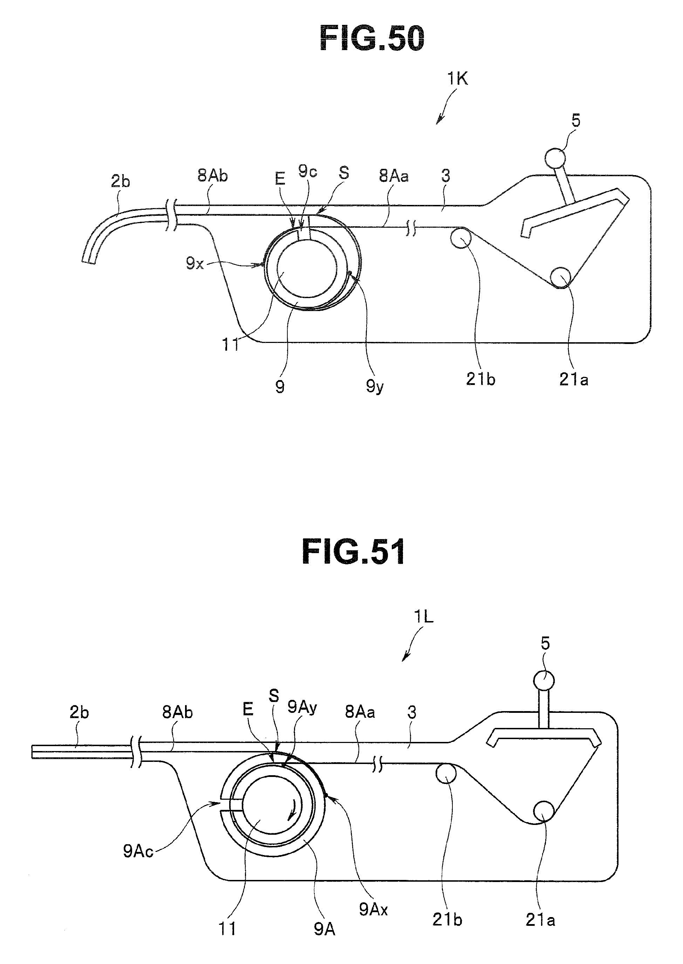

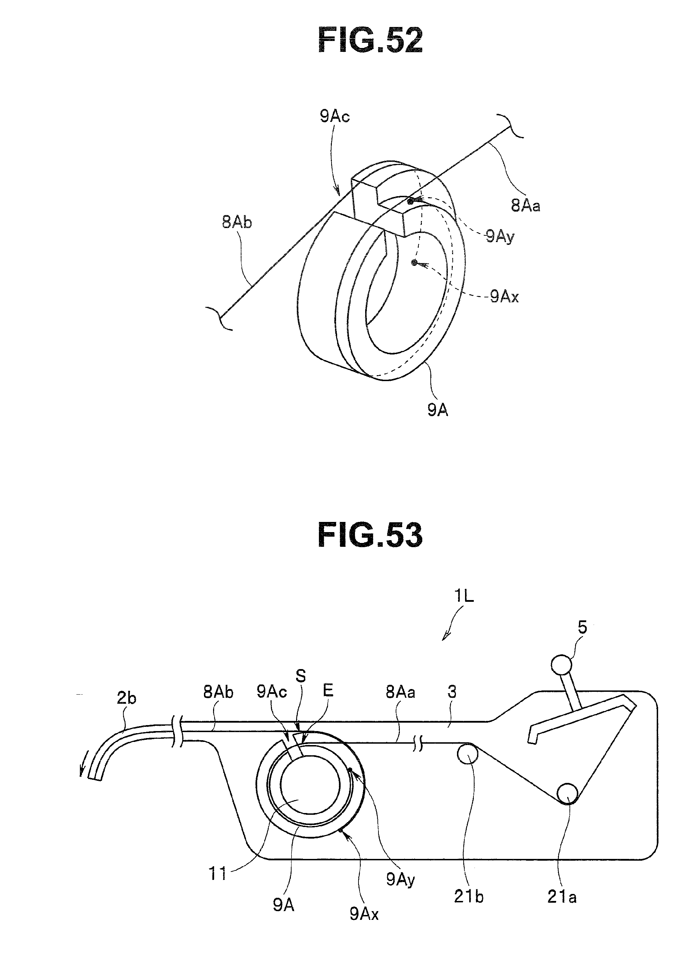

Figs. 51 to 53 are views showing a 12th embodiment of the present invention.

The present embodiment has a substantially same basic configuration as the above-described ninth embodiment and has been changed from the ninth embodiment in a position where a pulling member (a pulling wire 8) in a pulling member operation apparatus is fixed. Thus, in the description below, same components as those of the above-described first and ninth embodiments are denoted by equal reference numerals, a description of the components will be omitted, and only different components will be described in detail.

In an endoscope 1L according to the present embodiment, a part 9Ay where the other end of an operation input-side wire 8Aa is fixed to a C ring-shaped member 9A is set to a part to which the operation input-side wire 8Aa wrapping counterclockwise in Fig. 51 from an extension part E toward an operation unit 5 is wrapped across a notch portion 9Ac by an angle of substantially 360° in a circular circumferential direction, on an outer circumferential face of the C ring-shaped member 9A. A part 9Ax where the other end of a bending portion-side wire 8Ab is fixed to the C ring-shaped member 9A is substantially same as in the above-described ninth embodiment. Other components and actions are substantially same as in the ninth embodiment.

According to the 12th embodiment described above, same effects as those of the above-described first and 11th embodiments can be obtained.

### [13th Embodiment]

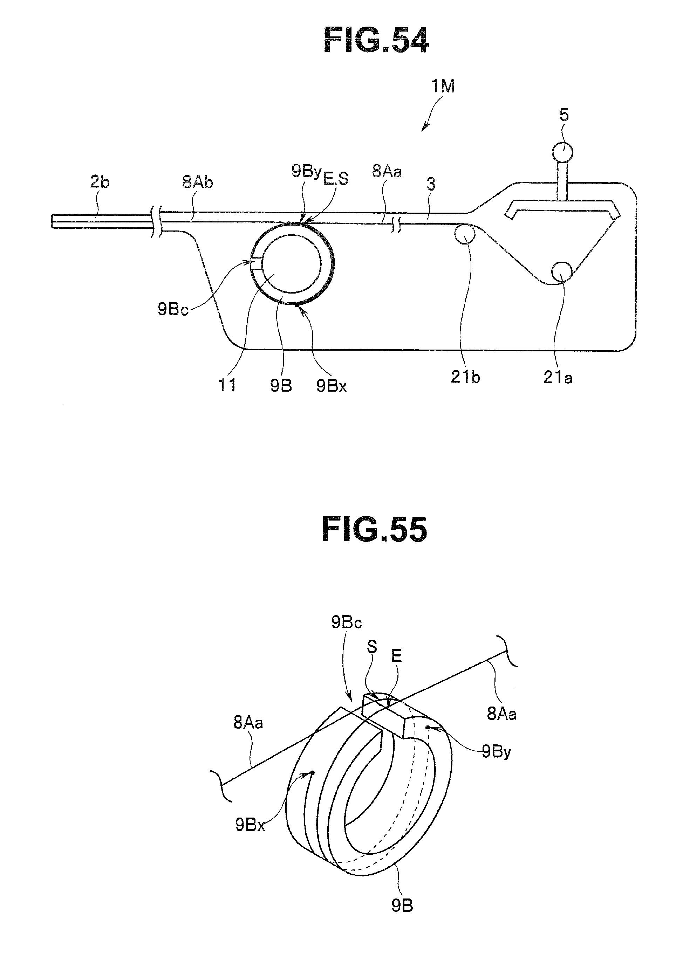

Figs. 54 to 56 are views showing a 13th embodiment of the present invention.

The present embodiment has a substantially same basic configuration as the above-described tenth embodiment and has been changed from the tenth embodiment in a position where a pulling member (a pulling wire 8) in a pulling member operation apparatus is fixed. Thus, in the description below, same components as those of the above-described first and tenth embodiments are denoted by equal reference numerals, a description of the components will be omitted, and only different components will be described in detail.

In an endoscope 1M according to the present embodiment, a part 9By where the other end of an operation input-side wire 8Aa is fixed to a C ring-shaped member 9B is a part to which the operation input-side wire 8Aa wrapping counterclockwise in Fig. 54 from an extension part E toward the operation unit 5 is wrapped across a notch portion 9Bc by an angle of substantially 360° in a circular circumferential direction, on an outer circumferential face of the C ring-shaped member 9B.

A part 9Bx where the other end of a bending portion-side wire 8Ab is fixed to the C ring-shaped member 9B is a part which is at an angle of substantially less than 180° in the circular circumferential direction with respect to an extension part S toward a bending portion 2b when the bending portion-side wire 8Ab wraps clockwise in Fig. 54 from the extension part S and substantially faces the extension part S, on the outer circumferential face of the C ring-shaped member 9B. Other components and actions are substantially same as in the above-described tenth embodiment.

According to the 13th embodiment described above, same effects as those of the above-described first and 11th embodiments can be obtained.

### [14th Embodiment]

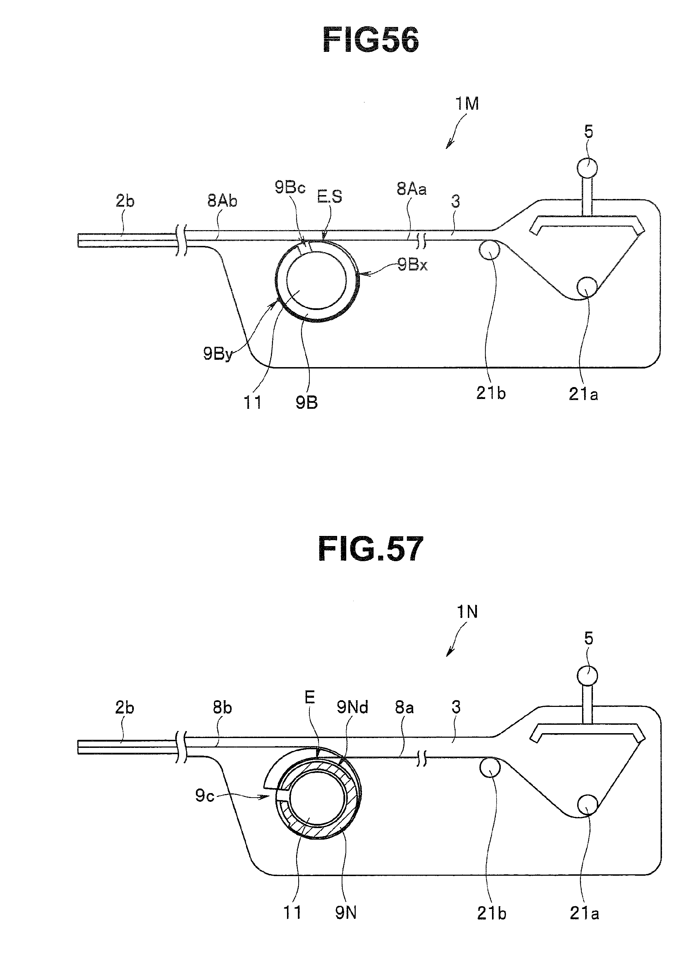

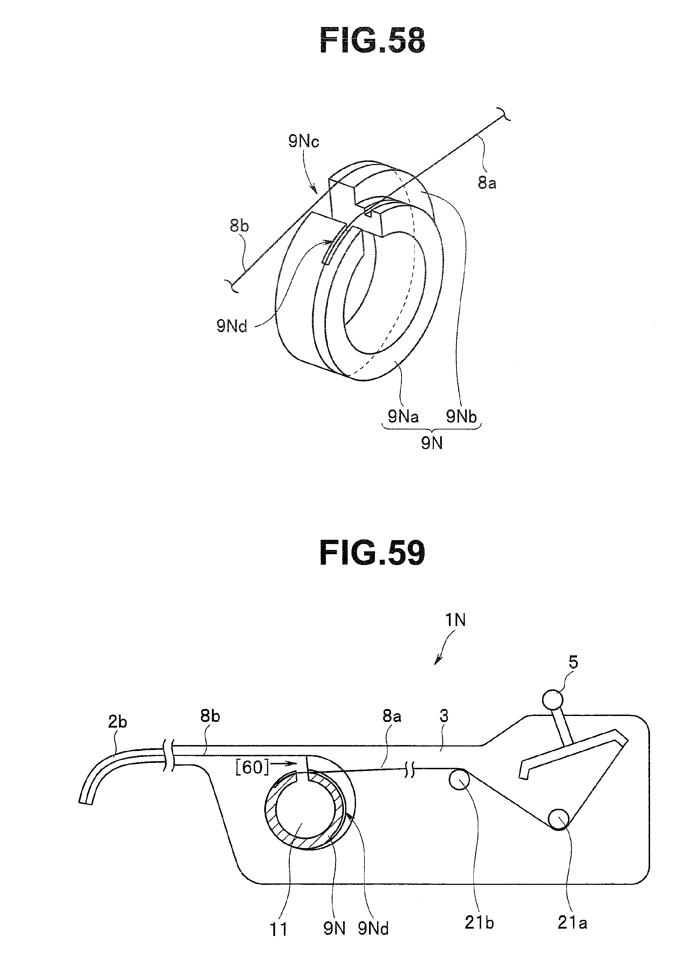

Figs. 57 to 61 are views showing a 14th embodiment of the present invention.

The present embodiment has a substantially same basic configuration as the above-described first embodiment and is slightly different from the first embodiment only in a shape of a C ring-shaped member in a pulling member operation apparatus. Thus, in the description below, same components as those of the first embodiment are denoted by equal reference numerals, a description of the components will be omitted, and only different components will be described in detail.

In a C ring-shaped member 9N in an endoscope 1N according to the present embodiment, a guide groove 9Nd which regulates movement of an operation input-side wire 8a in a width direction on an outer circumferential face of the C ring-shaped member 9N and guides the operation input-side wire 8a in a predetermined movement direction is formed at a predetermined part on an outer circumferential face of an operation wire extension portion 9Na where the operation input-side wire 8a extends of two C ring-shaped members (9Na and 9Nb).

The guide groove 9Nd is a part which crosses a notch portion 9Nc of the operation wire extension portion 9Na and is formed within a range around which the operation input-side wire 8a wraps when the C ring-shaped member 9N pivots.

Note that, as has been described in detail in the above-described first embodiment, a wire guide groove in a circumferential groove shape is provided in outer circumferential faces of the operation wire extension portion and the bending wire extension portion of the C ring-shaped member. The guide groove 9Nd is provided to be linked to the wire guide groove and is formed to be more noticeable than the wire guide groove.

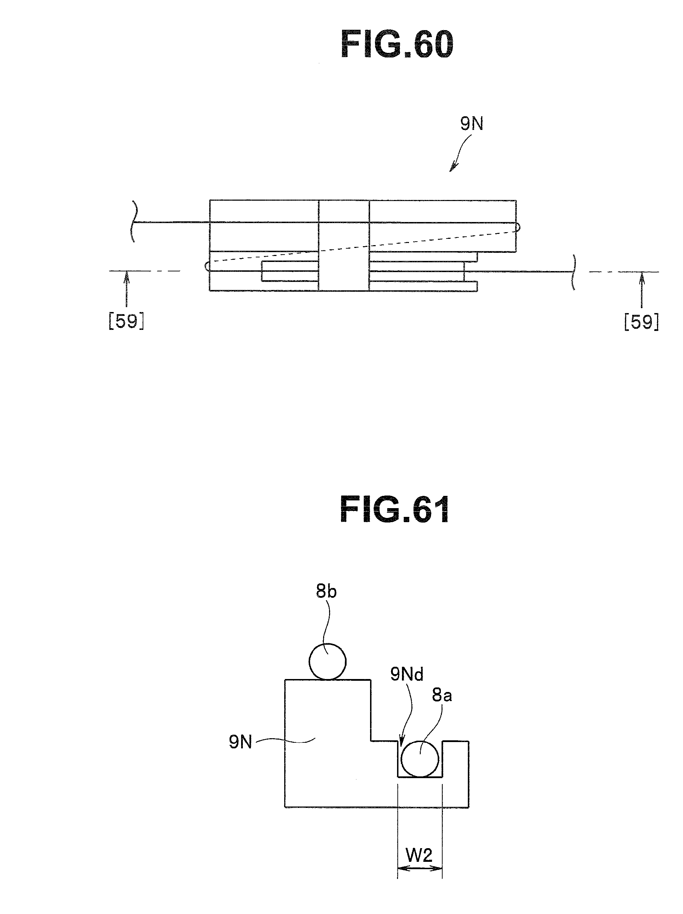

As shown in Fig. 61, a width dimension W2 of the guide groove 9Nd is set to be slightly larger than a diameter of the operation input-side wire 8a. A depth dimension of the guide groove 9Nd is desirably substantially equivalent to or slightly larger than the diameter of the operation input-side wire 8a. Other components are substantially same as the above-described first embodiment.

Since the guide groove 9Nd is formed at the part that crosses the notch portion 9Nc of the operation wire extension portion 9Na of the C ring-shaped member 9N in the present embodiment, the guide groove 9Nd guides C movement of the operation input-side wire 8a when an operation unit 5 is tilted and operated and inhibits the operation input-side wire 8a from coming off from the outer circumferential face of the C ring-shaped member 9N.

According to the 14th embodiment described above, same effects as those of the above-described first embodiment can be obtained. Additionally, according to the present embodiment, reliable movement of the operation input-side wire 8a can be ensured, and an ability for high-accuracy bending operation can be achieved.

### [15th Embodiment]

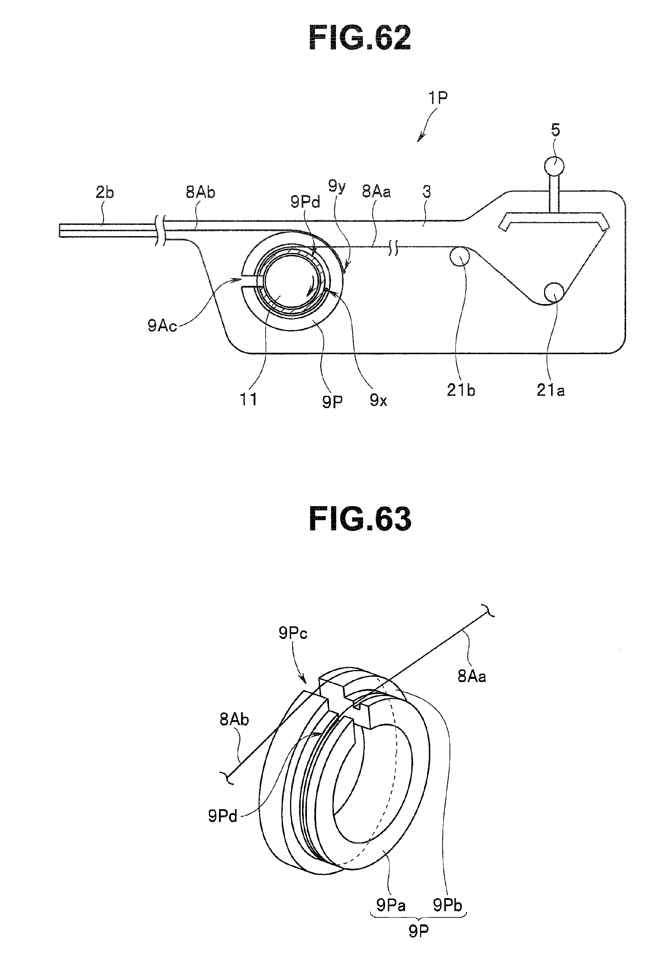

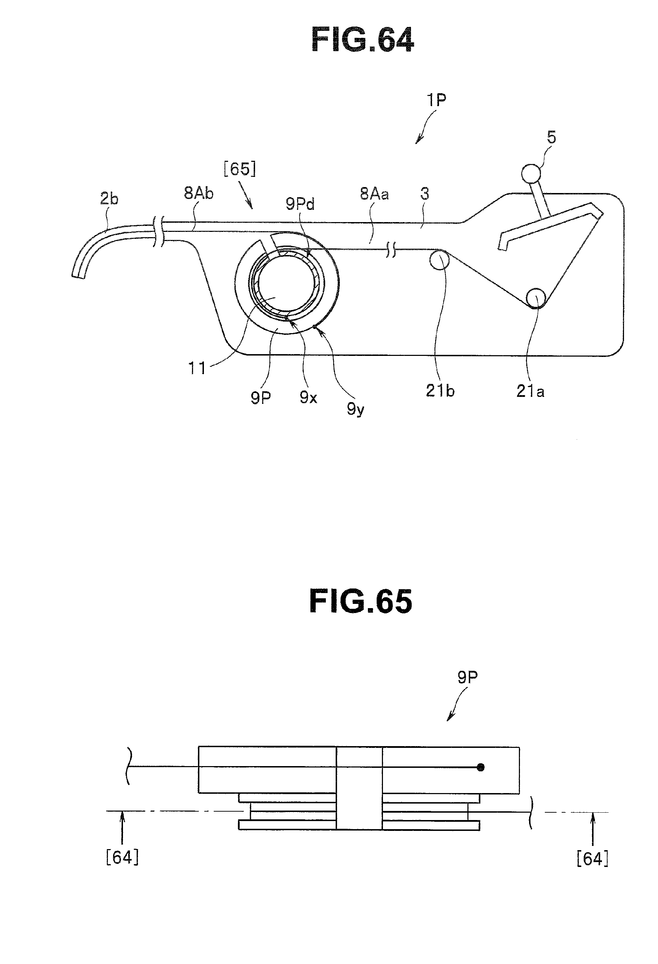

Figs. 62 to 65 are views showing a 15th embodiment of the present invention.

The present embodiment has a substantially same basic configuration as the above-described second embodiment and is slightly different from the second embodiment only in a shape of a C ring-shaped member in a pulling member operation apparatus, as in the above-described 14th embodiment. Thus, in the description below, same components as those of the above-described first and second embodiments are denoted by equal reference numerals, a description of the components will be omitted, and only different components will be described in detail.

In a C ring-shaped member 9P in an endoscope 1P according to the present embodiment, a guide groove 9Pd which regulates movement of an operation input-side wire 8Aa in a width direction on an outer circumferential face of the C ring-shaped member 9P and guides the operation input-side wire 8Aa in a predetermined movement direction is formed at a predetermined part on an outer circumferential face of an operation wire extension portion 9Pa where an operation input-side wire 8Aa extends of two C ring-shaped members (9Pa and 9Pb), substantially as in the above-described 14th embodiment. The guide groove 9Pd according to the present embodiment is formed over substantially a whole circumference on the outer circumferential face of the operation wire extension portion 9Pa.

Note that, as has been described in detail in the above-described first embodiment, a wire guide groove in a circumferential groove shape is provided in outer circumferential faces of the operation wire extension portion and the bending wire extension portion of the C ring-shaped member. The guide groove 9Pd is provided to be linked to the wire guide groove and is formed to be more noticeable than the wire guide groove.

A width dimension of the guide groove 9Pd in the present embodiment is set to be slightly larger than a diameter of the operation input-side wire 8Aa. A depth dimension of the guide groove 9Pd is desirably substantially equivalent to or slightly larger than the diameter of the operation input-side wire 8Aa. Other components are substantially same as in the above-described second embodiment. Actions are substantially same as in the above-described 14th embodiment.

According to the 15th embodiment described above, same effects as those of the above-described second and 14th embodiments can be obtained.

### [16th Embodiment]

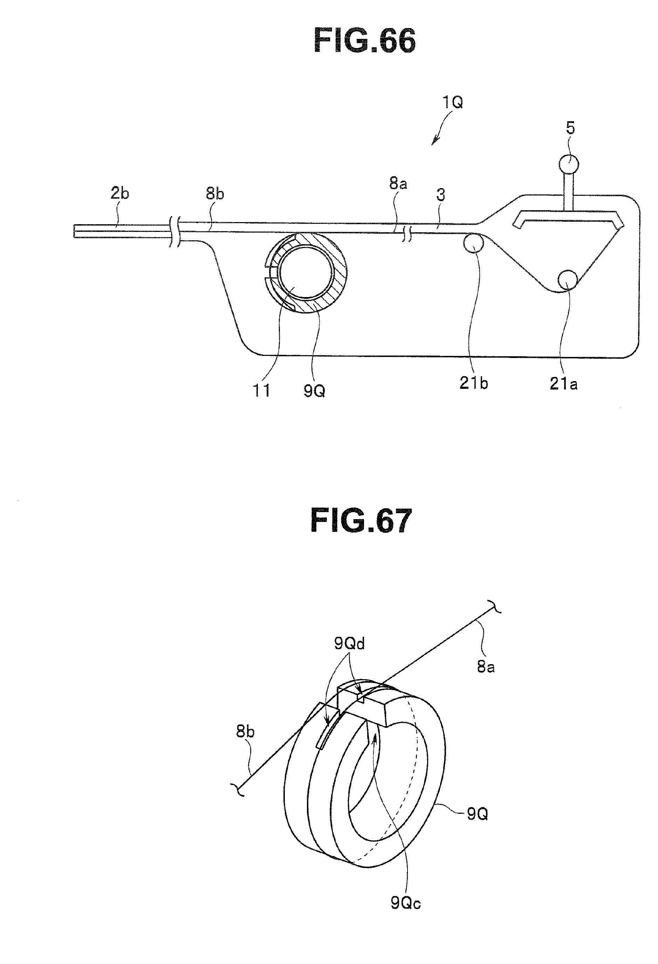

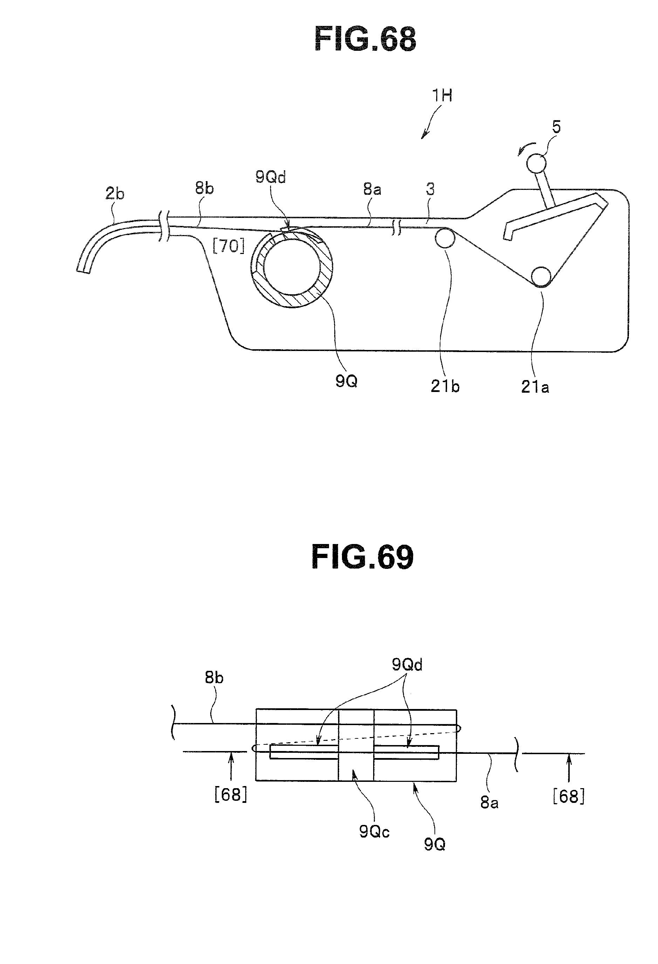

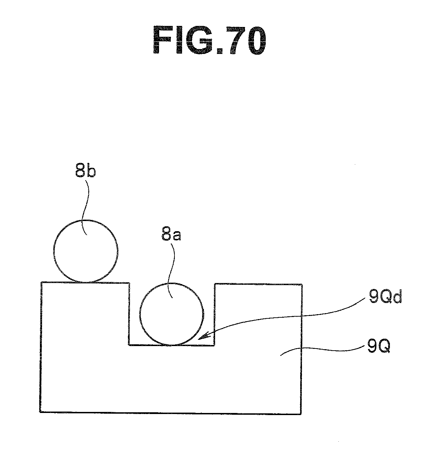

Figs. 66 to 70 are views showing a 16th embodiment of the present invention.

The present embodiment has a substantially same basic configuration as the above-described third embodiment and is slightly different from the third embodiment only in a shape of a C ring-shaped member in a pulling member operation apparatus, as in the above-described 14th embodiment. Thus, in the description below, same components as those of the above-described first and third embodiments are denoted by equal reference numerals, a description of the components will be omitted, and only different components will be described in detail.

In a C ring-shaped member 9Q in an endoscope 1 Q according to the present embodiment, a guide groove 9Qd which regulates movement of an operation input-side wire 8a in a width direction on an outer circumferential face of the C ring-shaped member 9Q and guides the operation input-side wire 8a in a predetermined movement direction is formed at a part where an operation input-side wire 8a extends of the C ring-shaped member 9Q. The guide groove 9Qd is a part which crosses a notch portion 9Qc and is formed within a range around which the operation input-side wire 8a wraps when the C ring-shaped member 9Q pivots.

Note that, as has been described in detail in the above-described first embodiment, a wire guide groove in a circumferential groove shape is provided in outer circumferential faces of an operation wire extension portion and a bending wire extension portion of the C ring-shaped member. The guide groove 9Qd is provided to be linked to the wire guide groove and is formed to be more noticeable than the wire guide groove.

A width dimension of the guide groove 9Qd in the present embodiment is set to be slightly larger than a diameter of the operation input-side wire 8a. A depth dimension of the guide groove 9Qd is desirably substantially equivalent to or slightly larger than the diameter of the operation input-side wire 8a. Other components are substantially same as in the above-described third embodiment. Actions are substantially same as in the above-described 14th embodiment.

According to the 16th embodiment described above, same effects as those of the above-described third and 14th embodiments can be obtained.

Note that a C ring-shaped member is formed to be elastically deformable and is formed to have a notch portion at a portion in each of the above-described embodiments. A pulling wire is wound around an outer circumferential face of the C ring-shaped member. The C ring-shaped member is configured to be narrowed at the notch portion against elastic force of the C ring-shaped member and be reduced in diameter by pulling the pulling wire. The C ring-shaped member is configured to be expanded at the notch portion due to the elastic force and be released from a diameter-reduced state when the pulling force of the pulling wire is released.

To supplement the amount of force in a direction expanding the notch portion at the time of release from the diameter-reduced state, for example, a configuration is conceivable in which an elasticized urging member, such as a coil spring or a flat spring, is disposed in a gap formed between faces facing the notch portion. With the above-described configuration, even if the pulling wire or the like affects the diameter reduction release action due to, for example, contact of the pulling wire with the C ring-shaped member when the C ring-shaped member is released from the diameter-reduced state, the effect below is obtained. When the C ring-shaped member is released from an amount of force of the diameter reduction action, the urging member supplements the diameter reduction release action, and the diameter reduction release action can be quickly and smoothly performed.

### [17th Embodiment]

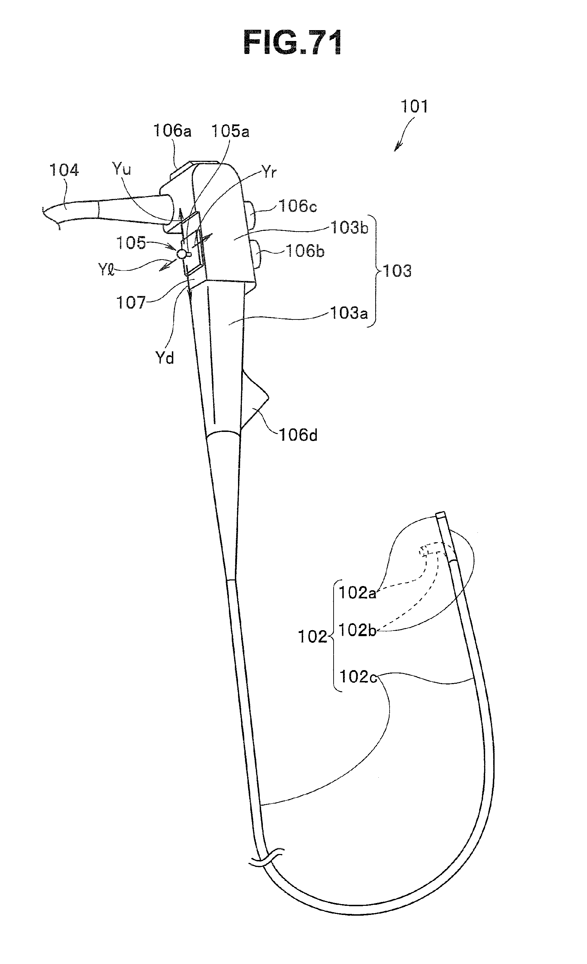

A 17th embodiment of the present invention will be described with reference to Figs. 71 to 77.

As shown in Fig. 71, an endoscope 101 according to the present embodiment is configured to include an elongated insertion portion 102, an operation portion 103 which is provided to be linked to a proximal end of the insertion portion 102, and a universal cord 104 which extends from a side portion of the operation portion 103.

The insertion portion 102 is composed of a distal end portion 102a, a bending portion 102b that is configured to be bendable in, for example, upward, downward, leftward, and rightward directions, and a flexible tube portion 102c that has flexibility and is formed to be long, all of which are provided to be linked in order from a distal end side.

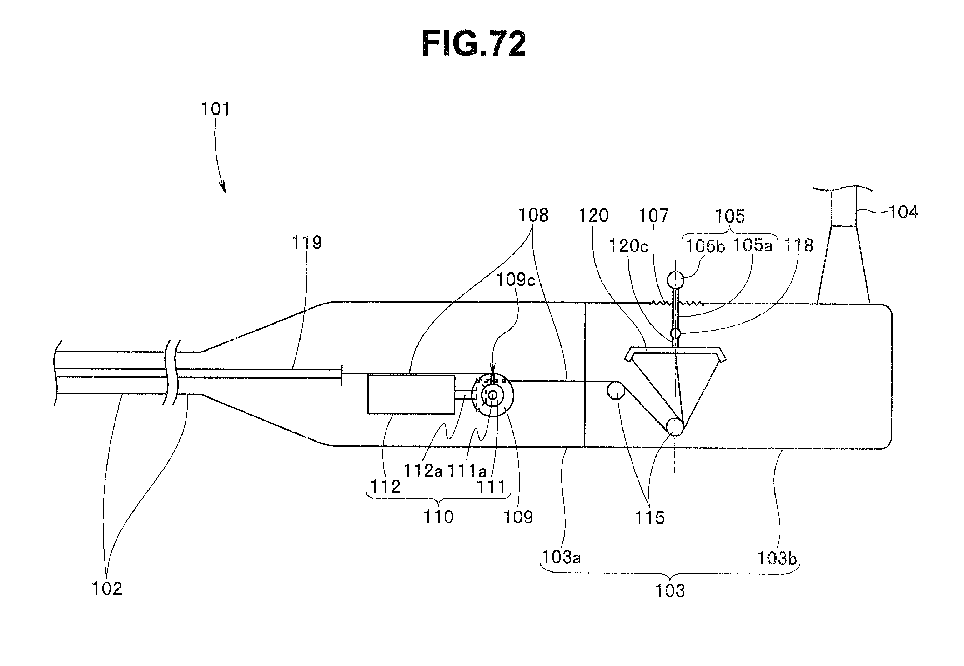

As shown in Figs. 71 and 72, the operation portion 103 is configured to include a grasping portion 103a which is provided to be linked to the insertion portion 102 and an operation portion body 103b which is provided to be linked to the grasping portion 103a. An operation unit 105 for giving an operation instruction to cause the bending portion 102b to make a bending motion is provided at the operation portion body 103b.

The operation unit 105 is a bending operation apparatus and is provided to extend, for example, orthogonally to a longitudinal axis of the operation portion 103 from an operation unit protrusion port (not shown) which is an opening provided at one face of the operation portion body 103b.

The operation unit 105 can be tilted in a direction of an arrow Yu, a direction of an arrow Yd, a direction of an arrow Yl, and a direction of an arrow Yr in Fig. 71. The bending portion 102b is configured to bend in an arbitrary one of an upward direction, a rightward direction, a downward direction, a leftward direction, a direction between the upward direction and the rightward direction, and the like by pulling or slackening a bending operation wire (to be described later) (hereinafter abbreviated as a bending wire) in response to a tilt operation with a tilt direction and a tilt angle of the operation unit 105.

In the present embodiment, the bending portion 102b is configured to bend in the four directions, the upward, downward, leftward, and rightward directions. The bending portion 102b, however, may be configured to bend only in the upward and downward directions. The suffixes u, d, l, and r denote the upward, downward, leftward, and rightward directions, in which the bending portion 102b is bent. In the description below, for example, reference character 108u denotes an upper bending wire, and reference character 108d denotes a lower bending wire. Note that the lowercase letter "l" is distinguished from the numeral "1" by writing the lowercase letter "l" in cursive script in the drawings.

For example, a switch 106a which gives instructions for various image pickup motions of an image pickup apparatus (not shown) provided inside the distal end portion 102a, an air/water feeding button 106b, a suction button 106c, and the like are provided at predefined positions of a sheath of the operation portion body 103b, in addition to the operation unit 105. A channel insertion port 106d which communicates with a treatment instrument channel (not shown) is provided at a sheath of the grasping portion 103a. Reference numeral 107 denotes a cover member. The cover member 107 holds the operation unit 105 so as to allow tilt operation of the operation unit 105 while watertightly covering the operation unit protrusion port and being in close contact with a shaft portion 105a.

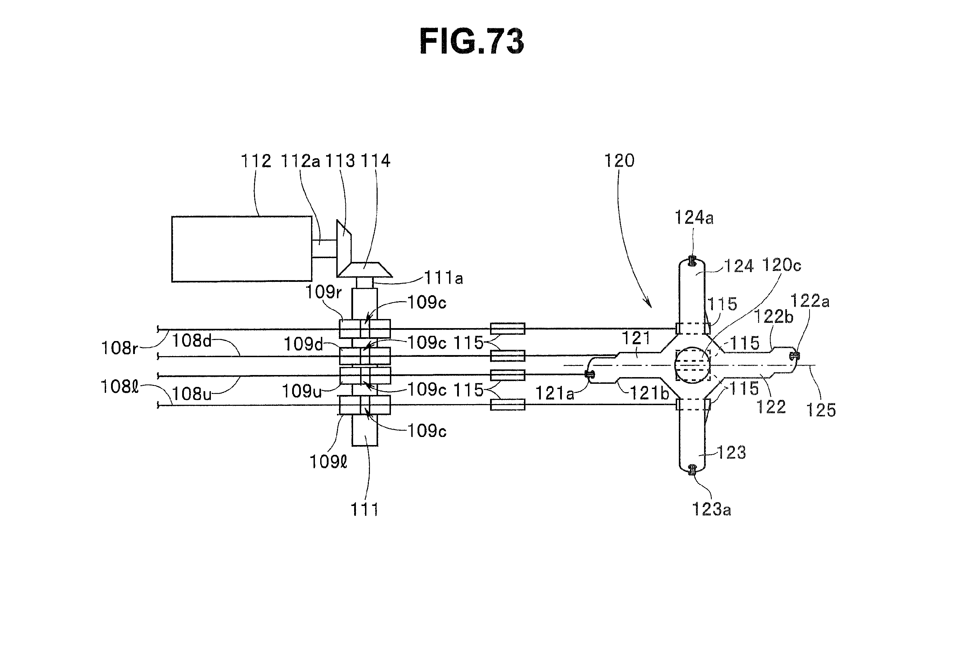

As shown in Figs. 72 and 73, four bending wires 108, four rotors 109, a bending drive section 110, a suspension frame 120 in a substantially cross shape, the shaft portion 105a of the operation unit 105, and a plurality of guide rollers 115 are disposed inside the operation portion 103.

The four bending wires 108 are pulling members and are the upper bending wire 108u, the lower bending wire 108d, a left bending wire 1081, and a right bending wire 108r. One ends of the respective bending wires 108u, 108d, 1081, and 108r are fixed to predefined positions of a distal end bending piece (not shown) constituting the bending portion 102b.

The four rotors 109 are driving force transmission portions which are elastically deformable, are ring-shaped, and have notches 109c. The four rotors 109 are an upper rotor 109u, a lower rotor 109d, a left rotor 1091, and a right rotor 109r. The upper bending wire 108u is wound around the upper rotor 109u, the lower bending wire 108d is wound around the lower rotor 109d, the left bending wire 1081 is wound around the left rotor 1091, and the right bending wire 108r is wound around the right rotor 109r.

The suspension frame 120 is a bending operation apparatus and is configured in a substantially cross shape. The suspension frame 120 includes an upper frame 121, a lower frame 122, a left frame 123, and a right frame 124, and a frame convex portion 120c serving as a shaft central axis protrudes from a central portion of the suspension frame 120. The shaft portion 105a of the operation unit 105 is coaxially attached and fixed to the frame convex portion 120c via a universal joint 118 which is pivotably disposed at a frame (not shown).

In the suspension frame 120, the upper frame 121 and the lower frame 122 are placed in line with each other with the frame convex portion 120c between the frames. An upward wire attaching portion 121 a is provided at an end portion of the upper frame 121, and a downward wire attaching portion 122a is provided at an end portion of the lower frame 122. The upper frame 121 includes an upper frame distal end crooked portion 121b, and the lower frame 122 includes a lower frame distal end crooked portion 122b. The upper frame distal end crooked portion 121b and the lower frame distal end crooked portion 122b are configured to be curved in different directions with an upper and lower frame center line 125 between the crooked portions.

The left frame 123 and the right frame 124 are orthogonal to the upper and lower frame center line 125 and are placed in line with each other with the frame convex portion 120c between the frames. A leftward wire attaching portion 123a is provided at an end portion of the left frame 123, and a rightward wire attaching portion 124a is provided at an end portion of the right frame 124.

The bending wires 108u, 108d, 1081, and 108r that are wound substantially once around the rotors 109u, 109d, 1091, and 109r and are then extended from the rotors 109u, 109d, 1091, and 109r are changed in travel path by the plurality of guide rollers 115 and reach the upward wire attaching portion 121a, the downward wire attaching portion 122a, the leftward wire attaching portion 123a, and the rightward wire attaching portion 124a of the suspension frame 120. The other ends of the respective bending wires 108u, 108d, 1081, and 108r are fixed to the wire attaching portion 121a, 122a, 123a, and 124a.

The bending drive section 110 is configured to include a pulley 111 and a motor 112. The individual rotors 109u, 109d, 1091, and 109r are placed at predefined positions of the pulley 111 to loosely fit on the pulley 111. Note that inner circumferential faces of the respective rotors 109u, 109d, 1091, and 109r are configured so as to be frictionally engageable with an outer circumferential face of the pulley 111.

In the present embodiment, a longitudinal axis of the pulley 111 and a longitudinal axis of the motor 112 intersect.

More specifically, the motor 112 is placed inside the grasping portion 103 a such that the longitudinal axis of the motor 112 has a parallel positional relationship with a longitudinal axis of the grasping portion 103a. That is, a motor shaft 112a of the motor 112 and a pulley shaft 111a which is a rotation axis of the pulley 111 are set to have an orthogonal positional relationship.

A first bevel gear 113 is disposed at the motor shaft 112a, and a second bevel gear 114 is disposed at the pulley shaft 111a. As a result, rotation of the motor shaft 112a is transmitted to the pulley shaft 111 a via the first bevel gear 113 and the second bevel gear 114, and the pulley 111 is rotated about the axis.

Reference numeral 119 denotes a coil pipe. The coil pipes 119 are provided corresponding to the respective bending wires 108u, 108d, 1081, and 108r. The corresponding bending wires 108u, 108d, 1081, and 108r are threaded through the respective coil pipes 119.

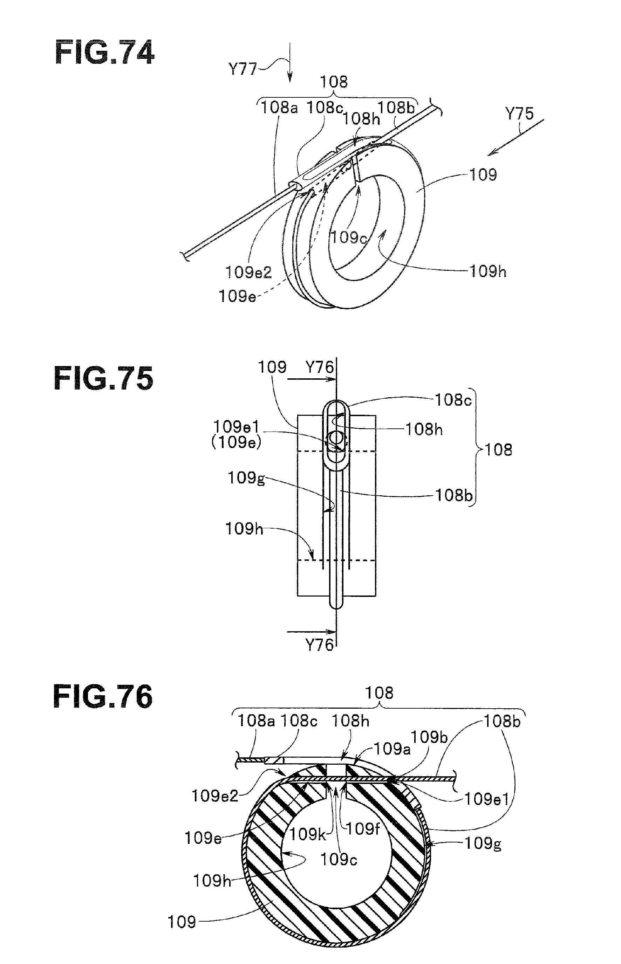

Configurations of the rotor 109 having the notch 109c and the bending wire 108 wound around the rotor 109 will be described with reference to Figs. 74 to 76.

As shown in Figs. 74 to 76, the rotor 109 has a through-hole 109h through which the pulley 111 is threaded and placed. The rotor 109 also has, at a predefined position, a wire threading hole 109e through which the bending wire 108 is threaded.

The wire threading hole 109e is a pulling member lead-out portion and is a straight hole including a first opening 109e1 and a second opening 109e2. The first opening 109e1 is formed on one side of the notch 109c at a rotor outer circumferential face. The second opening 109e2 is formed on the other side of the notch 109c at the rotor outer circumferential face. With the configuration, the bending wire 108 threaded through the wire threading hole 109e passes through the notch 109c. Thus, a third opening 109f and a fourth opening 109k derived from the wire threading hole 109e are formed in end faces of the notch 109c.

A groove 109g is formed along a circumferential direction on the first opening 109e1 side at the rotor outer circumferential face. The groove 109g is formed to place a wire relief member 108c (to be described later) constituting the bending wire 108 and is configured such that the member 108c is slidable. Positions where the first opening 109e1, the second opening 109e2, and the groove 109g described above are formed are set such that the first opening 109e1, the second opening 109e2, and the groove 109g are placed on an identical circumference on the outer circumferential face of the rotor 109.

In the present embodiment, the bending wire 108 is configured to include a first wire 108a, a second wire 108b, and a wire relief member 108c. The other end of the first wire 108a is integrally fixed to one end side of the wire relief member 108c by soldering, joining such as welding, or bonding. One end of the second wire 108b is integrally fixed to the other end side of the wire relief member 108c by soldering, joining such as welding, or bonding.

The wire relief member 108c is, for example, a metallic member having predefined resiliency. A long hole 108h is provided in the wire relief member 108c. A width dimension of the long hole 108h is set to be larger by an amount corresponding to a predefined clearance than a radial dimension of the second wire 108b. A length dimension of the long hole 108h is set in consideration of the amount of rotational movement of the rotor 109.

Note that reference character 109a shown in Fig. 76 denotes a wire take-up start position, which is located between the notch 109c and the first opening 109e1. Reference character 109b denotes a wire take-up end position, which corresponds to the first opening 109e1 in the present embodiment.

The bending wires 108u, 108d, 1081, and 108r are each configured to include the first wire 108a, the second wire 108b, and the wire relief member 108c described above. The bending wires 108u, 108d, 1081, and 108r led out inside the operation portion 103 then travel toward a proximal end side of the operation portion 103. The respective bending wires 108u, 108d, 1081, and 108r are wrapped around the rotors 109u, 109d, 1091, and 109r placed on the pulley 111 from the wire take-up start positions 109a, are threaded through the wire threading holes 109e from the second openings 109e2, and are led out from the first openings 109e1 constituting the take-up end positions. After the lead-out, the bending wires 108u, 108d, 1081, and 108r pass through the long holes 108h of the wire relief members 108c and are led out to an outside. With the configuration, the bending wires 108u, 108d, 1081, and 108r are wound once around outer circumferential faces of the rotors 109u, 109d, 1091, and 109r.

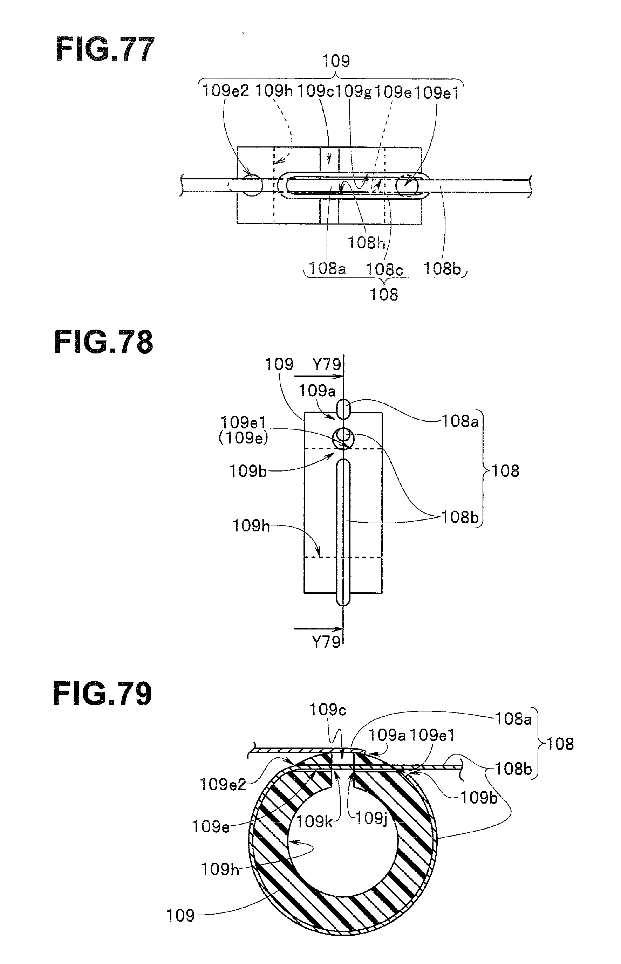

In the led-out state, each bending wire 108u, 108d, 1081, or 108r travels linearly inside the operation portion 103 without mutual interference between the wires 108a and 108b and a positional shift in a width direction (thrust direction) of the rotor outer circumferential face, as shown in Figs. 73 and 77.

The respective bending wires 108u, 108d, 1081, and 108r having passed through the long holes 108h are changed in travel path by the plurality of guide rollers 115 and are guided to the wire attaching portions 121a, 122a, 123a, and 124a of the suspension frame 120. The other ends of the respective second wires 108b are fixed to the wire attaching portions 121a, 122a, 123a, and 124a.

In the case, a midway portion of the wire relief member 108c of each bending wire 108 is placed at the wire take-up start position 109a. A portion on a proximal end side from the midway portion of the wire relief member 108c is slidably housed in the groove 109g. In the housed state, the other end of the wire relief member 108c is placed in a neighborhood of the first opening 109e1.

Action of the endoscope 101 with the above-described configuration will be described.

An operator drives the motor 112 and puts the pulley 111 into a rotating state when the insertion portion 102 of the endoscope 101 is to be inserted into, for example, a body. When the shaft portion 105a of the operation unit 105 is in an upright state, the bending wires 108u, 108d, 1081, and 108r that are wound around the rotors 109u, 109d, 1091, and 109r, respectively, placed on the pulley 111 are all in a predetermined slackened state. As a result, all the rotors 109u, 109d, 1091, and 109r glide over the pulley 111, and the bending portion 102b is held in a linear state.

When the operator tilts and operates the operation unit 105 in a direction of an arrow Yu in Fig. 71 to cause the bending portion 102b to make a bending action in, for example, the upward direction, the suspension frame 120 inclines. The inclination gradually changes the upper bending wire 108u fixed to the upward wire attaching portion 121 a from a slack state to a drawn state. In contrast, the other bending wires 108d, 1081, and 108r change to a slacker state.

As a result, of the bending wires 108u, 108d, 1081, and 108r wound in a slackened state around the rotors 109u, 109d, 1091, and 109r placed on the pulley 111, only the upward bending wire 108u is pulled. That is, the second wire 108b is pulled, and the upper rotor 109u is narrowed at the notch 109c against elastic force and is reduced in diameter.

The upper bending wire 108u wound around the upper rotor 109u in the present embodiment is placed on an identical circumference without being positionally shifted in a thrust direction on an outer circumferential face of the upper rotor 109u. Thus, the upper rotor 109u is reduced in diameter without being deformed in the thrust direction.

As a result, the upper rotor 109u enters a frictionally engaging state in which the inner circumferential face serving as a frictionally engaging surface of the upper rotor 109u is in uniform and close contact with the outer circumferential face of the pulley 111, and frictional resistance is generated over a whole close contact surface between the upper rotor 109u and the pulley 111. With the frictional resistance, the upper rotor 109u is rotated in a direction equal to a direction of the pulley 111 while gliding over the pulley 111.

As a result, the wire relief member 108c of the upward bending wire 108u housed in the groove 109g of the upper rotor 109u and the first wire 108a of the upward bending wire 108u placed on the insertion portion 102 side are moved, and the bending portion 102b starts a motion of bending in the upward direction.

If the operator continues to tilt and operate the operation unit 105 in the above-described direction so as to bring the upper rotor 109u into close contact with the pulley 111, frictional force between the upper rotor 109u and the pulley 111 in a close contact state increases further. For the reason, the first wire 108a of the upper bending wire 108u is further pulled with the rotation of the upper rotor 109u, and the bending portion 102b bends further in the upward direction.

As described above, the bending wire 108 is composed of the first wire 108a, the second wire 108b, and the wire relief member 108c having the long hole 108h, the rotor 109 is provided with the wire threading hole 109e, through which the second wire 108b of the bending wire 108 is threaded, and the groove 109g, in which the wire relief member 108c is slidably housed, is provided. The first opening 109e1, the second opening 109e2, and the groove 109g are set on an identical circumference. As a result, the bending wire 108 can be easily placed on the outer circumferential face of the rotor 109 by an amount corresponding to one turn on the identical circumference without being positionally shifted in the thrust direction.

### [First Modification of 17th Embodiment]

Note that, in the above-described 17th embodiment, the bending wire 108 is composed of the first wire 108a, the second wire 108b, and the wire relief member 108c having the long hole 108h. However, as shown in Figs. 78 and 79, the wire relief member 108c may not be provided, the other end of the first wire 108a may be fixed to the wire take-up start position 109a, and one end of the second wire 108b may be fixed in a neighborhood of the first opening 109e1 on a side opposite to the wire take-up start position 109a with the first opening 109e1 therebetween.

In the configuration, the groove 109g is unnecessary. In the present embodiment, the first opening 109e1, the second opening 109e2, a position where the first wire 108a is fixed, and a position where the second wire 108b is fixed are set so as to be placed on an identical circumference on an outer circumferential face of the rotor 109. Other components are same as those of the above-described 17th embodiment, equal members are denoted by equal reference numerals, and a description of the members will be omitted. With the configuration, same actions and effects as those of the 17th embodiment can be obtained.

### [Second Modification of 17th Embodiment]

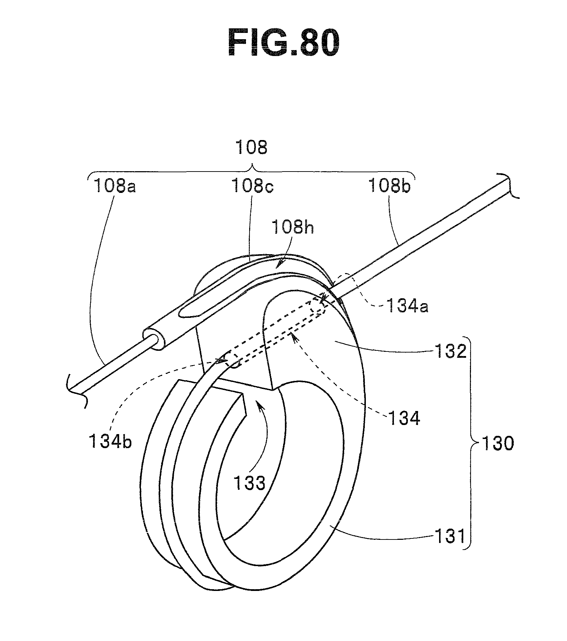

In the above-described 17th embodiment, the rotor 109 is ring-shaped. The rotor 109, however, is not limited to a ring shape, and a rotor 130 which is configured in a manner as shown in Fig. 80 may be adopted.

The rotor 130 may be configured to include an annular portion 131 and a rotation amount adjustment convex portion 132, include a notch 133 at the annular portion 131, and include a straight wire threading hole 134 at the rotation amount adjustment convex portion 132.

In the configuration, positions where a first opening 134a and a second opening 134b of the wire threading hole 134 are formed are set such that the first opening 134a and the second opening 134b are placed on an identical circumference on an outer circumferential face of the rotor 130.

With the configuration, same actions and effects as those of the above-described 17th embodiment can be obtained.

### [Third Modification of 17th Embodiment]

Note that the bending wire 108 is composed of the first wire 108a, the second wire 108b, and the wire relief member 108c having the long hole 108h in Fig. 80. The bending wire 108, however, may be the bending wire 108, in which the other end of the first wire 108a is fixed at a take-up start position, and one end of the second wire 108b is fixed in a neighborhood of a first opening 134a on a side opposite to the take-up start position with the first opening 134a therebetween.

### [Fourth Modification of 17th Embodiment]

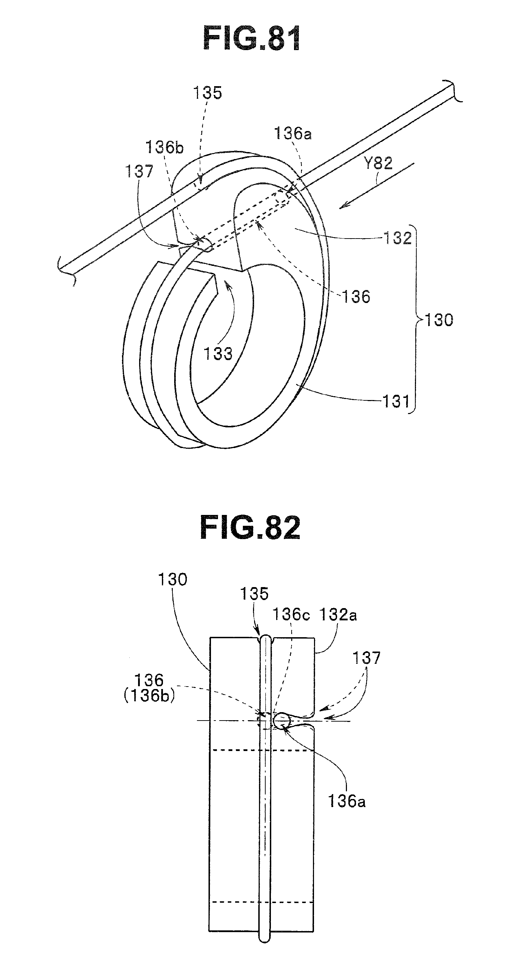

In a rotor 130, as shown in Figs. 81 and 82, a notch groove 137 including a wire threading hole 136 may be formed, for example, from one side face 132s of a rotation amount adjustment convex portion 132, instead of forming the above-described wire threading hole 134. Reference numeral 135 denotes a winding start groove 135, which defines a take-up start position of the bending wire 108.

In the configuration, the wire threading hole 136 is a crooked hole having a curved portion 136c at a midway portion. For the reason, a first opening 136a of the wire threading hole 136 is provided at a position which is positionally shifted from an identical circumference on a rotor outer circumferential face (to be described later) and prevents interference between wires. Positions where a second opening 136b of the wire threading hole 136 and the winding start groove 135 are formed are set such that the second opening 136b and the winding start groove 135 are placed on an identical circumference on the outer circumferential face of the rotor 130.

With the configuration, midway portion of one bending wire 108 is placed to be wound once around the outer circumferential face of the rotor 130 without a positional shift in a thrust direction, and same actions and effects as those of the above-described 17th embodiment can be obtained.

In addition, a need for the task of constructing the bending wire 108 with the first wire 108a, the second wire 108b, and the wire relief member 108c can be eliminated, and a need for the task of fixing the other end of the first wire 108a constituting the bending wire 108 to a take-up start position and fixing one end of the second wire 108b to a neighborhood of the wire take-up end position 109b can be eliminated.

### [Fifth Modification of 17th Embodiment]

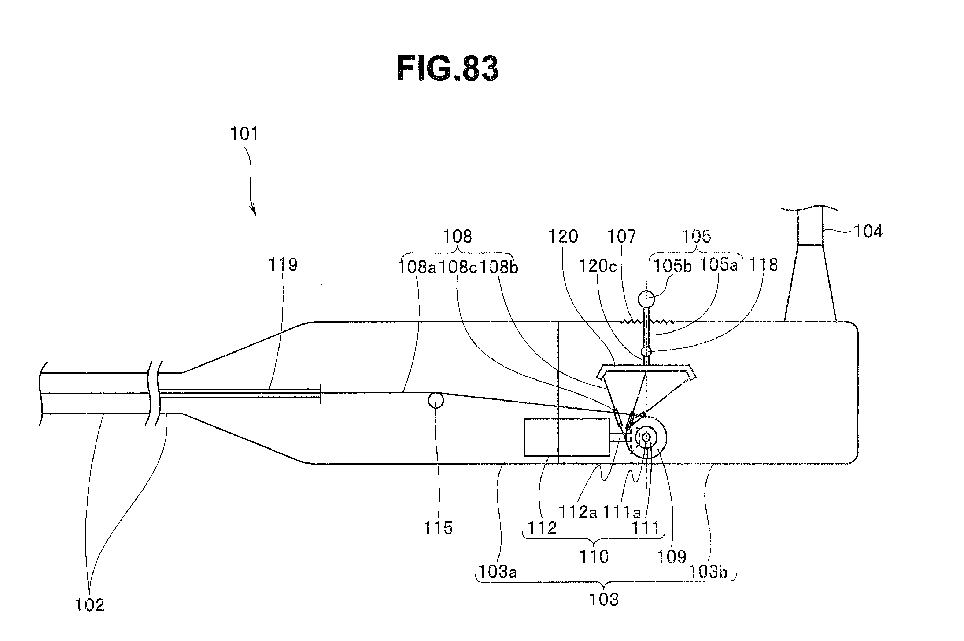

In a configuration in which a midway portion of the bending wire 108 is wound less than once around the rotor 109, as shown in Fig. 83, the bending wire 108, in which the wire relief member 108c having the long hole 108h is placed between the suspension frame 120 and the rotor 109, is used. In the present embodiment, one end portion of the long hole 108h of the wire relief member 108c is placed in a neighborhood of the first wire 108a that passes through the long hole 108h when the bending portion 102b is in a linear state, i.e., when the operation unit 105 is in an upright state.

With the configuration, a midway portion of the first wire 108a constituting the bending wire 108 can be placed to be wound around an outer circumferential face of the rotor 109 without a positional shift in a substantially thrust direction, as in the above-described 17th embodiment.

Note that an endoscope according to the present embodiment includes
(1) a pulling member, one end of which is fixed to a bending portion provided at an insertion portion,
   a bending drive section which outputs driving force that causes the bending portion to make a bending motion,
   a driving force transmission portion which has the pulling member wound around and placed on an outer circumferential face, includes a frictional engaging inner circumferential face frictionally engageable with the bending drive section, and is capable of being reduced in diameter by having a notch, and
   a bending operation apparatus for giving an operation instruction to pull the pulling member wound around the driving force transmission portion, reduce the driving force transmission portion in diameter, and cause the bending portion to make a bending motion, and
   the driving force transmission portion includes a pulling member lead-out portion which places, on an identical circumference of an outer circumferential face of the driving force transmission portion, the pulling member that is wound around the outer circumferential face, and crosses the notch and is extended to an outside from a take-up end position after the pulling member is extended from the bending portion and placed at a take-up start position of the outer circumferential face.
   In the endoscope (1),
(2) the pulling member lead-out portion is a hole which communicates with a first opening constituting the take-up end position and a second opening facing the first opening with the notch between the openings.
   In the endoscope (1),
(3) the pulling member lead-out portion is a notch groove having a hole which communicates with a first opening formed at the take-up end position and a second opening facing the first opening with the notch between the openings.
   In the endoscope (3),
(4) the notch groove is a crooked hole which has a cut and bent portion at a midway portion and in which the first opening and the second opening are positionally shifted.

According to the present embodiment, an endoscope can be achieved which reduces an annular member in diameter without distortion of the annular member by operation of an operation lever and can perform bending operation of a bending portion by pulling a pulling member with sufficient force against a pulley obtained through uniform and close contact of an inner surface of the annular member with an outer circumferential face of the pulley.

Note that the present invention is not limited to the above-described embodiments and that it is, of course, possible to implement various modifications and applications without departing from scope of the invention. Additionally, various stages of the invention are included in the embodiments, and various inventions can be extracted by appropriately combining the plurality of constituent features disclosed. For example, even if some constituent features are deleted from all constituent features shown in the individual embodiments, a configuration with the constituent features deleted can be extracted as an invention, as long as the problem to be solved by the invention can be solved, and the effects of the invention can be obtained. The present invention is not restricted by a specific embodiment, except as limited by the accompanying claims.

The present application is filed claiming priority from Japanese Patent Application No. 2012-025357 filed in Japan on February 8, 2012 and Japanese Patent Application No. 2012-006302 filed in Japan on January 16, 2012.

Disclosures of the individual basic applications are incorporated in the description, the claims, and the drawings of the present application.

### Industrial Applicability

The present invention can be applied not only to an endoscope control apparatus in a medical field but also to an endoscope control apparatus in an industrial field.

## Claims

1. An endoscope comprising:
a drive section which generates driving force for bending and driving a bending portion;
a C ring-shaped member which is a ring-shaped member frictionally engageable with a drive shaft of the drive section and has a notch portion at a portion of the ring-shaped member;
an operation input member for bending and operating the bending portion;
an operation input-side pulling member which is a first pulling member wound around the C ring-shaped member and extending from the C ring-shaped member toward the operation input member and is coupled to the operation input member such that a wrap distance, over which the operation input-side pulling member wraps around the C ring-shaped member across the notch portion from an extension position toward the operation input member on the C ring-shaped member, decreases with increase in the amount of operation of the operation input member; and
a bending portion-side pulling member which is a second pulling member wound around the C ring-shaped member and extending from the C ring-shaped member toward the bending portion and is coupled to the bending portion so as not to wrap around the C ring-shaped member across the notch portion from an extension position toward the bending portion on the C ring-shaped member.

2. The endoscope according to claim 1, wherein
the C ring-shaped member has a level difference portion between a position where the operation input-side pulling member starts to wrap around the C ring-shaped member and a position where the bending portion-side pulling member extends from the C ring-shaped member, and
the bending portion-side pulling member is placed so as not to wrap around the C ring-shaped member across the notch portion of the C ring-shaped member.

3. The endoscope according to claim 1, wherein
the C ring-shaped member includes a first C ring portion at which one end of the operation input-side pulling member is securely provided and a second C ring portion at which one end of the bending portion-side pulling member is securely provided, and
the second C ring portion is formed to be larger in diameter than the first C ring portion.

4. The endoscope according to claim 1, wherein the C ring-shaped member has an elasticized urging member at a part between facing surfaces of the notch portion.

5. The endoscope according to any one of claims 1 to 4, further having
a coil pipe member through which one end side of the operation input-side pulling member is threaded and which is securely provided on an outer circumferential face of the C ring-shaped member, and
which has a length corresponding to a region of wrapping around the C ring-shaped member from the position where the operation input-side pulling member starts to wrap around the C ring-shaped member.

6. The endoscope according to claim 1, wherein a part where the operation input-side pulling member is fixed to the C ring-shaped member is set to a neighborhood of a part to which the operation input-side pulling member is wrapped in a circular circumferential direction across the notch portion and which is at an angle of substantially less than 180° in the circular circumferential direction with respect to the notch portion and substantially faces the notch portion.

7. The endoscope according to claim 1, wherein a part where the operation input-side pulling member is fixed to the C ring-shaped member is set to a part to which the operation input-side pulling member is wrapped around the C ring-shaped member across the notch portion from an extension part toward the operation input member by an angle of substantially not less than 360° in a circular circumferential direction.

8. The endoscope according to claim 1, wherein the C ring-shaped member has a guide groove which guides movement of the operation input-side pulling member in a pulling direction.

9. The endoscope according to claim 1, wherein
the C ring-shaped member includes a first C ring portion at which one end of the operation input-side pulling member is securely provided and which has a first notch portion and a second C ring portion at which one end of the bending portion-side pulling member is securely provided and which has a second notch portion,
the first notch portion and the second notch portion are formed at parts shifted in a radial direction from each other, and
the first notch portion and the second notch portion are provided to be linked by a third notch portion which is formed in a circumferential direction of the C ring-shaped member.
